(19) Europäisches Patentamt
European Patent Office
Office européen des brevets

(11) EP 2 564 706 A1

(12) EUROPEAN PATENT APPLICATION

(43) Date of publication:
06.03.2013 Bulletin 2013/10

(21) Application number: 12194234.6

(22) Date of filing: 18.11.2005

(51) Int Cl.:
A01N 43/56 (2006.01) C07D 231/16 (2006.01)
C07D 401/04 (2006.01) C07D 405/12 (2006.01)
C07D 405/14 (2006.01)

(84) Designated Contracting States:
AT BE BG CH CY CZ DE DK EE ES FI FR GB GR HU IE IS IT LI LT LU LV MC NL PL PT RO SE SI SK TR

(30) Priority: 18.11.2004 US 629120 P
10.06.2005 US 689414 P

(62) Document number(s) of the earlier application(s) in accordance with Art. 76 EPC:
05851952.1 / 1 812 421

(71) Applicant: E. I. du Pont de Nemours and Company
Wilmington, DE 19898 (US)

(72) Inventor: Lham, George Philip
Wilmington, DE 19808 (US)

(74) Representative: Beacham, Annabel Rose
Dehns
St Bride's House
10 Salisbury Square
London
EC4Y 8JD (GB)

Remarks:
This application was filed on 26-11-2012 as a divisional application to the application mentioned under INID code 62.

# (54) Anthranilamide insecticides

(57) Disclosed are intermediate compounds for use in the synthesis of compounds of Formula 1, including all geometric and stereoisomers, N-oxides, and salts thereof,

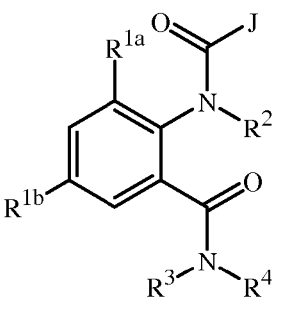

1

wherein
J
is a phenyl optionally substituted with one to four substituents independently selected from $R^5$; or J is a heterocyclic ring selected from the group consisting of J-1 to J-8;
$R^4$
is $C_4$-$C_{12}$ alkylcycloalkyl, $C_5$-$C_{12}$ alkenylcycloalkyl, $C_5$-$C_{12}$ alkynylcycloalkyl, $C_4$-$C_{12}$ cycloalkylalkyl, $C_5$-$C_{12}$ cycloalkylalkenyl, $C_5$-$C_{12}$ cycloalkylalkynyl, $C_4$-$C_{12}$ cycloalkenylalkyl or $C_4$-$C_{12}$ alkylcycloalkenyl; each optionally substituted with one to six substituents selected from $CH_3$ and halogen; or
$R^4$
is $C_3$-$C_5$ oxiranylalkyl, $C_3$-$C_5$ thiiranylalkyl, $C_4$-$C_6$ oxetanylalkyl, $C_4$-$C_6$ thietanylalkyl, 3-oxetanyl or 3-thietanyl, each optionally substituted with one to five substituents independently selected from $C_1$-$C_3$ alkyl, $C_1$-$C_3$ haloalkyl, halogen, CN, $C_2$-$C_4$ alkoxycarbonyl and $C_2$-$C_4$ haloalkoxycarbonyl; or
$R^4$
is $C_3$-$C_5$ aziridinylalkyl, $C_4$-$C_6$ azetidinylalkyl or 3-azetidinyl, each with $R^{10}$ attached to the nitrogen atom, and optionally substituted on carbon atoms with one to five substituents independently selected from $C_1$-$C_3$ alkyl, $C_1$-$C_3$ haloalkyl, halogen, CN, $C_2$-$C_4$ alkoxycarbonyl and $C_2$-$C_4$ haloalkoxycarbonyl; and
$R^{1a}$ $R^{1b}$ $R^2$ $R^3$ and $R^5$
are as defined in the disclosure.

EP 2 564 706 A1

## Description

### FIELD OF THE INVENTION

This invention relates to certain anthranilamides, their *N*-oxides, salts and compositions suitable for agronomic and nonagronomic uses, and methods of their use for controlling invertebrate pests such as arthropods in both agronomic and nonagronomic environments.

### BACKGROUND OF THE INVENTION

The control of invertebrate pests is extremely important in achieving high crop efficiency. Damage by invertebrate pests to growing and stored agronomic crops can cause significant reduction in productivity and thereby result in increased costs to the consumer. The control of invertebrate pests in forestry, greenhouse crops, ornamentals, nursery crops, stored food and fiber products, livestock, household, turf, wood products, and public and animal health is also important. Many products are commercially available for these purposes, but the need continues for new compounds that are more effective, less costly, less toxic, environmentally safer or have different modes of action.

PCT Patent Publication WO 03/015518 discloses N-acyl anthranilic acid derivatives of Formula i as arthropodicides

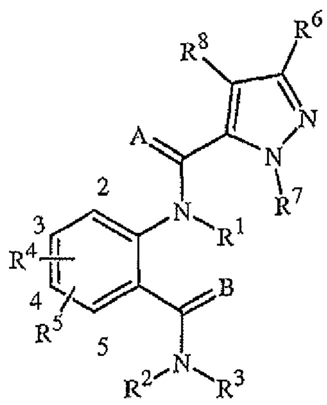

i

wherein, *inter alia,* A and B are independently O or S; $R^1$ is H, $C_1$-$C_6$ alkyl, $C_2$-$C_6$ alkoxycarbonyl or $C_2$-$C_6$ alkylcarbonyl; $R^2$ is H or $C_1$-$C_6$ alkyl; and $R^3$ is H or optionally substituted $C_1$-$C_6$ alkyl, $C_2$-$C_6$ alkenyl, $C_2$-$C_6$ alkynyl, or $C_3$-$C_6$ cycloalkyl.

### SUMMARY OF THE INVENTION

This invention is directed to compounds of Formula 1 including all geometric and stereoisomers, *N*-oxides, and agronomic or nonagronomic salts thereof, agricultural and nonagricultural compositions containing them and their use for controlling invertebrate pests:

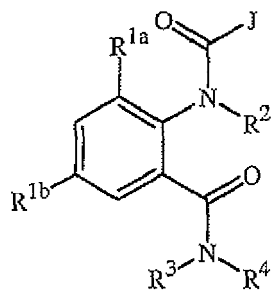

1

wherein:

J is a phenyl optionally substituted with one to four substituents independently selected from $R^5$; or

J is a heterocyclic ring selected from the group consisting of

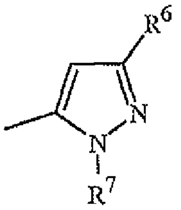
J-1 ,

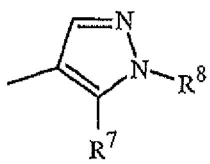
J-2 ,

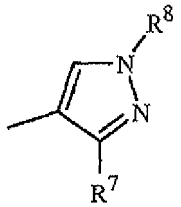
J-3 ,

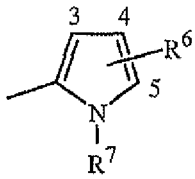
J-4

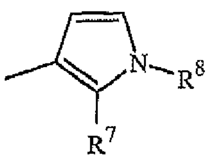
J-5 ,

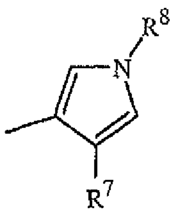
J-6 ,

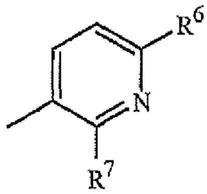
J-7 and

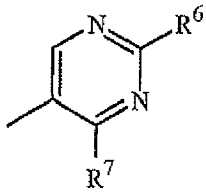
J-8 ;

$R^{1a}$ is $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, CHO, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkylcarbonyl, $C_2$-$C_4$ alkoxycarbonyl, $C_2$-$C_4$ alkylaminocarbonyl, $C_3$-$C_5$ dialkylaminocarbonyl, $C_1$-$C_4$ alkylamino or $C_2$-$C_6$ dialkylamino;

$R^{1b}$ is H, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, CHO, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkylcarbonyl, $C_2$-$C_4$ alkoxycarbonyl, $C_2$-$C_4$ alkylaminocarbonyl, $C_3$-$C_5$ dialkylaminocarbonyl, $C_1$-$C_4$ alkylamino or $C_2$-$C_6$ dialkylamino;

$R^2$ is H; or $C_1$-$C_6$ alkyl, $C_2$-$C_6$ alkenyl, $C_2$-$C_6$ alkynyl or $C_3$-$C_6$ cycloalkyl, each optionally substituted with one or more substituents selected from the group consisting of halogen, CN, $NO_2$, hydroxy, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, $C_2$-$C_4$ alkoxycarbonyl, $C_1$-$C_4$ alkylamino, $C_2$-$C_8$ dialkylamino and $C_3$-$C_6$ cycloalkylamino; or

$R^2$ is $C_2$-$C_6$ alkylcarbonyl, $C_2$-$C_6$ alkoxycarbonyl, $C_2$-$C_6$ alkylaminocarbonyl or $C_3$-$C_8$ dialkylaminocarbonyl;

$R^3$ is H, $C_1$-$C_6$ alkyl, $C_2$-$C_6$ alkenyl, $C_2$-$C_6$ alkynyl, $C_3$-$C_6$ cycloalkyl, $C_1$-$C_4$ alkoxy, $C_1$-$C_4$ alkylamino, $C_2$-$C_8$ dialkylamino, $C_3$-$C_6$ cycloalkylamino, $C_2$-$C_6$ alkoxycarbonyl or $C_2$-$C_6$ alkylcarbonyl;

$R^4$ is $C_4$-$C_{12}$ alkylcycloalkyl, $C_5$-$C_{12}$ alkenylcycloalkyl, $C_5$-$C_{12}$ alkynylcycloalkyl, $C_4$-$C_{12}$ cycloalkylalkyl, $C_5$-$C_{12}$ cycloalkylalkenyl, $C_5$-$C_{12}$ cycloalkylalkynyl, $C_4$-$C_{12}$ cycloalkenylalkyl or $C_4$-$C_{12}$ alkylcycloalkenyl, each optionally substituted with one to six substituents selected from $CH_3$ and halogen; or

R4 is $C_3$-$C_5$ oxiranylalkyl, $C_3$-$C_5$ thiiranylalkyl, $C_4$-$C_6$ oxetanylalkyl, $C_4$-$C_6$ thietanylalkyl, 3-oxetanyl or 3-thietanyl, each optionally substituted with one to five substituents independently selected from $C_1$-$C_3$ alkyl, $C_1$-$C_3$ haloalkyl, halogen, CN, $C_2$-$C_4$ alkoxycarbonyl and $C_2$-$C_4$ haloalkoxycarbonyl; or

$R^4$ is $C_3$-$C_5$ aziridinylalkyl, $C_4$-$C_6$ azetidinylalkyl or 3-azetidinyl, substituted with $R^{10}$ attached to the nitrogen atom, and optionally substituted on the carbon atoms with one to five substituents independently selected from $C_1$-$C_3$ alkyl, $C_1$-$C_3$ haloalkyl, halogen, CN, $C_2$-$C_4$ alkoxycarbonyl and $C_2$-$C_4$ haloalkoxycarbonyl;

each $R^5$ is independently $C_1$-$C_6$ alkyl, $C_3$-$C_6$ cycloalkyl, $C_1$-$C_6$ haloalkyl, halogen, CN, $C_1$-$C_4$ alkoxy, $C_1$-$C_4$ alkylthio, $C_1$-$C_4$ haloalkoxy, $C_1$-$C_4$ haloalkylthio, $C_1$-$C_4$ haloalkylsulfinyl or $C_1$-$C_4$ haloalkylsulfonyl; or

each $R^5$ is independently phenyl or pyridyl optionally substituted with one to three $R^9$;

each $R^6$ is independently selected from the group consisting of H, $C_1$-$C_6$ alkyl, $C_3$-$C_6$ cycloalkyl, $C_1$-$C_6$ haloalkyl, halogen, CN, $C_1$-$C_4$ alkoxy, $C_2$-$C_4$ alkoxycarbonyl, $C_1$-$C_4$ alkylthio, $C_1$-$C_4$ haloalkoxy, $C_1$-$C_4$ haloalkylthio, $C_1$-$C_4$ haloalkylsulfinyl and $C_1$-$C_4$ haloalkylsulfonyl;

$R^7$ is $C_1$-$C_6$ alkyl optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl, $C_1$-$C_4$ alkylsulfonyl, $C_2$-$C_4$ alkoxycarbonyl, $C_1$-$C_4$ alkylamino, $C_2$-$C_8$ dialkylamino and $C_3$-$C_6$ cycloalkylamino; or phenyl optionally substituted with one to three substituents selected from $R^9$; or

$R^7$ is

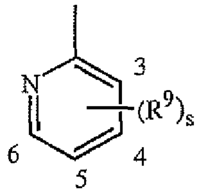

;

$R^8$ is H, $C_1$-$C_6$ alkyl, $C_1$-$C_6$ haloalkyl, $C_3$-$C_6$ alkenyl, $C_3$-$C_6$ haloalkenyl, $C_3$-$C_6$ alkynyl or $C_3$-$C_6$ haloalkynyl;

each $R^9$ is independently $C_1$-$C_6$ alkyl, $C_3$-$C_6$ cycloalkyl, $C_1$-$C_6$ haloalkyl, halogen, CN, $C_1$-$C_4$ alkoxy, $C_1$-$C_4$ alkylthio, $C_1$-$C_4$ haloalkoxy, $C_1$-$C_4$ haloalkylthio, $C_1$-$C_4$ haloalkylsulfinyl or $C_1$-$C_4$ haloalkylsulfonyl;

$R^{10}$ is H, $C_1$-$C_3$ alkyl, $C_1$-$C_3$ haloalkyl, $C_2$-$C_4$ alkylcarbonyl, $C_2$-$C_4$ haloalkylcarbonyl, $C_2$-$C_4$ alkoxycarbonyl or $C_1$-$C_3$ alkylsulfonyl; and

s is 0, 1 or 2;

provided that

(i) the compound of Formula 1 is other than N-[2-chloro-6-[[(1-methylcyclopropyl)amino]carbonyl]phenyl]-1-(3-chloro-2-pyridinyl)-3-(trifluoromethyl)-1*H*-pyrazole-5-carboxamide; and

(ii) the compound of Formula 1 is other than 3-bromo-1-(3-chloro-2-pyridinyl)-*N*-[4-cyano-2-[[(cyclopropylmethyl)amino]carbonyl]-6-methylphenyl]-1*H*-pyrazole-5-carboxamide.

This invention also provides a composition comprising a compound of Formula **1** and at least one additional component selected from the group consisting of a surfactant, a solid diluent and a liquid diluent, said composition optionally further comprising at least one additional biologically active compound or agent.

This invention also provides a composition for controlling an invertebrate pest comprising a biologically effective amount of a compound of Formula **1** and at least one additional component selected from the group consisting of a surfactant, a solid diluent and a liquid diluent, said composition optionally further comprising a biologically effective amount of at least one additional biologically active compound or agent.

This invention further provides a spray composition for controlling an invertebrate pest comprising a biologically effective amount of a compound of Formula 1 or the composition described above and a propellant. This invention also provides a bait composition for controlling an invertebrate pest comprising a biologically effective amount of a compound of Formula 1 or the composition described above, one or more food materials, optionally an attractant, and optionally a humectant.

This invention further provides a trap device for controlling an invertebrate pest comprising said bait composition and a housing adapted to receive said bait composition, wherein the housing has at least one opening sized to permit the invertebrate pest to pass through the opening so the invertebrate pest can gain access to said bait composition from a location outside the housing, and wherein the housing is further adapted to be placed in or near a locus of potential or known activity for the invertebrate pest.

This invention also provides a method for controlling an invertebrate pest comprising contacting the invertebrate pest or its environment with a biologically effective amount of a compound of Formula 1 (e.g., as a composition described herein). This invention also relates to such method wherein the invertebrate pest or its environment is contacted with a composition comprising a biologically effective amount of a compound of Formula **1** and at least one additional component selected from the group consisting of a surfactant, a solid diluent and a liquid diluent, said composition optionally further comprising a biologically effective amount of at least one additional biologically active compound or agent.

This invention also relates to an amide of Formula 10

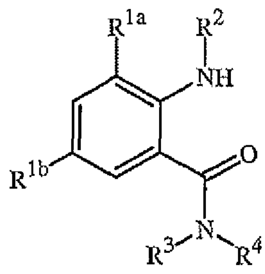

**10**

wherein $R^{1a}$, $R^{1b}$, $R^2$, $R^3$ and $R^4$ are as defined for Formula **1**, which is useful as an intermediate to prepare a compound of Formula **1.**

DETAILS OF THE INVENTION

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

In the above recitations, the term "alkyl", used either alone or in compound words such as "alkylthio" or "haloalkyl" includes straight-chain or branched alkyl, such as, methyl, ethyl, *n*-propyl, *i*-propyl, or the different butyl, pentyl or hexyl isomers. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl.

"Alkoxy" includes, for example, methoxy, ethoxy, n-propyloxy, isopropyloxy and the different butoxy, pentoxy and hexyloxy isomers. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include $CH_3S(O)$-, $CH_3CH_2S(O)$-, $CH_3CH_2CH_2S(O)$-, $(CH_3)_2CHS(O)$- and the different butylsulfinyl, pentylsulfinyl and hexylsulfinyl isomers. Examples of "alkylsulfonyl" include $CH_3S(O)_2$-, $CH_3CH_2S(O)_2$-, $CH_3CH_2CH_2S(O)_2$-, $(CH_3)_2CHS(O)_2$-, and the different butylsulfonyl, pentylsulfonyl and hexylsulfonyl isomers. "Alkylamino", "dialkylamino", and the like, are defined analogously to the above examples. "Cycloalkyl" includes, for example, cyclopropyl, cyclobutyl, cyclopentyl and cyclohexyl, The term "cycloalkylamino" includes the same groups linked through a nitrogen atom such as cyclopentylamino and cyclohexylamino. The term "(alkyl)cycloalkylamino" denotes a branched or straight-chain alkyl group and another cycloalkyl group both bonded to a nitrogen atom such as methylcyclopentylamino and ethylcyclohexylamino.

The term "alkylcycloalkyl" denotes alkyl substitution on a cycloalkyl moiety and includes, for example, ethylcyclopropyl, *i*-propylcyclobutyl, 3-methylcyclopentyl and 4-methylcyclohexyl, "Alkenylcycloalkyl", "alkynylcycloalkyl", and the like, are defined analogously to the above examples. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to straight-chain or branched alkyl groups. "Cyloalkylalkenyl", "cycloalkylalkynyl", and the like, are defined analogously to the above examples. "Cycloalkenyl" includes groups such as cyclopentenyl and cyclohexenyl as well as groups with more than one double bond such as 1,3- and 1,4-cyclohexadienyl. The term "cycloalkenylalkyl" denotes cycloalkenyl substitution on an alkyl moiety and includes, for example, cyclopentenylmethyl and 1-cyclohexenylethyl. The term "alkylcycloalkenyl" denotes alkyl substitution on a cycloalkenyl moiety and includes, for example, methylcyclopentenyl and 5-ethyl-3-cyclohexenyl.

The term "aromatic ring system" denotes fully unsaturated carbocycles and heterocycles in which the polycyclic ring system is aromatic (where aromatic indicates that the Hückel rule is satisfied for the ring system). The term "optionally

substituted" in connection with aromatic ring groups refers to groups that are unsubstituted or have at least one non-hydrogen substituent. Commonly, the number of optional substituents (when present) ranges from one to four.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different. Examples of "haloalkyl" include $F_3C$-, $ClCH_2$-, $CF_3CH_2$- and $CF_3CCl_2$-. The terms "haloalkenyl", "haloalkynyl", "halocycloalkyl", "haloalkoxy", "haloalkylthio", and the like, are defined analogously to the term "haloalkyl". Examples of "haloalkenyl" include $(Cl)_2C{=}CHCH_2$-and $CF_3CH_2CH{=}CHCH_2$-. Examples of "haloalkynyl" include $HC{\equiv}CCHCl$-, $CF_3C{\equiv}C$-, $CCl_3C{\equiv}C$- and $FCH_2C{\equiv}CCH_2$-. Examples of "haloalkoxy" include $CF_3O$-, $CCl_3CH_2O$-, $HCF_2CH_2CH_2O$- and $CF_3CH_2O$-. Examples of "haloalkylthio" include $CCl_3S$-, $CF_3S$-, $CCl_3CH_2S$- and $ClCH_2CH_2CH_2S$-. Examples of "haloalkylsulfinyl" include $CF_3S(O)$-, $CCl_3S(O)$-, $CF_3CH_2S(O)$- and $CF_3CF_2S(O)$-. Examples of "haloalkylsulfonyl" include $CF_3S(O)_2$-, $CCl_3S(O)_2$-, $CF_3CH_2S(O)_2$- and $CF_3CF_2S(O)_2$-.

"Alkylcarbonyl" denotes a straight-chain or branched alkyl moieties bonded to a C(=O) moiety. Examples of "alkylcarbonyl" include $CH_3C(=O)$-, $CH_3CH_2CH_2C(=O)$- and $(CH_3)_2CHC(=O)$-. Examples of "alkoxycarbonyl" include $CH_3OC(=O)$-, $CH_3CH_2OC(=O)$, $CH_3CH_2CH_2OC(=O)$-, $(CH_3)_2CHOC(=O)$- and the different butoxy- or pentoxycarbonyl isomers. Examples of "alkylaminocarbonyl" include $CH_3NHC(=O)$-, $CH_3CH_2NHC(=O)$-, $CH_3CH_2CH_2NHC(=O)$-, $(CH_3)_2CHNHC(=O)$- and the different butylamino- or pentylaminocarbonyl isomers. Examples of "dialkylaminocarbonyl" include $(CH_3)_2NC(=O)$-, $(CH_3CH_2)_2NC(=O)$-, $CH_3CH_2(CH_3)NC(=O)$-, $(CH_3)_2CHN(CH_3)C(=O)$-and $CH_3CH_2CH_2(CH_3)NC(=O)$-.

"Trialkylsilyl" includes three branched and/or straight-chain alkyl radicals attached to and linked through a silicon atom such as trimethylsilyl, triethylsilyl and *t*-butyldimethylsilyl.

The total number of carbon atoms in a substituent group is indicated by the "$C_i$-$C_j$" prefix where i and j are numbers from 2 to 8. For example, $C_1$-$C_4$ alkylsulfonyl designates methylsulfonyl through butylsulfonyl; $C_2$ alkoxyalkyl designates $CH_3OCH_2$; $C_3$ alkoxyalkyl designates, for example, $CH_3CH(OCH_3)$, $CH_3OCH_2CH_2$ or $CH_3CH_2OCH_2$; and $C_4$ alkoxyalkyl designates the various isomers of an alkyl group substituted with an alkoxy group containing a total of four carbon atoms, examples including $CH_3CH_2CH_2OCH_2$ and $CH_3CH_2OCH_2CH_2$.

When a compound is substituted with a substituent bearing a subscript that indicates the number of said substituents can exceed 1, said substituents (when they exceed 1) are independently selected from the group of defined substituents, e.g., $(R^9)_s$, s is 0, 1 or 2. When a group contains a substituent which can be hydrogen, for example $R^2$ or $R^9$, then, when this substituent is taken as hydrogen, it is recognized that this is equivalent to said group being unsubstituted.

Compounds of this invention can exist as one or more stereoisomers. The various stereoisomers include enantiomers, diastereomers, atropisomers and geometric isomers, One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. Accordingly, the present invention comprises compounds selected from Formula **1**, *N*-oxides, and agronomic and nonagronomic suitable salts thereof. The compounds of the invention may be present as a mixture of stereoisomers, individual stereoisomers, or as an optically active form.

One skilled in the art will appreciate that not all nitrogen containing heterocycles can form *N*-oxides since the nitrogen requires an available lone pair for oxidation to the oxide; one skilled in the art will recognize those nitrogen containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

The salts of the compounds of the invention include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. The salts of the compounds of the invention also include those formed with organic bases (e.g., pyridine, ammonia, or triethylamine) or inorganic bases (e.g., hydrides, hydroxides, or carbonates of sodium, potassium, lithium, calcium, magnesium or barium) when the compound contains an acidic group such as a carboxylic acid or phenol.

Embodiments of the present invention as described in the Summary of the Invention include:

Embodiment 1A. A compound of Formula **1** wherein $R^{1a}$ is $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$. haloalkylsulfonyl.

Embodiment 1B. A compound of Formula 1 wherein $R^{1a}$ is $CH_3$, $CF_3$, $OCF_3$, $OCHF_2$, $S(O)_nCF_3$, $S(O)_nCHF_2$, CN or halogen; and n is 0, 1 or 2.

Embodiment 1C. A compound of Formula **1** wherein $R^{1a}$ is $CH_3$, F, Cl, Br or I.

Embodiment 1D. A compound of Formula **1** wherein $R^{1a}$ is $CH_3$, Cl, Br or I.

Embodiment 1E. A compound of Formula **1** wherein $R^{1a}$ is $CH_3$ or Cl.

Embodiment 2A. A compound of Formula **1** wherein $R^{1b}$ is H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl.

Embodiment 2B. A compound of Formula **1** wherein $R^{1b}$ is H, $CH_3$, $CF_3$, $OCF_3$, $OCHF_2$, $S(O)_pCF_3$, $S(O)_pCHF_2$, CN or halogen; and p is 0, 1 or 2.

Embodiment 2C. A compound of Formula **1** wherein $R^{1b}$ is $CH_3$, $CF_3$, $OCF_3$, $OCHF_2$, $S(O)_pCF_3$, $S(O)_pCHF_2$, CN or halogen.

Embodiment 2D. A compound of Formula **1** wherein $R^{1b}$ is H, $CH_3$, $CF_3$, CN, F, Cl, Br or I.

Embodiment 2E. A compound of Formula **1** wherein $R^{1b}$ is $CH_3$, $CF_3$, CN, F, Cl, Br or I.

Embodiment 2F. A compound of Formula **1** wherein $R^{1b}$ is CN, F, Cl, Br or I.

Embodiment 2G. A compound of Formula **1** wherein $R^{1b}$ is Cl, Br or CN.

Embodiment 2H. A compound of Formula **1** wherein $R^{1b}$ is Cl or Br.

Embodiment 2I. A compound of Formula **1** wherein $R^{1b}$ is CN.

Embodiment 2J. A compound of Formula **1** wherein $R^{1b}$ is other than H.

Embodiment 2K. A compound of Formula **1** wherein $R^{1b}$ is other than CN.

Embodiment 3A. A compound of Formula **1** wherein $R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl.

Embodiment 3B. A compound of Formula **1** wherein $R^2$ is H.

Embodiment 4A. A compound of Formula **1** wherein $R^3$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl.

Embodiment 4B. A compound of Formula **1** wherein $R^3$ is H.

Embodiment 5A. A compound of Formula **1** wherein $R^4$ is $C_4$-$C_{12}$ alkylcycloalkyl optionally substituted with one to six substituents selected from $CH_3$ and halogen.

Embodiment 5B. A compound of Formula **1** wherein $R^4$ is 1-methylcycloalkyl optionally substituted with one to six substituents selected from $CH_3$ and halogen.

Embodiment 5C. A compound of Formula **1** wherein $R^4$ is 1-methylcyclopropyl optionally substituted with one to four substituents selected from $CH_3$ and halogen.

Embodiment 5D. A compound of Formula **1** wherein $R^4$ is 1-methylcyclobutyl optionally substituted with one to four substituents selected from $CH_3$ and halogen.

Embodiment 5E. A compound of Formula **1** wherein $R^4$ is ($C_1$-$C_8$ alkyl)($C_3$-$C_4$ cycloalkyl) optionally substituted with one to six substituents selected from $CH_3$ and halogen.

Embodiment 5F. A compound of Formula **1** wherein $R^4$ is ($C_2$-$C_8$ alkenyl)($C_3$-$C_4$ cycloalkyl) optionally substituted with one to six substituents selected from $CH_3$ and halogen.

Embodiment 5G. A compound of Formula **1** wherein $R^4$ is ($C_2$-$C_8$ alkynyl)($C_3$-$C_4$ cycloalkyl) optionally substituted with one to six substituents selected from $CH_3$ and halogen.

Embodiment 5H. A compound of Formula **1** wherein $R^4$ is ($C_1$-$C_8$ alkyl)($C_3$-$C_4$ cycloalkenyl) optionally substituted with one to six substituents selected from $CH_3$ and halogen.

Embodiment 6A. A compound of Formula **1** wherein $R^4$ is $C_4$-$C_{12}$ cycloalkylalkyl optionally substituted with one to six substituents selected from $CH_3$ and halogen.

Embodiment 6B. A compound of Formula **1** wherein $R^4$ is cyclopropylmethyl or cyclobutylmethyl; each optionally substituted with one to six substituents selected from $CH_3$ and halogen.

Embodiment 6C. A compound of Formula **1** wherein $R^4$ is ($C_3$-$C_4$ cycloalkyl)($C_1$-$C_8$ alkyl) optionally substituted with one to six substituents selected from $CH_3$ and halogen.

Embodiment 6D. A compound of Formula **1** wherein $R^4$ is ($C_3$-$C_4$ cycloalkyl)($C_2$-$C_8$ alkenyl) optionally substituted with one to six substituents selected from $CH_3$ and halogen.

Embodiment 6E. A compound of Formula **1** wherein $R^4$ is ($C_3$-$C_4$ cycloalkyl)($C_2$-$C_8$ alkynyl) optionally substituted with one to six substituents selected from $CH_3$ and halogen.

Embodiment 6F. A compound of Formula **1** wherein $R^4$ is ($C_3$-$C_4$ cycloalkenyl)($C_1$-$C_8$ alkyl) optionally substituted with one to six substituents selected from $CH_3$ and halogen.

Embodiment 6G. A compound of Formula **1** wherein $R^4$ is other than optionally substituted $C_4$-$C_6$ cycloalkylalkyl.

Embodiment 6H. A compound of Formula **1** wherein $R^4$ is other than optionally substituted ($C_3$-$C_4$ cycloalkyl)($C_1$-$C_6$ alkyl).

Embodiment 6I. A compound of Formula **1** wherein $R^4$ is other than cyclopropylmethyl.

Embodiment 6J. A compound of Formula **1** wherein $R^4$ is other than 1-cyclopropylethyl.

Embodiment 6K. A compound of Formula **1** wherein $R^4$ is other than (2-methylcyclopropyl)methyl.

Embodiment 6L. A compound of Formula **1** wherein $R^4$ is other than (2,2-dichloro-1-methylcyclopropyl)methyl.

Embodiment 6M. A compound of Formula **1** wherein $R^4$ is other than (1-methylcyclopropyl)methyl.

Embodiment 6N. A compound of Formula **1** wherein $R^4$ is other than 1-cyclobutylethyl.

Embodiment 7A. A compound of Formula **1** wherein $R^4$ is 1-methylcyclopropyl, cyclopropylmethyl or 1-cyclopropylethyl, each optionally substituted with one to two halogen on cyclopropyl.

Embodiment 7B. A compound of Formula **1** wherein $R^4$ is 1-methylcyclopropyl, cyclopropylmethyl or 1-cyclopropylethyl.

Embodiment 7C. A compound of Formula **1** wherein $R^4$ is 1-methylcyclopropyl, cyclopropylmethyl or 1-cyclopropylethyl, each substituted with two halogen on cyclopropyl.

Embodiment 7D. A compound of Formula **1** wherein $R^4$ is 1-methylcyclopropyl, optionally substituted with one to two halogen on cyclopropyl.

Embodiment 7E. A compound of Formula **1** wherein $R^4$ is 1-methylcyclopropyl.

Embodiment 7F. A compound of Formula **1** wherein $R^4$ is cyclopropylmethyl or 1-cyclopropylethyl, each optionally substituted with one to two halogen on cyclopropyl.

Embodiment 7G. A compound of Formula **1** wherein $R^4$ is cyclopropylmethyl or 1-cyclopropylethyl.

Embodiment 7H. A compound of Formula **1** wherein $R^4$ is cyclopropylmethyl optionally substituted with one to two halogen on cyclopropyl.

Embodiment 71. A compound of Formula **1** wherein $R^4$ is cyclopropylmethyl.

Embodiment 7J. A compound of Formula **1** wherein $R^4$ is 1-cyclopropylethyl optionally substituted with one to two halogen on cyclopropyl.

Embodiment 7K. A compound of Formula **1** wherein $R^4$ is 1-cyclopropylethyl.

Embodiment 7L. A compound of Formula **1** wherein $R^4$ is other than 1-methylcyclopropyl.

Embodiment 7M. A compound of Formula **1** wherein $R^4$ is other than optionally substituted 1-methylcyclopropyl.

Embodiment 7N. A compound of Formula **1** wherein $R^4$ is other than optionally substituted $C_4$-$C_{12}$ alkylcycloalkyl.

Embodiment 70. A compound of Formula **1** wherein $R^4$ is other than optionally substituted $C_5$-$C_{12}$ alkenylcycloalkyl.

Embodiment 7P. A compound of Formula **1** wherein $R^4$ is other than optionally substituted $C_5$-$C_{12}$ alkynylcycloalkyl.

Embodiment 7Q. A compound of Formula **1** wherein $R^4$ is other than optionally substituted $C_4$-$C_{12}$ cycloalkylalkyl.

Embodiment 7R. A compound of Formula **1** wherein $R^4$ is other than optionally substituted $C_5$-$C_{12}$ cycloalkylalkenyl.

Embodiment 7S. A compound of Formula **1** wherein $R^4$ is other than optionally substituted $C_5$-$C_{12}$ cycloalkylalkynyl.

Embodiment 7T. A compound of Formula **1** wherein $R^4$ is other than optionally substituted $C_4$-$C_{12}$ cycloalkenylalkyl.

Embodiment 7U. A compound of Formula **1** wherein $R^4$ is other than optionally substituted $C_4$-$C_{12}$ alkylcycloalkenyl.

Embodiment 7V. A compound of Formula **1** wherein $R^4$ is 1-methylcyclopropyl, and $R^{1b}$ is other than H.

Embodiment 8A. A compound of Formula **1** wherein $R^4$ is $C_3$-$C_5$ oxiranylalkyl, $C_4$-$C_6$ oxetanylalkyl or 3-oxetanyl, each optionally substituted with 1 to 2 substituents independently selected from $CH_3$, $CF_3$, halogen, CN and $C(O)OCH_3$.

Embodiment 8B. A compound of Formula **1** wherein $R^4$ is oxiranylmethyl, 2-oxetanylmethyl, 3-oxetanylmethyl or 3-oxetanyl, each optionally substituted with 1 to 2 $CH_3$.

Embodiment 8C. A compound of Formula **1** wherein $R^4$ is oxiranylmethyl.

Embodiment 8D. A compound of Formula **1** wherein $R^4$ is 2-oxetanylmethyl.

Embodiment 8E. A compound of Formula **1** wherein $R^4$ is 3-oxetanylmethyl.

Embodiment 8F. A compound of Formula **1** wherein $R^4$ is 3-oxetanyl.

Embodiment 8G. A compound of Formula **1** wherein $R^4$ is other than optionally substituted oxiranylmethyl.

Embodiment 8H. A compound of Formula **1** wherein $R^4$ is other than optionally substituted 2-oxetanylmethyl.

Embodiment 8I. A compound of Formula **1** wherein $R^4$ is other than optionally substituted 3-oxetanylmethyl.

Embodiment 8J. A compound of Formula **1** wherein $R^4$ is other than optionally substituted $C_3$-$C_5$ oxiranylalkyl, $C_3$-$C_5$ thiiranylalkyl, $C_4$-$C_6$ oxetanylalkyl, $C_4$-$C_6$ thietanylalkyl, 3-oxetanyl or 3-thietanyl.

Embodiment 8K. A compound of Formula **1** wherein $R^4$ is other than optionally substituted $C_3$-$C_5$ aziridinylalkyl, $C_4$-$C_6$ azetidinylalkyl or 3-azetidinyl.

Embodiment 9A. A compound of Formula **1** wherein $R^4$ is aziridinylmethyl, 2-azetidinylmethyl, 3-azetidinylmethyl or 3-azetidinyl, each with $R^{10}$ attached to the nitrogen atom, and optionally substituted on carbon atoms with 1 to 2 substituents independently selected from $CH_3$, $CF_3$, halogen, CN and $C(O)OCH_3$.

Embodiment 9B. A compound of Formula **1** wherein $R^4$ is aziridinylmethyl, 2-azetidinylmethyl, 3-azetidinylmethyl or 3-azetidinyl, each with $R^{10}$ attached to the nitrogen atom, and optionally substituted on the carbon atoms with 1

to 2 $CH_3$.

Embodiment 9C. A compound of Formula **1** wherein $R^{10}$ is H or $C_1$-$C_3$ alkyl.

Embodiment 10A. A compound of Formula **1** wherein each $R^6$ is independently selected from the group consisting of H, $CH_3$, $CF_3$, $CH_2CF_3$, $CHF_2$, $OCH_2CF_3$, $OCHF_2$ and halogen.

Embodiment 10B. A compound of Formula **1** wherein each $R^6$ is independently halogen, $OCH_2CF_3$, $OCHF_2$ or $CF_3$;

Embodiment 10C. A compound of Formula **1** wherein each $R^6$ is independently Cl, Br, $OCH_2CF_3$ or $CF_3$.

Embodiment 10D. A compound of Formula **1** wherein each $R^6$ is Cl, Br, $CF_3$ or $C_1$-$C_2$ fluoroalkoxy.

Embodiment 11A. A compound of Formula **1** wherein $R^7$ is a phenyl ring optionally substituted with one to three substituents selected from $R^9$

Embodiment 11B. A compound of Formula **1** **wherein** each $R^9$ is independently H, $C_1$-$C_4$ alkyl, $C_1$-$C_4$ haloalkyl, halogen or CN.

Embodiment 11 C. A compound of Formula **1** wherein $R^7$ is

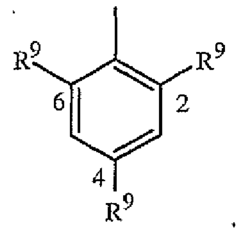

Embodiment 11D. A compound of Formula **1** wherein each $R^9$ is independently H, $CH_3$, $CF_3$, CN or halogen.

Embodiment 12A. A compound of Formula 1 wherein $R^7$ is

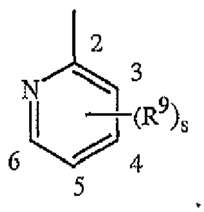

Embodiment 12B. A compound of Formula **1** wherein each $R^9$ is independently $C_1$-C4 alkyl, $C_1$-$C_4$ haloalkyl, halogen or CN; and s is 0, 1 or 2.

Embodiment 12C. A compound of Formula **1** wherein $R^7$ is

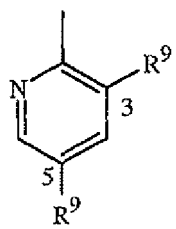

Embodiment 12D. A compound of Formula **1** wherein each $R^9$ is independently H, $CH_3$, $CF_3$, CN or halogen.

Embodiment 13A. A compound of Formula **1** wherein $R^8$ is $C_1$-$C_4$ alkyl or $C_1$-$C_4$ haloalkyl.

Embodiment 13B. A compound of Formula **1** wherein $R^8$ is $CH_2CF_3$ or $CHF_2$.

Embodiment 14A. A compound of Formula **1** wherein J is phenyl optionally substituted with one to four $R^5$.

Embodiment 14B. A compound of Formula **1** wherein each $R^5$ is independently $C_1$-$C_4$ alkyl, $C_1$-$C_4$ haloalkyl, $C_1$-$C_2$ haloalkoxy, halogen or CN.

Embodiment 15A. A compound of Formula **1** wherein J is a heterocyclic ring selected from the group consisting of J-1, J-2, J-3, J-4, J-5, J-6, J-7 and J-8.

Embodiment 15B. A compound of Formula **1** wherein J is J-1, J-2, J-4, J-7 or J-8.

Embodiment 15C. A compound of Formula **1** wherein J is J-1, J-2 or J-4.

Embodiment 15D. A compound of Formula **1** wherein J is J-7 or J-8.

Embodiment 15E. A compound of Formula **1** wherein J is J-1.

Embodiment 15F. A compound of Formula **1** wherein J is J-2.
Embodiment 15G. A compound of Formula **1** wherein J is J-3.
Embodiment 15H. A compound of Formula **1** wherein J is J-4.
Embodiment 15I. A compound of Formula **1** wherein J is J-5.
Embodiment 15J. A compound of Formula **1** wherein J is J-6.
Embodiment 15K. A compound of Formula **1** wherein J is J-7.
Embodiment 15L, A compound of Formula **1** wherein J is J-8.

Embodiments of this invention, including Embodiments 1A-15L above as well as any other embodiments described herein, can be combined in any manner, and the descriptions of variables in the embodiments pertain not only to the compounds of Formula **1** but also to the starting compounds and intermediate compounds, including the compounds of Formula **10,** useful for preparing the compounds of Formula **1.** In addition, embodiments of this invention, including Embodiments 1A-15L above as well as any other embodiments described herein, and any combination thereof, pertain to the compositions, mixtures and methods of the present invention which can comprise the compounds described in such embodiment and any combination thereof.

Examples of combinations of Embodiments 1A-15L include:

Embodiment A. A compound of Formula **1** wherein

$R^{1a}$ is $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
$_R$1b is H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
$R^2$ and $R^3$ are each independently H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and
$R^4$ is $C_4$-$C_{12}$ alkylcycloalkyl or $C_4$-$C_{12}$ cycloalkylalkyl, each optionally substituted with one to six substituents selected from $CH_3$ and halogen; or
$R^4$ is $C_3$-$C_5$ oxiranylalkyl, $C_4$-$C_6$ oxetanylalkyl or 3-oxetanyl, each optionally substituted with 1 to 2 substituents independently selected from $CH_3$, $CF_3$, halogen, CN and $C(O)OCH_3$,

Embodiment A1. A compound of Formula 1 wherein

$R^{1a}$is $C_1$-$C_4$ alkyl, $C_1$-$C_4$ haloalkyl, halogen, CN, $NO_2$ $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
$R^{1b}$ is H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
$R^2$ and $R^3$ are each independently H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and
$R^4$ is ($C_1$-$C_8$)alkyl($C_3$-$C_4$)Cycloalkyl or ($C_3$-$C_4$)cycloalkyl($C_1$-$C_8$)alkyl, each optionally substituted with one to six substituents selected from $CH_3$ and halogen; or
$R^4$ is $C_3$-$C_5$ oxiranylalkyl, $C_4$-$C_6$ oxetanylalkyl or 3-oxetanyl, each optionally substituted with 1 to 2 substituents independently selected from $CH_3$, $CF_3$, halogen, CN and $C(O)OCH_3$.

Embodiment B. A compound of Embodiments A or A1 wherein

$R^{1a}$ is $CH_3$, $CF_3$, $OCF_3$, $OCHF_2$, $S(O)_nCF_3$, $S(O)_nCHF_2$, CN or halogen;
$R^{1b}$ is H, $CH_3$, $CF_3$, $OCF_3$, $OCHF_2$, $S(O)_pCF_3$, $S(O)_pCHF_2$, CN or halogen;
$R^2$ and $R^3$ are H;
n is 0,1 or 2; and
pis 0,1 or 2.

Embodiment C. A compound of Embodiment B wherein

each $R^5$ is independently $C_1$-$C_4$ alkyl, $C_1$-$C_4$ haloalkyl, $C_1$-$C_2$ haloalkoxy, halogen or CN;
each $R^6$ is independently H, $CH_3$, $CF_3$, $CH_2CF_3$, $CHF_2$, $OCH_2CF_3$, $OCHF_2$ or halogen;
$R^7$ is phenyl optionally substituted with one to three substituents selected from $R^9$; or $R^7$ is

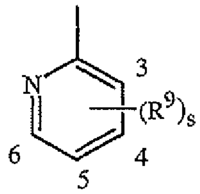

;

each $R^9$ is independently $C_1$-$C_4$ alkyl, $C_1$-$C_4$ haloalkyl, halogen or CN;
$R^8$ is $CH_2CF_3$ or $CHF^2$; and
s is 0,1 or 2.

Embodiment D. A compound of Embodiment C wherein

each $R^6$ is independently halogen, $OCH_2CF_3$, $OCHF_2$ or $CF_3$;
$R^7$ is

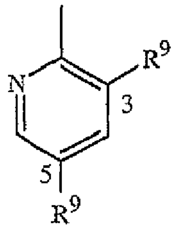

or

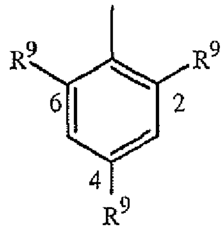

;

and
each $R^9$ is independently H, $CH_3$,$CF_3$, CN or halogen.

Embodiment E. A compound of Embodiment D wherein J is J-1, J-2, J-4, J-7 or J"8.
Embodiment F. A compound of Embodiment E wherein

$R^{1a}$ is $CH_3$, F, Cl, Br or I;
$R^{1b}$ is H, $CH_3$, $CF_3$, CN, F, Cl, Br or I; and
each $R^6$ is independently Cl, Br, $OCH_2CF_3$ or $CF_3$.

Embodiment G. A compound of Embodiment F wherein

J is J-2, J-4, J-7 or J-8; and
$R^4$ is 1-methylcyclopropyl, 1-methylcyclobutyl, cyclopropylmethyl or cyclobutylmethyl; each optionally substituted with one to four $CH_3$ or halogen; or
$R^4$ is oxiranylmethyl, 2-oxetanylmethyl, 3-oxetanylmethyl or 3-oxetanyl, each optionally substituted with 1 to 2 $CH_3$.

Embodiment H. A compound of Embodiment F wherein

J is J-1; and
$R^4$ is 1-methylcyclopropyl, 1-methylcyclobutyl, cyclopropylmethyl or cyclobutylmethyl, each optionally substituted with one to four $CH_3$ or halogen; or
$R^4$ is oxiranylmethyl, 2-oxetanylmethyl, 3-oxetanylmethyl or 3-oxetanyl, each optionally substituted with 1 to 2 $CH_3$;

provided that when $R^4$ is 1-methylcyclopropyl, then $R^{1b}$ is other than H,

Specific embodiments include compounds of Formula **1** selected from the group consisting of:

1-(3-chloro-2-pyridinyl)-*N*-[4-cyano-2-[[(cyclopropylmethyl)amino]carbonyl]-6-methylphenyl]-3-(trifluoromethyl)-1*H*-pyrazole-5-carboxamide;
3-bromo-*N*-[4-chloro-2-[[(cyclopropylmethyl)amino]carbonyl]-6-methylphenyl]-1-(3-chloro-2-pyridinyl)-1*H*-pyrazole-5-carboxamide;
3-bromo-1-(3-chloro-2-pyridinyl)-*N*-[2,4-dichloro-6-[[(2-oxetanylmethyl)amino]carbonyl]phenyl]-1*H*-pyrazole-5-carboxamide;
3-bromo-*N*-[4-chloro-2-methyl-6-[[(2-oxetanylmethyl)amino]carbonyl]phenyl]-1-(3-chloro-2-pyridinyl)-1*H*-pyrazole-5-carboxamide;
3-chloro-1-(3-chloro-2-pyridinyl)-*N*-[2,4-dichloro-6-[[(2-oxetanylmethyl)amino]carbonyl]phenyl]-1*H*-pyrazole-5-carboxamide;
1-(2-chlorophenyl)-*N*-[4-cyano-2-methyl-6-[[(2-oxetanylmethyl)amino]carbonyl]phenyl]-3-(trifluoromethyl)-1*H*-pyrazole-5-carboxamide;
3-bromo-1-(3-chloro-2-pyridinyl)-*N*-[4-cyano-2-methyl-6-[[(2-oxetanylmethyl)amino]carbonyl]phenyl]-1*H*-pyrazole-5-carboxamuide;
3-bromo-*N*-[4-chloro-2-methyl-6-[[(1-methylcyclopropyl)amino]carbonyl]phenyl]-1-(3-chloro-2-pyridinyl)-1*H*-pyrazole-5-carboxamide;
3-bromo-1-(3-chloro-2-pyridinyl)-*N*-[2,4-dichloro-6-[[(1-methylcyclopropyl)amino]carbonyl]phenyl]-1*H*-pyrazole-5-carboxamide;
3-bromo-1-(3-chloro-2-pyridinyl)-*N*-[4-cyano-2-methyl-6-[[(1-methylcyclopropyl)amino]carbonyl]phenyl]-1*H*-pyrazole-5-carboxamide;
3-bromo-1-(2-chlorophenyl)-*N*-[4-cyano-2-[[(cyclopropylmethyl)amino]carbonyl]-6-methylphenyl]-1*H*-pyrazole-5-carboxamide;
3-bromo-*N*-[4-chloro-2-[[(cyclopropylmethyl)amino]carbonyl]-6-methylphenyl]-1-(2-chlorophenyl)-1*H*-pyrazole-5-carboxamide;
3-bromo-*N*-[4-chloro-2-[[(1-cyclopropylethyl)amino]carbonyl]-6-methylphenyl]-1-(3-chloro-2-pyridinyl)-1*H*-pyrazole-5-carboxamide;
3-bromo-*N*-[4-chloro-2-[[(1-cyclopropylethyl)amino]carbonyl]-6-methylphenyl]-1-(2-chlorophenyl)-1*H* -pyrazole-5-carboxamide; and
3-bromo-1-(3-chloro-2-pyridinyl)-*N*-[4-cyano-2-[[(1-cyclopropylethyl)amino]carbonyl]-6-methylphenyl]-1*H*-pyrazole-5-carboxamide.

Further specific embodiments include any combination of the compounds of Formula 1 selected from the group immediately above.

Also noteworthy as embodiments of the present invention are compositions comprising a compound of any of the preceding Embodiments, as well as any other embodiments described herein, and any combinations thereof, and at least one additional component selected from the group consisting of a surfactant, a solid diluent and a liquid diluent, said composition optionally further comprising at least one additional biologically active compound or agent.

Also noteworthy as embodiments of the present invention are compositions for controlling an invertebrate pest comprising a biologically effective amount of a compound of any of the preceding Embodiments, as well as any other embodiments described herein, and any combinations thereof, and at least one additional component selected from the group consisting of a surfactant, a solid diluent and a liquid diluent, said composition optionally further comprising a biologically effective amount of at least one additional biologically active compound or agent. Embodiments of the invention further include methods for controlling an invertebrate pest comprising contacting the invertebrate pest or its environment with a biologically effective amount of a compound of any of the preceding Embodiments, as well as any other embodiments described herein, and any combinations thereof (e.g., as a composition described herein).

Embodiments of the invention also include a composition comprising a compound of any of the preceding Embodiments, as well as any other embodiments described herein, and any combinations thereof, in the form of a soil drench liquid formulation. Embodiments of the invention further include methods for controlling an invertebrate pest comprising contacting the soil with a liquid composition as a soil drench comprising a biologically effective amount of a compound of any of the preceding Embodiments, as well as any other embodiments described herein, and any combinations thereof.

Embodiments of the invention also include a spray composition for controlling an invertebrate pest comprising a biologically effective amount of a compound of any of the preceding Embodiments, as well as any other embodiments described herein, and any combinations thereof and a propellant. Embodiments of the invention further include a bait composition for controlling an invertebrate pest comprising a biologically effective amount of a compound of any of the preceding Embodiments, as well as any other embodiments described herein, and any combinations thereof, one or more food materials, optionally an attractant, and optionally a humectant. Embodiments of the invention also include a device for controlling an invertebrate pest comprising said bait composition and a housing adapted to receive said bait composition, wherein the housing has at least one opening sized to permit the invertebrate pest to pass through the opening so the invertebrate pest can gain access to said bait composition from a location outside the housing, and wherein the housing is further adapted to be placed in or near a locus of potential or known activity for the invertebrate pest.

Of note is a compound of Formula 1 wherein the cycloalkyl or cycloalkenyl moiety of the $R^4$ substituent is a $C_3$-$C_4$ carbocyclic ring. Accordingly in $R^4$ of said compound, "$C_4$-$C_{12}$ alkylcycloalkyl" consists of "($C_1$-$C_8$ alkyl)($C_3$-$C_4$ cycloalkyl)", "$C_5$-$C_{12}$ alkenylcycloalkyl" consists of "($C_2$-$C_8$ alkenyl)($C_3$-$C_4$ cycloalkyl)", "$C_5$-$C_{12}$ alkynylcycloalkyl" consists of "($C_2$-$C_8$ alkynyl)($C_3$-$C_4$ cycloalkyl)", "$C_4$-$C_{12}$ cycloalkylalkyl" consists of "($C_3$-$C_4$ cycloalkyl)($C_1$-$C_8$ alkyl)", "$C_5$-$C_{12}$ cycloalkylalkenyl" consists of "($C_3$-$C_4$ cycloalkyl)($C_2$-$C_8$ alkenyl)", "$C_5$-$C_{12}$ cycloalkylalkynyl" consists of "($C_3$-$C_4$ cycloalkyl)($C_2$-$C_8$ alkynyl,)", "$C_4$-$C_{12}$ cycloalkenylalkyl" consists of "($C_3$-$C_4$ cycloalkenyl)($C_1$-$C_8$ alkyl)" and "$C_4$-$C_{12}$ alkylcycloalkenyl" consists of "($C_1$-$C_8$ alkyl)($C_3$-$C_4$ cycloalkenyl)".

Of note is a compound of Formula **1** other than 3-bromo-*N*-[4-chloro-2-[[(cyclopropylmethyl)amino]carbonyl]-6-methylphenyl]"1-(3-chloro-2-pyridinyl)-1*H*-pyrazole-5-carboxamide.

Of note is a mixture comprising 3-bromo-*N*-4-chloro-2-[[(cyclopropylmethyl)amino]-carbonyl]-6-methylphenyl]-1-(3-chloro-2-pyridinyl)-1*H*-pyrazole-5-carboxamide, and at least one additional biologically active compound or agent. Of particular note is a synergistic mixture comprising 3-bromo-*N*-[4-chloro-2-[[(cyclopropylmethyl)amino]-carbonyl]-6-methylphenyl]-1-(3-chloro-2-pyridinyl)-1*H*-pyrazole-5-carboxamide, and at least one additional biologically active compound or agent. Of further note is a synergistic mixture comprising 3-bromo-*N*-[4-chloro-2-[[(cyclopropylmethyl) amino]carbonyl]-6-methylphenyl]-1-(3-chloro-2-pyridinyl)-1*H*-pyrazole-5-carboxamide, and imidacloprid or thiamethoxam.

Of note is a compound of Formula **1** other than 3-bromo-*N*-[4-chloro-2-[[(1-cyclopropylethyl)amino]carbonyl]-6-mothylphenyl]-1-(3-chloro-2-pyridinyl)-1*H*-pyrazole-5-carboxamide.

Of note is a mixture comprising 3-bromo-*N*-[4-chloro-2-[[(1-cyclopropylethyl)amino]-carbonyl]-6-methylphenyl]-1-(3-chloro-2-pyridinyl)-1*H*-pyrazole-5-carboxamide, and at least one additional biologically active compound or agent.

Of note is a compound of Formula **1,** or an *N*-oxide thereof, wherein when J is J-1, $R^6$ is $CF_3$, $R^7$ is 3-chloro-2-pyridinyl, $R^2$ and $R^3$ are H, $R^{1a}$ is Me, and $R^{1b}$ is H or Cl, then $R^4$ is other than cyclopropylmethyl.

Of note is a mixture comprising a compound of Formula **1,** or an *N*-oxide thereof, wherein J is J-1, $R^6$ is $CF_3$, $R^7$ is 3-chloro-2-pyridinyl, $R^2$ and $R^3$ are H, $R^{1a}$ is Me, $R^{1b}$ is H or Cl, and $R^4$ is cyclopropylmethyl, and at least one additional biologically active compound or agent.

Of note is a compound of Formula **1** wherein when J is J-1, $R^6$ is $CF_3$, $R^7$ is 3-chloro-2-pyridinyl, $R^2$ and $R^3$ are H, $R^{1a}$ is Me, and $R^{1b}$ is Cl, then $R^4$ is other than (2-methylcyclopropyl)methyl, (2,2-dichloro-1-methylcyclopropyl)methyl, (1-methyl-cyclopropyl)methyl or 1-cyclobutylethyl.

Of note is a mixture comprising a compound of Formula 1 wherein J is J-1, $R^6$ is $CF_3$, $R^7$ is 3-chloro-2-pyridinyl, $R^2$ and $R^3$ are H, $R^{1a}$ is Me, $R^{1b}$ is Cl, and $R^4$ is (2-methylcyclopropyl)methyl, (2,2-dichloro-1-methylcyclopropyl)methyl, (1-methylcyclopropyl)methyl or 1-cyclobutylethyl, and at least one additional biologically active compound or agent.

Of note is a compound of Formula **1** wherein when J is J-1, $R^6$ is Br, Cl, $CF_3$ or $OCH_2CF_3$, $R^7$ is 2-pyridinyl optionally substituted with halogen at positions 3 and/or 5 of the pyridinyl ring, $R^2$ and $R^3$ are H, $R^4$ is 1-cyclopropylethyl, and $R^{1a}$ is Me, Et, halogen, $CF_3$, $CHF_2$ or $OCHF_2$, then $R^{1b}$ is other than H, halogen, $CF_3$, $CRF_2$, $NO_2$, OMe, $CH=CH_2$, $CH=CCl_2$, C=CH, C=Cl, $C(O)CH_3$, $C(O)CF_3$, C(O)OMe or C(O)O*i*-Pr,

Of note is a mixture comprising a compound of Formula **1** wherein J is J-1, $R^6$ is Br, Cl, $CF_3$ or $OCH_2CF_3$, $R^7$ is 2-pyridinyl optionally substituted with halogen at positions 3 and/or 5 of the pyridinyl ring, $R^2$ and $R^3$ are H, $R^4$ is 1-cyclopropylethyl, $R^{1a}$ is Me, Et, halogen, $CF_3$, $CHF_2$ or $OCHF_2$, and $R^{1b}$ is H, halogen, $CF_3$, $CHF_2$, $NO_2$, OMe, $CH=CH_2$, $CH=CCl_2$, $C\equiv CH$, $C\equiv CI$, $C(O)CH_3$, $C(O)CF_3$, C(O)OMe or C(O)O*i*-Pr, and at least one additional biologically active compound or agent.

Of note is a compound of Formula **1,** or an *N*-oxide thereof, wherein when J is J-1, $R^7$ is optionally substituted 2-pyridinyl, $R^{1a}$ is Me, Et, halogen, $CF_3$, $CBF_2$ or $OCHF_2$, and $R^2$ and $R^3$ are H, then $R^4$ is other than cyclopropylmethyl, 1-cyclopropylethyl, (2-methylcyclopropyl)methyl, (2,2-dichloro-1-methylcyclopropyl)methyl, (1-methyleyelopropyl)-methyl or 1-cyclobutylethyl.

Of note is a mixture comprising a compound of Formula **1,** or an *N*-oxide thereof, wherein J is J-1, $R^7$ is optionally substituted 2-pyridinyl, $R^{1a}$ is Me, Et, halogen, $CF_3$, $CHF_2$ or $OCBF_2$, $R^2$ and $R^3$ are H, and $R^4$ is cyclopropylmethyl, 1-cyclopropylethyl, (2-methylcyclopropyl)methyl, (2,2-dichloro-1-methylcyclopropyl)methyl, (1-methylcyclopropyl)-methyl or 1-cyclobutylethyl, and at least one additional biologically active compound or agent.

Of note is a compound of Formula **1**, or an *N*-oxide thereof, wherein when J is J-1, $R^7$ is optionally substituted 2-pyridinyl, and $R^2$ and $R^3$ are H, then $R^4$ is other than cyclopropylmethyl, 1-cyclopropylethyl (2-methylcyclopropyl) methyl, (2,2-dichloro-1-methylcyclopropyl)methyl, (1-methylcyclopropyl)methyl or 1-cyclobutylethyl.

Of note is a mixture comprising a compound of Formula 1, or an *N*-oxide thereof, wherein J is J-1, $R^7$ is optionally substituted 2-pyridinyl, $R^2$ and $R^3$ are H, and $R^4$ is cyclopropylmethyl, 1-cyclopropylethyl (2-methylcyclopropyl)methyl, (2,2-dichloro-1-methylcyclopropyl)methyl, (1-methylcyclopropyl)methyl or 1-cyclobutylethyl, and at least one additional biologically active compound or agent.

Of note is a compound of Formula **1**, or an *N*-oxide thereof, wherein when J is J-1, $R^7$ is optionally substituted 2-pyridinyl, and $R^2$ and $R^3$ are H, then $R^4$ is other than optionally substituted ($C_3$-$C_4$ cycloalkyl)($C_1$-$C_6$ alkyl).

Of note is a mixture comprising a compound of Formula **1,** or an *N*-oxide thereof, wherein J is J-1, $R^7$ is optionally substituted 2-pyridinyl, $R^2$ and $R^3$ are H, and $R^4$ is optionally substituted ($C_3$-$C_4$ cycloalkyl)($C_1$-$C_6$ alkyl), and at least one additional biologically active compound or agent.

Of note is a mixture comprising 3-bromo-1-(3-chloro-2-pyridinyl)-*N*-[4-cyano-2-[[(cyclopropylmethyl)amino]-carbonyl]-6-methylphenyl]-1*H*-pyrazole-5-carboxamide, and at least one additional biologically active compound or agent. Of particular note is a synergistic mixture comprising 3-bromo-1-(3-chloro-2-pyridinyl)-*N*-[4-cyano-2-[[(cyclopropylmethyl)amino]-carbonyl]-6-methylphenyl]-1*H*-pyrazole-5-carboxamide, and at least one additional biologically active compound or agent. Of further note is a synergistic mixture comprising 3-bromo-1-(3-chloro-2-pyridinyl)-*N*-[4-cyano-2-[[(cyclopropylmethyl)-amino]carbonyl]-6-methylphenyl]-1*H*-pyrazole-5-carboxamide, and imidacloprid or thiamethoxam.

Of note is a compound of Formula 1 wherein when $R^{1a}$ is Me, Cl, Br or F, $R^{1b}$ is CN, $R^2$ is H, $R^3$ is H or Me, J is J-1, $R^6$ is F, Cl, Br, $C_1$-$C_4$ haloalkyl or $C_1$-$C_4$ haloalkoxy, $R^7$ is 2-pyridinyl substituted with F, Cl or Br as $R^{9a}$ at position 3 and unsubstituted at position 5 or substituted with F or Cl as $R^{9b}$ at position 5 of the pyridinyl ring, then $R^4$ is other than $C_4$-$C_6$ cycloalkylalkyl.

Of note is a compound of Formula 1 wherein when $R^{1b}$ is CN, J is J-1, $R^7$ is optionally substituted 2-pyridinyl, $R^2$ is H and $R^3$ is H or Me, then $R^4$ is other than $C_4$-$C_6$ cycloalkylalkyl.

Of note that a compound of Formula 1 wherein when $R^{1b}$ is CN, J is J-1, and $R^7$ is optionally substituted 2-pyridinyl, then $R^4$ is other than $C_4$-$C_6$ cycloalkylalkyl.

Of note are compounds of Embodiments 1A through 2J, 3A through 5D; 6A-6B; 7A-7U; 10A-10D; 11A-11D; 12A-12D; 13A-13B; 14A-14B; 15A-15L; and any combination of the foregoing, which are compounds of Embodiment 8J and/or Embodiment 8K. Also of note is a combination of the compounds of Embodiments 8J and 8K and the compound 3-bromo-1-(3-chloro-2pyridinyl)-*N*-[4-cyano-2-[[cyclopropylmethyl)amino]carbonyl]-6-methylphenyl]-1*H*-pyrazole-5-carboxamide.

Compounds of Formula **1** can be prepared by one or more of the following methods and variations as described in Schemes 1-8. The definitions of J, $R^{1a}$, $R^{1b}$, $R^2$, $R^3$ and $R^4$ in the compounds of Formulae **1-11** below are as defined above in the Summary of the Invention. Formula **3a** is a subset of Formula **3,** likewise Formula **10a** is a subset of Formula **10,** and Formula **1a** is a subset of Formula **1.**

Compounds of Formula **1** can be prepared by the reaction of benzoxazinones of Formula **2** with amines of Formula **3** as outlined in Scheme 1.

Scheme 1

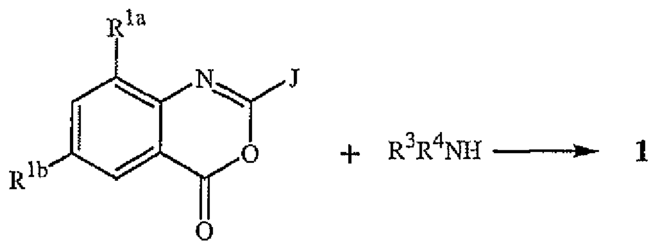

**2** **3**

The reaction can be run neat or in a variety of suitable solvents including tetrahydrofuran, diethyl ether, dichloromethane, chloroform or lower alcohols such as methanol or ethanol with optimum temperatures ranging from room temperature to the reflux temperature of the solvent. The general reaction of benzoxazinones with amines to produce anthranilamides is well documented in the chemical literature. For a review of benzoxazinone chemistry see Jakobsen et al., Bioorganic and Medicinal Chemistry 2000, 8, 2095-2103 and references cited within. See also G. M. Coppola, J. Heterocyclic Chemistry 1999,36,563-588.

Benzoxazinones of Formula **2** can be prepared by a variety of methods. Three methods that are especially useful are detailed in Schemes 2-4. In Scheme 2, a benzoxazinone of Formula **2** is prepared directly via coupling of a carboxylic acid of Formula **4** with an anthranilic acid of Formula **5.**

Scheme 2

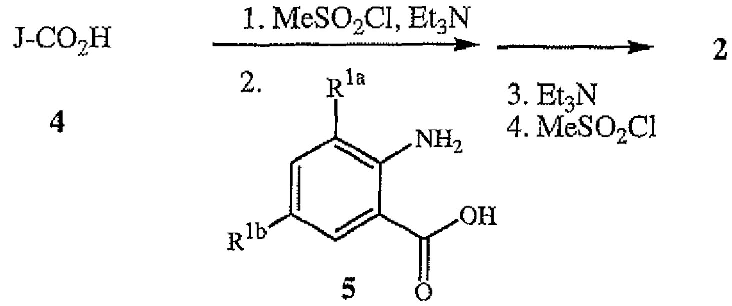


This involves sequential addition of methanesulfonyl chloride in the presence of a tertiary amine such as triethylamine to a pyrazolecarboxylic acid of Formula **4,** followed by the addition of the anthranilic acid of Formula **5,** followed by a second addition of triethylamine and methanesulfonyl chloride. This method generally affords good yields of the benzoxazinone.

Scheme 3 depicts an alternate preparation for benzoxazinones of Formula **2** involving coupling of an acid chloride of Formula **7** with an isatoic anhydride of Formula **6** to provide the Formula **2** benzoxazinone directly. Solvents such as pyridine or pyridine/acetonitrile are suitable for this reaction. The acid chlorides of Formula **7** are available from the corresponding acids of Formula **4** by known methods such as chlorination with thionyl chloride or oxalyl chloride.

Scheme 3

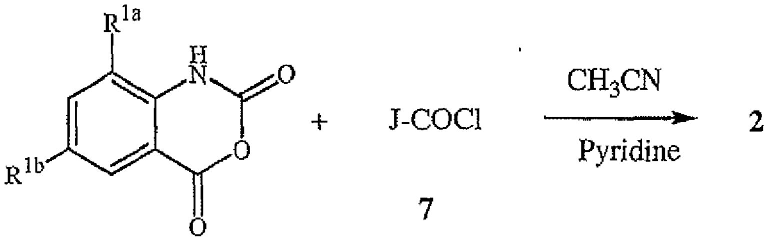


6

In Scheme 4, the benzoxazinone of Formula **2** is prepared directly via coupling of a carboxylic acid of Formula **4** with an anthranilic acid of Formula **5.** This involves sequential addition of a pyridine base such as 3-picoline to a mixture of the pyrazolecarboxylic acid of Formula **4** and the anthranilic acid of Formula **5,** followed by addition of methanesulfonyl chloride. This method affords very good yields of the benzoxazinone. For additional references related to the preparation of representative benzoxazinones of Formula **2** see PCT Patent Publications WO 2003/015519, 2004/011447 and 2004/067528. Anthranilic acids of Formula **5** are available commercially or by a variety of known methods.

Scheme 4

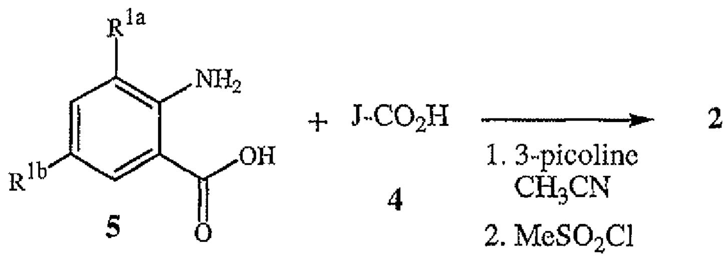


In Scheme 1, when the amine of Formula **3** is a primary amine ($R^3$ is H) and not commercially available, for example, 2-oxetanylmethyl amine, the amine of Formula **3** can be prepared by reacting the corresponding alcohol of Formula **8** with phthalimide by the Mitsunobu reaction to yield a compound of Formula **9** (Scheme 5). Treatment with hydrazine hydrate at high temperature in protic solvent such as ethyl alcohol yields the amine of Formula **3a.** For general reviews of a wide variety of methods known in the art for preparing amines, see Mitsunobu, O. Comprehensive Organic

Synthesis; Trost, B. M., Fleming, I., Eds.; Pergamon: Oxford, 1991; Vol. 6, pages 65-101. For a general review describing methods for preparing secondary amines, see Salvatore, R. N. et al. Tetrahedron 2001,57,7785-7811.

Scheme 5

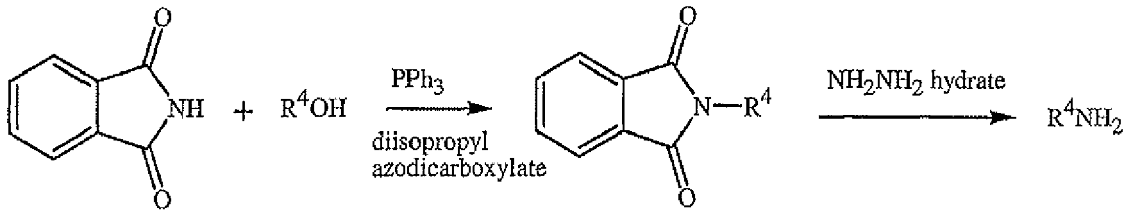


8 9 3a

An alternate method for the preparation of compounds of Formula **1** is depicted in Scheme **6.** In this process an amide of Formula **10** is coupled directly with an acid of Formula **4** to produce the anthranilamide of Formula **1.** This method involves addition of two or more equivalents of an amine base, such as pyridine or picoline, to an acid of Formula **4** followed by addition of a sulfonyl halide such as methanesulfonyl chloride. The amide of Formula 10 is then added resulting in a direct coupling to produce the anthranilamide of Formula 1.

Scheme 6

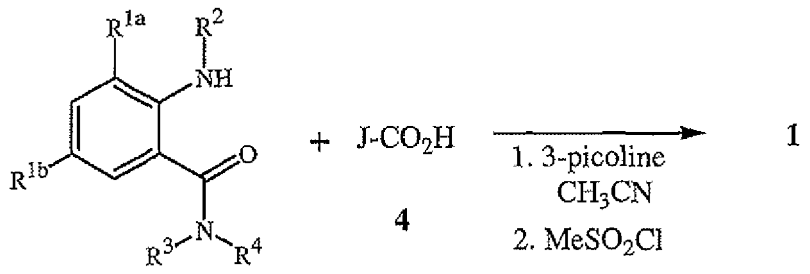


10

Following the procedures described for Scheme 6, a preferred set of amides of Formula 10a can be used to prepare a preferred set of anthranilamides of Formula la as shown in Scheme 7.

Scheme 7

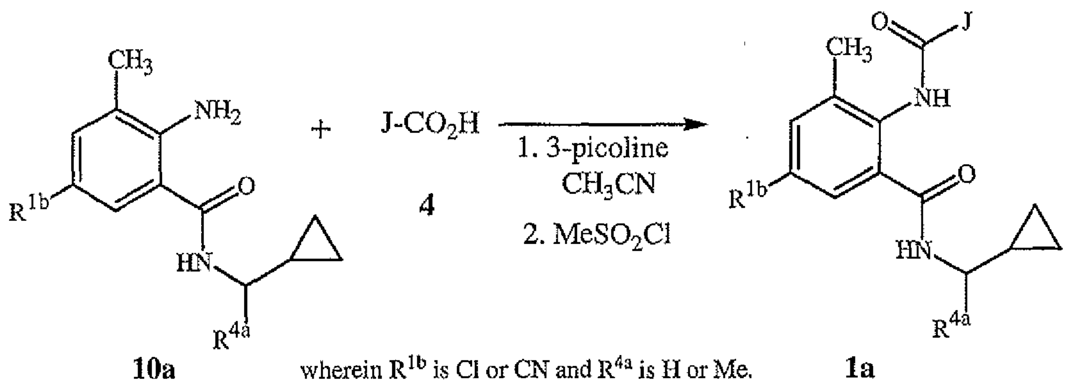


10a wherein $R^{1b}$ is Cl or CN and $R^{4a}$ is H or Me. 1a

Amides of Formula 10 may be prepared as shown in Scheme 8 by known methods involving reaction of the amine of Formula 3 with an isatoic anhydride of Formula 11.

Scheme 8

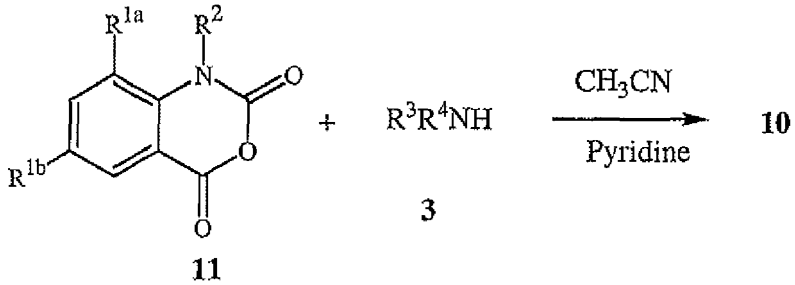


It is recognized that some reagents and reaction conditions described above for preparing compounds of Formula 1 may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts,P. G. M. Protective Groups in Organic Synthesis, 2nd ed.; Wiley; New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula **1.** One skilled in the art will also recognize that it may be necessary to perform a combination of the steps illustrated in the above schemes in an order other than that implied by the particular sequence presented to prepare the compounds of Formula **1**.

One skilled in the art will also recognize that compounds of Formula 1 and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. $^1H$ NMR spectra are reported in ppm downfield from tetramethylsilane; "s" means singlet, "d" means doublet, "t" means triplet, "q" means quartet, "m" means multiplet, "dd" means doublet of doublets, "dt" means doublet of triplets, "br s" means broad singlet and "br t" means broad triplet.

EXAMPLE 1

Preparation of 1-(3-chloro-2-pyridinyl)-*N*-14-cyano-2-[[(cyclopropylmethyl)amino]carbonyl]-6-methylphenyl]-3-(trifluoromethyl)-1*H*-pyrazole-5-carboxamide

2-[1-(3-Chloro-2-pyridinyl)-3-(trifluoromethyl)-1*H*-pyrazol-5-yl]-8-methyl-4-oxo-4*H*-3,1-benzoxazine-6-carbonitrile (150 mg, 0.35 mmol), prepared by the procedure described in PCT Patent Publication WO2004/067528, in acetonitrile (10 mL) was combined with cyclopropylmethylamine hydrochloride (112 mg, 1.0 mmol) and triethylamine (0.145 mL, 1.0 mmol). The resulting solution was heated at reflux for several minutes and then stirred at ambient temperature for 15 minutes. Water was added (10 mL) and the mixture was cooled to 0 °C to precipitate a solid. The solid was collected by filtration and washed successively with water and ether/hexane to yield the title compound as a white solid (139 mg), m.p. 235-236 °C.

$^1H$ NMR ($CDCl_3$) $\delta$ 10.7 (br s, 1H); 8.50 (d, 1H), 7.90 (d, 1H), 7.63 (s, 1H), 7.61 (s, 1H) 7.43 (dd, 1H), 7.28 (s, 1H), 6.35 (br t, 1H), 3.29 (dd, 2H), 2.26 (s, 3H), 1.04 (m, 1H), 0.60 (m, 2H), 0.28 (m, 2H).

EXAMPLE 2

Preparation of 3-bromo-*N*-chloro-2-[[(cyclopropylmethyl)amino]carbonyl]-6-methylphenyl]-1-(3-chloro-2-pyridinyl)-1*H*-pyrazole-5-carboxamide

2-[3-bromo-1-(3-chloro-2-pyridinyl)-1*H*-pyrazole-5-yl]-6-chloro-8-methyl-4*H*-3,1-benzoxazin-4-one (157 mg, 0.35 mmol), prepared by the procedure described in PCT Patent Publication WO2003/015519, in acetonitrile (10 mL) was combined with cyclopropylmethylamine hydrochloride (112 mg, 1.0 mmol) and triethylamine (0.145 mL, 1.0 mmol). The resulting solution was heated at reflux for several minutes and then stirred at ambient temperature for 15 minutes. Water was added (10 mL) and the mixture was cooled to 0 °C to precipitate a solid. The solid was collected by filtration and washed successively with water and ether/hexane to yield 170 mg of the title compound as a white solid, m.p. 172-173 °C.

$^1H$ NMR ($CDCl_3$) δ 10,1 (br s, 1H), 8.46 (d, 1H), 7.85 (d, 1H), 7.40 (dd, 1H), 7.26 (s, 2H), 7.07 (s, 1H), 6.23 (br t, 1H), 3.25 (dd, 2H), 2.19 (s, 3H), 1.0 (m, 1H), 0.58 (m, 2H) 0.26 (m, 2H).

EXAMPLE 3

Preparation of 3-bromo-*N*-[4-chloro-2-methyl-6-[[(2-oxetanylmethyl)amino]carbonyl]phenyl]-1-(3-chloro-2-pyridinyl)-1*H*-pyrazole-5-carboxamide

Step A: Preparation of 2-(2-oxetanylmethyl)-1*H*-isoindole-1,3(2*H*)-dione

2-Hydroxymethyloxetane (0.250 g, 2.84 mmol), phthalimide (0.501 g, 3.4 mmol) and triphenylphosphine (0.892 g, 3.4 mmol) were dissolved in tetrahydrofuran. Diisopropyl azodicarboxylate (0.659 mL, 3.4 mmol) was then added over approximately 5 minutes, and the solution was stirred at room temperature for two hours. The reaction mixture was concentrated under reduced pressure and purified via medium pressure liquid chromatography (ethyl acetate/hexane gradient) to yield the title compound (0.485 g) as a light yellow solid.
$^1H$ NMR ($CDCl_3$ δ 7.86 (m 2H), 7.72 (m 2H), 5.06 (m, 1H), 4.62 (m, 2H), 4.08 (m 1H), 3.92 (m 1H), 2.73 (m, 1H), 2.54 (m, 1H).

Step B: Preparation of 3-bromo-*N*-[4-chloro-2methyl-6-[[(2-oxetanylmethyl)amino]carbonyl]phenyl)-1-(3-chloro-2-pyridinyl)-1*H*-pyrazole-5-carboxamide

To a solution of 2-(2-oxetanylmethyl)-1*H*-isoindole-1,3(2*H*)-dione (i.e. the product from Step A) (0.150 g, 0.691 mmol) in ethanol (10 mL) was added hydrazine hydrate (0.035 g, 0.691 mmol). The reaction mixture was refluxed for 16 hours. The resulting mixture was filtered through a sintered glass frit funnel directly into a flask containing a solution of 2-[3-bromo-1-(3-chloro-2-pyridinyl)-1*H*-pyrazole-5-yl]-6-chloro-8-methyl-4*H*-3,1-benzoxazin-4-one (0,312 g, 0.691 mmol), prepared by the procedure described in PCT publication WO2003/015519,in dichloromethane (10 mL). The reaction mixture was stirred at room temperature for 24 hours. The reaction was concentrated under reduced pressure and the crude product was purified by medium pressure liquid chromatography on silica gel (eluted with ethyl acetate-hexanes gradient) to afford the title compound, a compound of the present invention, as a white solid (0.196 g), m.p, 95-97 °C.
$^1H$ NMR ($CDCl_3$) δ 10.1 (br s, 1H), 8.43 (m 1H), 7.82 (m 1H), 7.35 (m 1H), 7.23 (m, 2H), 7.09 (br s, 1H), 6.84 (m 1H), 4.92 (m, 1H), 4.64 (m 1H), 4.44 (m, 1H), 3.67 (m, 1H), 3.53 (m, 1H), 2.65 (m, 1H), 2.41 (m, 1H), 2.14 (s, 3H).

EXAMPLE 4

Alternative preparation of 3-bromo-*N*-[4-chloro-2-[[(cyclopropylmethyl)amino]carbonyl]-6-methylphenyl]-1-(3-chloro-2-pyridinyl)-1*H*-pyrazole-5-carboxamide

Step A: Preparation of 2-amino-5-chloro-*N*-(cyclopropylmethyl)-3-methylbenzamide

A solution of 6-chloro-8-methyl-2*H*-3,1-benzoxazine-2,4(1*H*)-dione (1.0 g, 4.74 mmol), prepared by the procedure described in PCT Patent Publication WO2003/015519, in ethyl acetate (300 mL) was heated to reflux to dissolve most of the solids. The resulting solution was cooled to room temperature and cyclopropylmethylamine (0.61 mL, 7.1 mmol) was added. The mixture was stirred at room temperature overnight. The precipitated solid was filtered and discarded. The filtrate was concentrated to dryness. The residual solid was rinsed with hexane, collected by filtration, and dried to yield the title compound as a white solid (0.74 g), m.p. 127-128 °C.
$^1H$ NMR (DMSO-$d_6$) δ 8.46 (br t, 1H), 7.43 (s, 1H), 7.12 (s, 1H), 6.33 (b s, 2H), 3.08 (t, 2H) 2.08 (s, 3H), 1.00 (m, 1H), 0.42 (dd, 2H), 0.21 (dd, 2H).

Step B; Preparation of 3-bromo-*N*-[4-chloro-2-[[(cyclopropylmethyl)amino]-carbonyl]-6-methylphenyl]-1-(3-chloro-2-pyridinyl)-1*H*-pyrazole-5-carboxamide

To a solution of 3-bromo-1-(3-chloro-2-pyridinyl)-1*H*-pyrazole-5-carboxylic acid (0.2 g, 0.66 mmol), prepared by the procedure described in PCT Patent Publication WO2003/015519, in acetonitrile (20 mL) was added 3-picoline (0.161 mL, 1.66 mmol) followed by methanesulfonyl chloride (0.054 mL, 0.70 mmol), and the mixture was then stirred at room temperature for 10 minutes. After this time 2-amino-5-chloro-*N*-(cyclopropylmethyl)-3-methylbenzamide (0.158 g, 0,66 mmol) was added and the mixture was stirred at room temperature for 2 hours, The reaction mixture was diluted with ethyl acetate and washed with 1 N HCl followed by saturated aqueous NaCl. The organic phase was dried over magnesium sulfate and concentrated. The residual solids were purified by chromatography on silica gel to afford the

title compound, a compound of the present invention, as a white solid (0.100 g), m.p. 166-168 °C. Spectral data was consistent with that of Example 2.

EXAMPLE 5

Preparation of 3-bromo-*N*-[4-chloro-2-[[(1-cyclopropylethyl)amino]carbonyl]-6-methylphenyl]-1-(3-chloro-2-pyridinyl)-1*H*-pyrazole-5-carboxamide

Step A: Preparation of 1-cyclopropylethanone oxime

A mixture of 1-cyclopropylethanone (Aldrich, 6.55 g, 78 mmol), hydroxylamine hydrochloride (7.86 g, 113.1 mmol) and sodium acetate (9.92 g, 121.7 mmol) in ethanol (50 mL) was heated at reflux for 16 hours. The reaction was then partitioned between aqueous sodium bicarbonate and ethyl acetate. The organic solution was washed with water, dried over magnesium sulfate and filtered. The filtrate was concentrated to afford the title compound (5.8 g) as clear colorless oil. $^1$H NMR indicated a mixture of *E* and Z isomers.
$^1$H NMR ($CDCl_3$) $\delta$ 8.9 (br s, 1H), 2.44 and 1.60 (2 m, 1H), 1.72 and 1.55 (2 s, 3H), 0.85 and 0.74 (2 m, 4H).

Step B: Preparation of $\alpha$-methylcyclopropanemethanamine

To a solution of 1-cyclopropylethanone oxime (i.e. the product of Step A, 0.5 g, 5.0 mmol) in diethyl ether (10 mL) was added a 1.0 M solution of lithium aluminum hydride in diethyl ether (5.0 mL, 5.0 mmol), and the reaction mixture was stirred at room temperature for 30 minutes. The mixture was then heated to reflux for an additional 8 hours. The reaction mixture was cooled and quenched by successive dropwise addition of water (1.0 mL), 15% aqueous NaOH, (1.0 mL) and water (3.0 mL). The ether layer was decanted from the aqueous layer, and the aqueous layer was twice further extracted with diethyl ether. The ether extracts were dried over magnesium sulfate and filtered to yield 16 mL of a stock solution of the title amine in ether, which was used directly in Step C.

Step C: Preparation of 3-bromo-*N*-[4-chloro-2-[[(1-cyclopropylethyl) amino] carbonyl]-6-methylphenyl]-1-(3-chloro-2-pyridinyl)-1*N*-pyrazole-5-carboxamide

A solution of 2-[3-bromo-1-(3-chloro-2-pyridinyl)-1*H*-pyrazole-5-yl]-6-chloro-8-methyl-4*H*-3,1-benzoxazin-4-one (0.080 g, 0.18 mmol), prepared by the procedure described in PCT Patent Publication WO2003/015519, in acetonitrile (5 mL) was combined with an ether solution (6 mL) containing an excess of $\alpha$-methylcyclopropanemethanamine (i.e. the product of Step B). The resulting mixture was heated to reflux for several minutes and then stirred at room temperature overnight. The reaction mixture was concentrated, and the solids were purified by chromatography on silica gel to afford the title compound, a compound of the present invention, as a white solid (0.027 g), m.p. 182-183 °C.
$^1$H NMR ($CDCl_3$) $\delta$ 10.15 (s, 1H), 8.48 (d, 1H), 7.83 (d, 1H), 7.38 (m, 1H), 7.26 (m, 2H) 7.03 (s, 1H), 6.08 (d, 1H), 3.50 (m, 1H), 2.19 (s, 3H), 1.27 (d, 3H), 0.88 (m, 1H), 0.57 (m, 1H), 0.46 (m, 1H), 0.37 (m, 1H), 0.27 (m, 1H).

EXAMPLE 6

Preparation of 3-bromo-1-(3-chloro-2-pyridinyl)-*N*-[4-cyano-2-[[(1-cyclopropylethyl) amino]carbonyl]-6-methylphenyl]-1*H*-pyrazole-5-carboxamide

A mixture of 2-[3-bromo-1-(3-chloro-2-pyridinyl)-1*H*-pyrazol-5-yl]-8-methyl-4-oxo-4*H*-3,1-benzoxazine-6-carbonitrile (0.200 g, 0.45 mmol), prepared by the procedure described in PCT Patent Publication WO2004/067528, in acetonitrile (25 mL) was warmed to a homogeneous solution, and then combined with an ether solution (4 mL) containing an excess of $\alpha$-methylcyclopropanemethanamine (i.e. the product from Step B of Example 5). The resulting mixture was stirred at ambient temperature for 20 minutes. The reaction mixture was concentrated, and the solid residue was suspended in diethyl ether, and collected by filtration to afford the title compound, a compound of the present invention, as a solid (0.099 g), m.p. 244-245 °C.
$^1$H NMR ($CDCl_3$) $\delta$ 10.06 (s, 1H), 8.48 (d, 1H), 7.86 (m, 1H), 7.60 (d, 2H), 7.41 (m, 1H), 7.05 (s, 1H), 6.20 (d, 1H), 3.49 (m, 1H), 2.24 (s, 3H), 1.31 (d, 3H), 0.89 (m, 1H), 0.60 (m, 1H), 0.50 (m, 1H), 0.38 (m, 1H), 0.32 (m, 1H).

EXAMPLE 7

Preparation of 3-bromo-*N*-[4-chloro-2-[[(1-cyclopropylethyl)amino]carbonyl]-6-methylphenyl]-1-(2-chlorophenyl)-1*H*-pyrazole-5-carboxamide

Step A: Preparation of (2*E*)-[(2-chlorophenyl)hydrazono]acetic acid

To a solution of 2-chlorophenyl hydrazine hydrochloride (18.8 g, 0.105 mol) in water (300 mL) at room temperature was added concentrated hydrochloric acid (13.2 g, 0.136 mol), followed by dropwise addition of 50% glyoxylic acid (17.1 g, 0.115 mol) over 20 minutes to form a thick precipitate. The reaction mixture was then stirred for 30 minutes. The product was isolated by filtration, washed with water, and then dissolved in ethyl acetate (400 mL). The resulting solution was dried ($MgSO_4$) and concentrated under reduced pressure to afford the title product as a tan solid (20.5 g).
$^1H$ NMR (DMSO-$d_6$) $\delta$ 12.45 (s, 1H), 10.7 (s, 1H), 7.59 (d, 1H), 7.54 (s, 1H), 7.40 (d, 1H), 7.23 (t, 1H), 6.98 (t, 1H).

StepB: Preparation of (2-chlorophenyl)carbonohydrazonic dibromide

To a solution of (2*E*)-[(2-chlorophenyl)hydrazono]acetic acid (i.e. the product from Step A) (20.5 g, 0.103 mol) in *N,N*-dimethylformamide (188 mL) at 0 °C was added *N*-bromosuccinimide (35.7 g, 0.206 mol) portionwise over 30 min. The resulting mixture was stirred overnight at ambient temperature. The reaction mixture was diluted with water (150 mL) and extracted with diethyl ether (3 x 200 mL). The combined organic extracts were dried ($MgSO_4$), and purified by silica gel chromatography to afford the title compound as a red oil (12.0 g).
$^1H$ NMR ($CDCl_3$) $\delta$ 8.15 (br d, 1H), 7.41 (d, 1H), 7.31 (d, 1H), 7.21 (d, 1H), 6.90 (d, 1H).

Step C: Preparation of methyl 3-bromo-1-(2-chlorophenyl)-4,5-dihydro-1*H*-pyrazole-5-carboxylate

To a solution of (2-chlorophenyl)carbonohydrazonic dibromide (i.e. the product from Step B) (12.0 g, 38.5 mmol) in *N,N*-dimethylformamide (110 mL) was added methyl acrylate (13.85 mL, 153.8 mmol) in one portion, followed by dropwise addition of *N,N*-diisopropylethylamine (7.38 mL, 42.3 mmol) over 15 minutes. The reaction mixture was then stirred at ambient temperature for 1 h. The reaction mixture was diluted with water (200 mL) and extracted with diethyl ether (2 x 200 mL). The combined extracts were washed with water and brine. The ether extracts were dried ($MgSO_4$) and concentrated under reduced pressure to afford the title compound (12.2 g).
$^1H$ NMR ($CDCl_3$) $\delta$ 7.4 (t, 1H), 7.34 (d, 1H), 7.21 (d, 1H), 7.1 (t, 1H), 5.2 (m, 1H), 3.55 (s, 3H), 3.4 (m,1H).

Step D: Preparation of methyl 3-bromo-1-(2-chlorophenyl)-1*H*-pyrazole-5-carboxylate

To a solution of methyl 3-bromo-1-(2-chlorophenyl)-4,5-dihydro-1H-pyrazole-5-carboxylate (i.e. the product from Step C) (12.2 g, 38.4 mmol) in acetone (400 mL) was added potassium permanganate (24.2 g, 153.6 mmol) in approximately 1-gram portions every 10 minutes while maintaining the reaction temperature below 40 °C. The reaction mixture was then stirred at ambient temperature overnight. The reaction mixture was filtered through Celite® diatomaceous filter aid to remove solids, and then washed with diethyl ether (4 x 100 mL). After removal of the solvent, the crude product was purified by chromatography on silica gel to afford the title compound as an oil (5.8 g), which solidified on standing.
$^1H$ NMR ($CDCl_3$) $\delta$ 7.5 (d, 1H), 7.4-7.5 (m, 3H), 7.01 (s, 1H), 3.784 (s, 3H).

Step E: Preparation of 3-bromo-1-(2-chlorophenyl)-1*H*-pyrazole-5-carboxylic acid

To a solution of methyl 3-bromo-1-(2-chlorophenyl)-1H-pyrazole-5-carboxylate (i.e. the product from Step D) (5.8 g, 18.4 mmol) in methanol (40 mL) was added 12% aqueous sodium hydroxide (8.8 g, 30.5 mmol). The reaction mixture was stirred at ambient temperature for 2 h. The reaction mixture was then diluted with water (100 mL) and washed with diethyl ether (2 x 75 mL). The aqueous solution was acidified with concentrated hydrochloric acid to pH 2 and then extracted with ethyl acetate (3 x 150 mL). The combined ethyl acetate extracts were dried ($MgSO_4$) and concentrated under reduced pressure to afford the title compound (5.8 g).
$^1H$ NMR ($CDCl_3$) $\delta$ 7.4-7.55 (m, 4H), 7.1 (s, 1H).

Step F: Preparation of 2-[3-bromo-1-(2-chlorophenyl)-1*H*-pyrazol-5-yl]-6-chloro-8-methyl-4*H*-3,1-benzoxazin-4-one

3-Bromo-1-(2-chlorophenyl)-1H-pyrazole-5-carboxylic acid (i.e. the product from Step E) (0.165 g, 0.55 mmol), 2-amino-3-methyl-5-chlorobenzoic acid (0.101 g, 0.55 mmol) and 3-picoline (0.277 mL, 2.8 mmol) were combined with

acetonitrile (10 mL) and cooled to -10 °C. A solution of methanesulfonyl chloride (0.11 mL, 1.4 mmol) in acetonitrile (5 mL) was then added dropwise, and the reaction mixture was stirred at ambient temperature overnight. Water (10 mL) was added dropwise to the mixture to precipitate a solid. The solid was collected by filtration, washed successively with water and hexane, and then dried under nitrogen to afford the title compound as a white solid (0.216 g).
$^1H$ NMR (DMSO-$d_6$) δ 7.90 (d, 1H), 7.73 (m, 2H), 7.6 (m, 3H), 7.48 (s, 1H), 1.73 (s, 3H).

Step G: Preparation of 3-bromo-*N*-[4-chloro-2-[[(1-cyclopropylethyl)amino]carbonyl]-6-methylphenyl]-1-(2-chlorophenyl)-1*H*-pyrazole-5-carboxamide

A solution of 2-[3-bromo-1-(2-chlorophenyl)-1*H*-pyrazol-5-yl]-6-chloro-8-methyl-4*H*-3,1-benzoxazin-4-one (i.e. the product from Step F) (0.080 g, 0.18 mmol) in acetonitrile (20 mL) was combined with an ether solution (5 mL) containing an excess of α-methylcyclopropane methanamine (i.e. the product from Step B of Example 5). The resulting mixture was heated to reflux for several minutes and then stirred at room temperature overnight. The reaction mixture was concentrated, and the solid residue was suspended in diethyl ether and collected by filtration to afford the title compound, a compound of the present invention, as a solid (0.035 g), m.p. 180-181 °C.
$^1H$ NMR ($CDCl_3$) δ 10.03 (s, 1H), 7.49 (m, 1H), 7.42 (m, 1H), 7.381 (m, 2H), 7.26 (s,1H), 7.23 (s, 1H), 7.041 (s, 1H), 6.10 (d, 1H), 3.47 (m, 1H), 2.184 (s, 3H), 1.27 (d, 3H), 0.84 (m, 1H), 0.54 (m, 1H), 0.46 (m, 1H), 0.35 (m, 1H), 0.29 (m, 1H).

EXAMPLE 8

Preparation of 3-bromo-*N*-[4-chloro-2-methyl-6-[[(1-methylcyclopropyl)amino]carbonyl]-phenyl]-1-(3-chloro-2-pyridinyl)-1*H*-pyrazole-5-carboxamide

A mixture of 1,1-dimethylethyl (1-methylcyclopropyl)carbamate (0.300 g, 1.75 mmol) and 0.5 mL of trifluoroacetic acid was stirred overnight at room temperature. To the mixture, acetonitrile (15 mL) was added, followed by 2-[3-bromo-1-(3-chloro-2-pyridinyl)-1*H*-pyrazole-5-yl]-6-chloro-8-methyl-4*H*-3,1-benzoxazin-4-one (0.200 g, 0.44 mmol) and triethylamine (0.400 mL, 2.86 mmol). The reaction mixture was then heated at reflux for 2 h and then cooled to room temperature. The precipitated solid was collected by filtration, and washed with diethyl ether and hexane to afford the title compound, a compound of the present invention, as a solid (0.056 g), m.p. > 250 °C.
$^1H$ NMR ($CDCl_3$) δ 10.15 (s, 1H), 8.45 (d, 1H), 7.83 (d, 1H), 7.39 (m, 1H), 7.20 (d, 1H), 7.12 (d, 1H), 6.43 (s, 1H), 2.16 (s, 3H), 1.42 (s, 3H), 0.78 (m, 2H), 0.75 (m, 2H).
By the procedures described herein together with methods known in the art, the following compounds of Tables 1 to 10 can be prepared. The following abbreviations are used in the Tables which follow: CN means cyano, 2-Cl-Ph means 2-chlorophenyl, and 3-Cl-2-Py means 3-chloro-2-pyridinyl.

Table 1

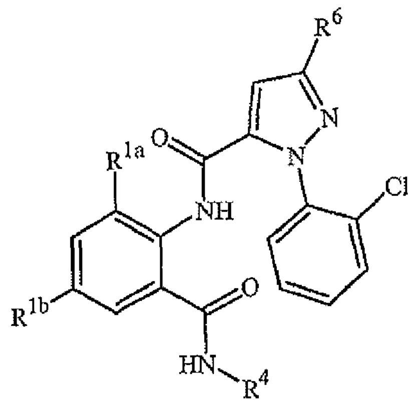

| $R^{1a}$ | $R^{1b}$ | $R^6$ | $R^4$ | $R^{1a}$ | $R^{1b}$ | $R^6$ | $R^4$ |
|---|---|---|---|---|---|---|---|
| Me | Cl | $CF_3$ | 1-methylcyclopropyl | Me | Cl | $CF_3$ | 1-methylcyclobutyl |
| Me | Cl | Br | 1-methylcyclopropyl | Me | Cl | Br | 1-methylcyclobutyl |
| Me | Cl | Cl | 1-methylcyclopropyl | Me | Cl | Cl | 1-methylcyclobutyl |
| Me | Br | $CF_3$ | 1-methylcyclopropyl | Me | Br | $CF_3$ | 1-methylcyclobutyl |
| Me | Br | Br | 1-methylcyclopropyl | Me | Br | Br | 1-methylcyclobutyl |

(continued)

| $R^{1a}$ | $R^{1b}$ | $R^6$ | $R^4$ | $R^{1a}$ | $R^{1b}$ | $R^6$ | $R^4$ |
|---|---|---|---|---|---|---|---|
| Me | Br | Cl | 1-methylcyclopropyl | Me | Br | Cl | 1-methylcyclobutyl |
| Me | CN | $CF_3$ | 1-methylcyclopropyl | Me | CN | $CF_3$ | 1-methylcyclobutyl |
| Me | CN | Br | 1-methylcyclopropyl | Me | CN | Br | 1-methylcyclobutyl |
| Me | CN | Cl | 1-methylcyclopropyl | Me | CN | Cl | 1-methylcyclobutyl |
| Cl | Cl | $CF_3$ | 1-methylcyclopropyl | Cl | Cl | $CF_3$ | 1-methylcyclobutyl |
| Cl | Cl | Br | 1-methylcyclopropyl | Cl | Cl | Br | 1-methylcyclobutyl |
| Cl | Cl | Cl | 1-methylcyclopropyl | Cl | Cl | Cl | 1-methylcyclobutyl |
| Br | Br | $CF_3$ | 1-methylcyclopropyl | Br | Br | $CF_3$ | 1-methylcyclobutyl |
| Br | Br | Br | 1-methylcyclopropyl | Br | Br | Br | 1-methylcyclobutyl |
| Br | Br | Cl | 1-methylcyclopropyl | Br | Br | Cl | 1-methylcyclobutyl |
| Me | Cl | $CF_3$ | 2-methylcyclopropyl | Me | Cl | $CF_3$ | 2,2-dimethylcyclopropyl |
| Me | Cl | Br | 2-methylcyclopropyl | Me | Cl | Br | 2,2-dimethylcyclopropyl |
| Me | Cl | Cl | 2-methylcyclopropyl | Me | Cl | Cl | 2,2-dimethylcyclopropyl |
| Me | Br | $CF_3$ | 2-methylcyclopropyl | Me | Br | $CF_3$ | 2,2-dimethylcyclopropyl |
| Me | Br | Br | 2-methylcyclopropyl | Me | Br | Br | 2,2-dimethylcyclopropyl |
| Me | Br | Cl | 2-methylcyclopropyl | Me | Br | Cl | 2,2-dimethylcyclopropyl |
| Me | CN | $CF_3$ | 2-methylcyclopropyl | Me | CN | $CF_3$ | 2,2-dimethylcyclopropyl |
| Me | CN | Br | 2-methylcyclopropyl | Me | CN | Br | 2,2-dimethylcyclopropyl |
| Me | CN | Cl | 2-methylcyclopropyl | Me | CN | Cl | 2,2-dimethylcyclopropyl |
| Cl | Cl | $CF_3$ | 2-methylcyclopropyl | Cl | Cl | $CF_3$ | 2,2-dimethylcyclopropyl |
| Cl | Cl | Br | 2-methylcyclopropyl | Cl | Cl | Br | 2,2-dimethylcyclopropyl |
| Cl | Cl | Cl | 2-methylcyclopropyl | Cl | Cl | Cl | 2,2-dimethylcyclopropyl |
| Br | Br | $CF_3$ | 2-methylcyclopropyl | Br | Br | $CF_3$ | 2,2-dimethylcyclopropyl |
| Br | Br | Br | 2-methylcyclopropyl | Br | Br | Br | 2,2-dimethylcyclopropyl |
| Br | Br | Cl | 2-methylcyclopropyl | Br | Br | Cl | 2,2-dimethylcyclopropyl |
| Me | Cl | $CF_3$ | cyclopropylmethyl | Me | Cl | $CF_3$ | 1-cyclopropylethyl |
| Me | Cl | Br | cyclopropylmethyl | Me | Cl | Br | 1-cyclopropylethyl |
| Me | Cl | Cl | cyclopropylmethyl | Me | Cl | Cl | 1-cyclopropylethyl |
| Me | Br | $CF_3$ | cyclopropylmethyl | Me | Br | $CF_3$ | 1-cyclopropylethyl |
| Me | Br | Br | cyclopropylmethyl | Me | Br | Br | 1-cyclopropylethyl |
| Me | Br | Cl | cyclopropylmethyl | Me | Br | Cl | 1-cyclopropylethyl |
| Me | CN | $CF_3$ | cyclopropylmethyl | Me | CN | $CF_3$ | 1-cyclopropylethyl |
| Me | CN | Br | cyclopropylmethyl | Me | CN | Br | 1-cyclopropylethyl |
| Me | CN | Cl | cyclopropylmethyl | Me | CN | Cl | 1-cyclopropylethyl |
| Cl | Cl | $CF_3$ | cyclopropylmethyl | Cl | Cl | $CF_3$ | 1-cyclopropylethyl |
| Cl | Cl | Br | cyclopropylmethyl | Cl | Cl | Br | 1-cyclopropylethyl |
| Cl | Cl | Cl | cyclopropylmethyl | Cl | Cl | Cl | 1-cyclopropylethyl |
| Br | Br | $CF_3$ | cyclopropylmethyl | Br | Br | $CF_3$ | 1-cyclopropylethyl |
| Br | Br | Br | cyclopropylmethyl | Br | Br | Br | 1-cyclopropylethyl |
| Br | Br | Cl | cyclopropylmethyl | Br | Br | Cl | 1-cyclopropylethyl |
| Me | Cl | $CF_3$ | 1-cyclopropyl-(1-methyl)ethyl | Me | Cl | $CF_3$ | (2,2-dimethylcyclopropyl)methyl |
| Me | Cl | Br | 1-cyclopropyl-(1-methyl)ethyl | Me | Cl | Br | (2,2-dimethylcyclopropyl)methyl |
| Me | Cl | Cl | 1-cyclopropyl-(1-methyl)ethyl | Me | Cl | Cl | (2,2-dimethylcyclopropyl)methyl |
| Me | Br | $CF_3$ | 1-cyclopropyl-(1-methyl)ethyl | Me | Br | $CF_3$ | (2,2-dimethylcyclopropyl)methyl |
| Me | Br | Br | 1-cyclopropyl-(1-methyl)ethyl | Me | Br | Br | (2,2-dimethylcyclopropyl)methyl |
| Me | Br | Cl | 1-cyclopropyl-(1-methyl)ethyl | Me | Br | Cl | (2,1-dimethylcyclopropyl)methyl |
| Me | CN | $CF_3$ | 1-cyclopropyl-(1-methyl)ethyl | Me | CN | $CF_3$ | (2,2-dimethylcyclopropyl)methyl |
| Me | CN | Br | 1-cyclopropyl-(1-methyl)ethyl | Me | CN | Br | (2,2-dimethylcyclopropyl)methyl |
| Me | CN | Cl | 1-cyclopropyl-(1-methyl)ethyl | Me | CN | Cl | (2,2-dimethylcyclopropyl)methyl |
| Cl | Cl | $CF_3$ | 1-cyclopropyl-(1-methyl)ethyl | Cl | Cl | $CF_3$ | (2,2-dimethylcyclopropyl)methyl |

(continued)

| $R^{1a}$ | $R^{1b}$ | $R^6$ | $R^4$ | $R^{1a}$ | $R^{1b}$ | $R^6$ | $R^4$ |
|---|---|---|---|---|---|---|---|
| Cl | Cl | Br | 1-cyclopropyl-(1-methyl)ethyl | Cl | Cl | Br | (2,2-dimethylcyclopropyl)methyl |
| Cl | Cl | Cl | 1-cyclopropyl-(1-methyl)ethyl | Cl | Cl | Cl | (2,2-dimethylcyclopropyl)methyl |
| Br | Br | $CF_3$ | 1-cyclopropyl-(1-methyl)ethyl | Br | Br | $CF_3$ | (2,2-dimethylcyclopropyl)methyl |
| Br | Br | Br | 1-cyclopropyl-(1-methyl)ethyl | Br | Br | Br | (2,2-dimethylcyclopropyl)methyl |
| Br | Br | Cl | 1-cyclopropyl-(1-methyl)ethyl | Br | Br | Cl | (2,2-dimethylcyclopropyl)methyl |
| Me | Cl | $CF_3$ | (2,2-dichlorocyclopropyl)methyl | Me | CN | Cl | (2,2-dichlorocyclopropyl)methyl |
| Me | Cl | Br | (2,2-dichlorocyclopropyl)methyl | Cl | Cl | $CF_3$ | (2,2-dichlorocyclopropyl)methyl |
| Me | Cl | Cl | (2,2-dichlorocyclopropyl)methyl | Cl | Cl | Br | (2,2-dichlorocyclopropyl)methyl |
| Me | Br | $CF_3$ | (2,2-dichlorocyclopropyl)methyl | Cl | Cl | Cl | (2,2-dichlorocyclopropyl)methyl |
| Me | Br | Br | (2,2-dichlorocyclopropyl)methyl | Br | Br | $CF_3$ | (2,2-dichlorocyclopropyl)methyl |
| Me | Br | Cl | (2,2-dichlorocyclopropyl)methyl | Br | Br | Br | (2,2-dichlorocyclopropyl)methyl |
| Me | CN | $CF_3$ | (2,2-dichlorocyclopropyl)methyl | Br | Br | Cl | (2,2-dichlorocyclopropyl)methyl |
| Me | CN | Br | (2,2-dichlorocyclopropyl)methyl | | | | |

Table 2

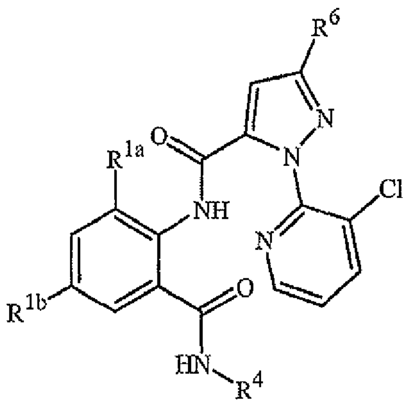

| $R^{1a}$ | $R^{1b}$ | $R^6$ | $R^4$ | $R^{1a}$ | $R^{1b}$ | $R^6$ | $R^4$ |
|---|---|---|---|---|---|---|---|
| Me | Cl | $CF_3$ | 1-methylcyclopropyl | Me | Cl | $CF_3$ | 1-methylcyclobutyl |
| Me | Cl | Br | 1-methylcyclopropyl | Me | Cl | Br | 1-methylcyclobutyl |
| Me | Cl | Cl | 1-methylcyclopropyl | Me | Cl | Cl | 1-methylcyclobutyl |
| Me | Br | $CF_3$ | 1-methylcyclopropyl | Me | Br | $CF_3$ | 1-methylcyclobutyl |
| Me | Br | Br | 1-methylcyclopropyl | Me | Br | Br | 1-methylcyclobutyl |
| Me | Br | Cl | 1-methylcyclopropyl | Me | Br | Cl | 1-methylcyclobutyl |
| Me | CN | $CF_3$ | 1-methylcyclopropyl | Me | CN | $CF_3$ | 1-methylcyclobutyl |
| Me | CN | Br | 1-methylcyclopropyl | Me | CN | Br | 1-methylcyclobutyl |
| Me | CN | Cl | 1-methylcyclopropyl | Me | CN | Cl | 1-methylcyclobutyl |
| Cl | Cl | $CF_3$ | 1-methylcyclopropyl | Cl | Cl | $CF_3$ | 1-methylcyclobutyl |
| Cl | Cl | Br | 1-methylcyclopropyl | Cl | Cl | Br | 1-methylcyclobutyl |
| Cl | Cl | Cl | 1-methylcyclopropyl | Cl | Cl | Cl | 1-methylcyclobutyl |
| Br | Br | $CF_3$ | 1-methylcyclopropyl | Br | Br | $CF_3$ | 1-methylcyclobutyl |
| Br | Br | Br | 1-methylcyclopropyl | Br | Br | Br | 1-methylcyclobutyl |
| Br | Br | Cl | 1-methylcyclopropyl | Br | Br | Cl | 1-methylcyclobutyl |
| Me | Cl | $CF_3$ | 2-methylcyclopropyl | Me | Cl | $CF_3$ | 2,2-dimethylcyclopropyl |
| Me | Cl | Br | 2-methylcyclopropyl | Me | Cl | Br | 2,2-dimethylcyclopropyl |
| Me | Cl | Cl | 2-methylcyclopropyl | Me | Cl | Cl | 2,2-dimethylcyclopropyl |
| Me | Br | $CF_3$ | 2-methylcyclopropyl | Me | Br | $CF_3$ | 2,2-dimethylcyclopropyl |
| Me | Br | Br | 2-methylcyclopropyl | Me | Br | Br | 2,2-dimethylcyclopropyl |

(continued)

| $R^{1a}$ | $R^{1b}$ | $R^6$ | $R^4$ | $R^{1a}$ | $R^{1b}$ | $R^6$ | $R^4$ |
|---|---|---|---|---|---|---|---|
| Me | Br | Cl | 2-methylcyclopropyl | Me | Br | Cl | 2,2-dimethylcyclopropyl |
| Me | CN | $CF_3$ | 2-methylcyclopropyl | Me | CN | $CF_3$ | 2,2-dimethylcyclopropyl |
| Me | CN | Br | 2-methylcyclopropyl | Me | CN | Br | 2,2-dimethylcyclopropyl |
| Me | CN | Cl | 2-methylcyclopropyl | Me | CN | Cl | 2,2-dimethylcyclopropyl |
| Cl | Cl | $CF_3$ | 2-methylcyclopropyl | Cl | Cl | $CF_3$ | 2,2-dimethylcyclopropyl |
| Cl | Cl | Br | 2-methylcyclopropyl | Cl | Cl | Br | 2,2-dimethylcyclopropyl |
| Cl | Cl | Cl | 2-methylcyclopropyl | Cl | Cl | Cl | 2,2-dimethylcyclopropyl |
| Br | Br | $CF_3$ | 2-methylcyclopropyl | Br | Br | $CF_3$ | 2,2-dimethylcyclopropyl |
| Br | Br | Br | 2-methylcyclopropyl | Br | Br | Br | 2,2-dimethylcyclopropyl |
| Br | Br | Cl | 2-methylcyclopropyl | Br | Br | Cl | 2,2-dimethylcyclopropyl |
| Me | Cl | $CF_3$ | cyclopropylmethyl | Me | Cl | $CF_3$ | 1-cyclopropylethyl |
| Me | Cl | Br | cyclopropylmethyl | Me | Cl | Br | 1-cyclopropylethyl |
| Me | Cl | Cl | cyclopropylmethyl | Me | Cl | Cl | 1-cyclopropylethyl |
| Me | Br | $CF_3$ | cyclopropylmethyl | Me | Br | $CF_3$ | 1-cyclopropylethyl |
| Me | Br | Br | cyclopropylmethyl | Me | Br | Br | 1-cyclopropylethyl |
| Me | Br | Cl | cyclopropylmethyl | Me | Br | Cl | 1-cyclopropylethyl |
| Me | CN | $CF_3$ | cyclopropylmethyl | Me | CN | $CF_3$ | 1-cyclopropylethyl |
| Me | CN | Br | cyclopropylmethyl | Me | CN | Br | 1-cyclopropylethyl |
| Me | CN | Cl | cyclopropylmethyl | Me | CN | Cl | 1-cyclopropylethyl |
| Cl | Cl | $CF_3$ | cyclopropylmethyl | Cl | Cl | $CF_3$ | 1-cyclopropylethyl |
| Cl | Cl | Br | cyclopropylmethyl | Cl | Cl | Br | 1-cyclopropylethyl |
| Cl | Cl | Cl | cyclopropylmethyl | Cl | Cl | Cl | 1-cyclopropylethyl |
| Br | Br | $CF_3$ | cyclopropylmethyl | Br | Br | $CF_3$ | 1-cyclopropylethyl |
| Br | Br | Br | cyclopropylmethyl | Br | Br | Br | 1-cyclopropylethyl |
| Br | Br | Cl | cyclopropylmethyl | Br | Br | Cl | 1-cyclopropylethyl |
| Me | Cl | $CF_3$ | 1-cyclopropyl-(1-methyl)ethyl | Me | Cl | $CF_3$ | (2,2-dimethylcyclopropyl)methyl |
| Me | Cl | Br | 1-cyclopropyl-(1-methyl)ethyl | Me | Cl | Br | (2,2-dimethylcyclopropyl)methyl |
| Me | Cl | Cl | 1-cyclopropyl-(1-methyl)ethyl | Me | Cl | Cl | (2,2-dimethylcyclopropyl)methyl |
| Me | Br | $CF_3$ | 1-cyclopropyl-(1-methyl)ethyl | Me | Br | $CF_3$ | (2,2-dimethylcyclopropyl)methyl |
| Me | Br | Br | 11-cyclopropyl-(1-methyl)ethyl | Me | Br | Br | (2,2-dimethylcyclopropyl)methyl |
| Me | Br | Cl | 1-cyclopropyl-(1-methyl)ethyl | Me | Br | Cl | (2,2-dimethylcyclopropyl)methyl |
| Me | CN | $CF_3$ | 1-cyclopropyl-(1-methyl)ethyl | Me | CN | $CF_3$ | (2,2-dimethylcyclopropyl)methyl |
| Me | CN | Br | 1-cyclopropyl-(1-methyl)ethyl | Me | CN | Br | (2,2-dimethylcyclopropyl)methyl |
| Me | CN | Cl | 1-cyclopropyl-(1-methyl)ethyl | Me | CN | Cl | (2,2-dimethylcyclopropyl)methyl |
| Cl | Cl | $CF_3$ | 1-cyclopropyl-(1-methyl)ethyl | Cl | Cl | $CF_3$ | (2,2-dimethylcyclopropyl)methyl |
| Cl | Cl | Br | 1-cyclopropyl-(1-methyl)ethyl | Cl | Cl | Br | (2,2-dimethylcyclopropyl)methyl |
| Cl | Cl | Cl | 1-cyclopropyl-(1-methyl)ethyl | Cl | Cl | Cl | (2,2-dimethylcyclopropyl)methyl |
| Br | Br | $CF_3$ | 1-cyclopropyl-(1-methyl)ethyl | Br | Br | $CF_3$ | (2,2-dimethylcyclopropyl)methyl |
| Br | Br | Br | 1-cyclopropyl-(1-methyl)ethyl | Br | Br | Br | (2,2-dimethylcyclopropyl)methyl |
| Br | Br | Cl | 1-cyclopropyl-(1-methyl)ethyl | Br | Br | Cl | (2,2-dimethylcyclopropyl)methyl |
| Me | Cl | $CF_3$ | (2,2-dichlorocyclopropyl)methyl | Me | CN | Cl | (2,2-dichlorocyclopropyl)methyl |
| Me | Cl | Br | (2,2-dichlorocyclopropyl)methyl | Cl | Cl | $CF_3$ | (2,2-dichlorocyclopropyl)methyl |
| Me | Cl | Cl | (2,2-dichlorocyclopropyl)methyl | Cl | Cl | Br | (2,2-dichlorocyclopropyl)methyl |
| Me | Br | $CF_3$ | (2,2-dichlorocyclopropyl)methyl | Cl | Cl | Cl | (2,2-dichlorocyclopropyl)methyl |
| Me | Br | Br | (2,2-dichlorocyclopropyl)methyl | Br | Br | $CF_3$ | (2,2-dichlorocyclopropyl)methyl |
| Me | Br | Cl | (2,2-dichlorocyclopropyl)methyl | Br | Br | Br | (2,2-dichlorocyclopropyl)methyl |
| Me | CN | $CF_3$ | (2,2-dichlorocyclopropyl)methyl | Br | Br | Cl | (2,2-dichlorocyclopropyl)methyl |
| Me | CN | Br | (2,2-dichlorocyclopropyl)methyl | | | | |

Table 3

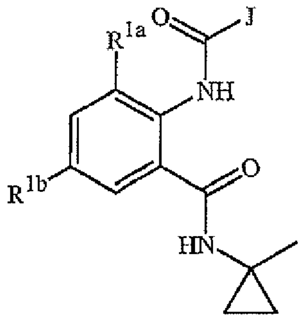

J is selected from the group consisting of

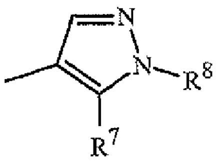

J-2

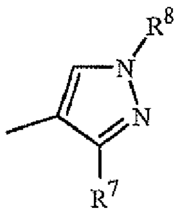

,

J-3

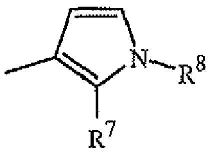

J-5

and

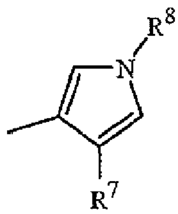

.

J-6

| J | $R^{1a}$ | $R^{1b}$ | $R^7$ | $R^8$ | J | $R^{1a}$ | $R^{1b}$ | $R^7$ | $R^8$ |
|---|---|---|---|---|---|---|---|---|---|
| J-2 | Me | Cl | 2-Cl-Ph | $CH_2CF_3$ | J-3 | Me | Cl | 2-Cl-Ph | $CH_2CF_3$ |
| J-2 | Me | Cl | 2-Cl-Ph | $CHF_2$ | J-3 | Me | Cl | 2-Cl-Ph | $CHF_2$ |
| J-2 | Me | Cl | 3-Cl-2-Py | $CH_2CF_3$ | J-3 | Me | Cl | 3-Cl-2-Py | $CH_2CF_3$ |
| J-2 | Me | Cl | 3-Cl-2-Py | $CHF_2$ | J-3 | Me | Cl | 3-Cl-2-Py | $CHF_2$ |
| J-2 | Me | CN | 2-Cl-Ph | $CH_2CF_3$ | J-3 | Me | CN | 2-Cl-Ph | $CH_2CF_3$ |
| J-2 | Me | CN | 2-Cl-Ph | $CHF_2$ | J-3 | Me | CN | 2-Cl-Ph | $CHF_2$ |
| J-2 | Me | CN | 3-Cl-2-Py | $CH_2CF_3$ | J-3 | Me | CN | 3-Cl-2-Py | $CH_2CF_3$ |
| J-2 | Me | CN | 3-Cl-2-Py | $CHF_2$ | J-3 | Me | CN | 3-Cl-2-Py | $CHF_2$ |
| J-2 | Cl | Cl | 2-Cl-Ph | $CH_2CF_3$ | J-3 | Cl | Cl | 2-Cl-Ph | $CH_2CF_3$ |
| J-2 | Cl | Cl | 2-Cl-Ph | $CHF_2$ | J-3 | Cl | Cl | 2-Cl-Ph | $CHF_2$ |
| J-2 | Cl | Cl | 3-Cl-2-Py | $CH_2CF_3$ | J-3 | Cl | Cl | 3-Cl-2-Py | $CH_2CF_3$ |
| J-2 | Cl | Cl | 3-Cl-2-Py | $CHF_2$ | J-3 | Cl | Cl | 3-Cl-2-Py | $CHF_2$ |
| J-2 | Br | Br | 2-Cl-Ph | $CH_2CF_3$ | J-3 | Br | Br | 2-Cl-Ph | $CH_2CF_3$ |
| J-2 | Br | Br | 2-Cl-Ph | $CHF_2$ | J-3 | Br | Br | 2-Cl-Ph | $CHF_2$ |
| J-2 | Br | Br | 3-Cl-2-Py | $CH_2CF_3$ | J-3 | Br | Br | 3-Cl-2-Py | $CH_2CF_3$ |
| J-2 | Br | Br | 3-Cl-2-Py | $CHF_2$ | J-3 | Br | Br | 3-Cl-2-Py | $CHF_2$ |
| J-5 | Me | Cl | 2-Cl-Ph | $CH_2CF_3$ | J-6 | Me | Cl | 2-Cl-Ph | $CH_2CF_3$ |
| J-5 | Me | Cl | 2-Cl-Ph | $CHF_2$ | J-6 | Me | Cl | 2-Cl-Ph | $CHF_2$ |
| J-5 | Me | Cl | 3-Cl-2-Py | $CH_2CF_3$ | J-6 | Me | Cl | 3-Cl-2-Py | $CH_2CF_3$ |
| J-5 | Me | Cl | 3-Cl-2-Py | $CHF_2$ | J-6 | Me | Cl | 3-Cl-2-Py | $CHF_2$ |
| J-5 | Me | CN | 2-Cl-Ph | $CH_2CF_3$ | J-6 | Me | CN | 2-Cl-Ph | $CH_2CF_3$ |
| J-5 | Me | CN | 2-Cl-Ph | $CHF_2$ | J-6 | Me | CN | 2-Cl-Ph | $CHF_2$ |
| J-5 | Me | CN | 3-Cl-2-Py | $CH_2CF_3$ | J-6 | Me | CN | 3-Cl-2-Py | $CH_2CF_3$ |
| J-5 | Me | CN | 3-Cl-2-Py | $CHF_2$ | J-6 | Me | CN | 3-Cl-2-Py | $CHF_2$ |
| J-5 | Cl | Cl | 2-Cl-Ph | $CH_2CF_3$ | J-6 | Cl | Cl | 2-Cl-Ph | $CH_2CF_3$ |
| J-5 | Cl | Cl | 2-Cl-Ph | $CHF_2$ | J-6 | Cl | Cl | 2-Cl-Ph | $CHF_2$ |
| J-5 | Cl | Cl | 3-Cl-2-Py | $CH_2CF_3$ | J-6 | Cl | Cl | 3-Cl-2-Py | $CH_2CF_3$ |
| J-5 | Cl | Cl | 3-Cl-2-Py | $CHF_2$ | J-6 | Cl | Cl | 3-Cl-2-Py | $CHF_2$ |
| J-5 | Br | Br | 2-Cl-Ph | $CH_2CF_3$ | J-6 | Br | Br | 2-Cl-Ph | $CH_2CF_3$ |
| J-5 | Br | Br | 2-Cl-Ph | $CHF_2$ | J-6 | Br | Br | 2-Cl-Ph | $CHF_2$ |

(continued)

J-2 J-3 J-5 J-6

| J | R1a | R1b | R7 | R8 | J | R1a | R1b | R7 | R8 |
|---|---|---|---|---|---|---|---|---|---|
| J-5 | Br | Br | 3-Cl-2-Py | $CH_2CF_3$ | J-6 | Br | Br | 3-Cl-2-Py | $CH_2CF_3$ |
| J-5 | Br | Br | 3-Cl-2-Py | $CHF_2$ | J-6 | Br | Br | 3-Cl-2-Py | $CHF_2$ |

Table 4

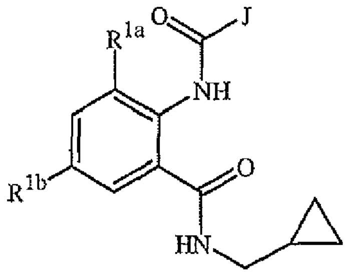

J is selected from the group consisting of

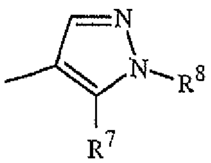

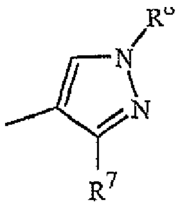

,

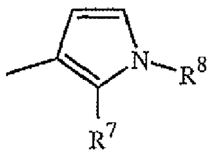

and

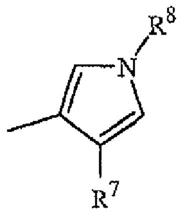

.

J-2 J-3 J-5 J-6

| J | $R^{1a}$ | $R^{1b}$ | $R^7$ | $R^8$ | J | $R^{1a}$ | $R^{1b}$ | $R^7$ | $R^8$ |
|---|---|---|---|---|---|---|---|---|---|
| J-2 | Me | Cl | 2-Cl-Ph | $CH_2CF_3$ | J-3 | Me | Cl | 2-Cl-Ph | $CH_2CF_3$ |
| J-2 | Me | Cl | 2-Cl-Ph | $CHF_2$ | J-3 | Me | Cl | 2-Cl-Ph | $CHF_2$ |
| J-2 | Me | Cl | 3-Cl-2-Py | $CH_2CF_3$ | J-3 | Me | Cl | 3-Cl-2-Py | $CH_2CF_3$ |
| J-2 | Me | Cl | 3-Cl-2-Py | $CHF_2$ | J-3 | Me | Cl | 3-Cl-2-Py | $CHF_2$ |
| J-2 | Me | CN | 2-Cl-Ph | $CH_2CF_3$ | J-3 | Me | CN | 2-Cl-Ph | $CH_2CF_3$ |
| J-2 | Me | CN | 2-Cl-Ph | $CHF_2$ | J-3 | Me | CN | 2-Cl-Ph | $CHF_2$ |
| J-2 | Me | CN | 3-Cl-2-Py | $CH_2CF_3$ | J-3 | Me | CN | 3-Cl-2-Py | $CH_2CF_3$ |
| J-2 | Me | CN | 3-Cl-2-Py | $CHF_2$ | J-3 | Me | CN | 3-Cl-2-Py | $CHF_2$ |
| J-2 | Cl | Cl | 2-Cl-Ph | $CH_2CF_3$ | J-3 | Cl | Cl | 2-Cl-Ph | $CH_2CF_3$ |
| J-2 | Cl | Cl | 2-Cl-Ph | $CHF_2$ | J-3 | Cl | Cl | 2-Cl-Ph | $CHF_2$ |
| J-2 | Cl | Cl | 3-Cl-2-Py | $CH_2CF_3$ | J-3 | Cl | Cl | 3-Cl-2-Py | $CH_2CF_3$ |
| J-2 | Cl | Cl | 3-Cl-2-Py | $CHF_2$ | J-3 | Cl | Cl | 3-Cl-2-Py | $CHF_2$ |
| J-2 | Br | Br | 2-Cl-Ph | $CH_2CF_3$ | J-3 | Br | Br | 2-Cl-Ph | $CH_2CF_3$ |
| J-2 | Br | Br | 2-Cl-Ph | $CHF_2$ | J-3 | Br | Br | 2-Cl-Ph | $CHF_2$ |
| J-2 | Br | Br | 3-Cl-2-Py | $CH_2CF_3$ | J-3 | Br | Br | 3-Cl-2-Py | $CH_2CF_3$ |
| J-2 | Br | Br | 3-Cl-2-Py | $CHF_2$ | J-3 | Br | Br | 3-Cl-2-Py | $CHF_2$ |
| J-5 | Me | Cl | 2-Cl-Ph | $CH_2CF_3$ | J-6 | Me | Cl | 2-Cl-Ph | $CH_2CF_3$ |
| J-5 | Me | Cl | 2-Cl-Ph | $CHF_2$ | J-6 | Me | Cl | 2-Cl-Ph | $CHF_2$ |
| J-5 | Me | Cl | 3-Cl-2-Py | $CH_2CF_3$ | J-6 | Me | Cl | 3-Cl-2-Py | $CH_2CF_3$ |
| J-5 | Me | Cl | 3-Cl-2-Py | $CHF_2$ | J-6 | Me | Cl | 3-Cl-2-Py | $CHF_2$ |
| J-5 | Me | CN | 2-Cl-Ph | $CH_2CF_3$ | J-6 | Me | CN | 2-Cl-Ph | $CH_2CF_3$ |
| J-5 | Me | CN | 2-Cl-Ph | $CHF_2$ | J-6 | Me | CN | 2-Cl-Ph | $CHF_2$ |
| J-5 | Me | CN | 3-Cl-2-Py | $CH_2CF_3$ | J-6 | Me | CN | 3-Cl-2-Py | $CH_2CF_3$ |
| J-5 | Me | CN | 3-Cl-2-Py | $CHF_2$ | J-6 | Me | CN | 3-Cl-2-Py | $CHF_2$ |
| J-5 | Cl | Cl | 2-Cl-Ph | $CH_2CF_3$ | J-6 | Cl | Cl | 2-Cl-Ph | $CH_2CF_3$ |

(continued)

| J | R1a | R1b | R7 | R8 | J | R1a | R1b | R7 | R8 |
|---|---|---|---|---|---|---|---|---|---|
| J-5 | Cl | Cl | 2-Cl-Ph | $CHF_2$ | J-6 | Cl | Cl | 2-Cl-Ph | $CHF_2$ |
| J-5 | Cl | Cl | 3-Cl-2-Py | $CH_2CF_3$ | J-6 | Cl | Cl | 3-Cl-2-Py | $CH_2CF_3$ |
| J-5 | Cl | Cl | 3-Cl-2-Py | $CHF_2$ | J-6 | Cl | Cl | 3-Cl-2-Py | $CHF_2$ |
| J-5 | Br | Br | 2-Cl-Ph | $CH_2CF_3$ | J-6 | Br | Br | 2-Cl-Ph | $CH_2CF_3$ |
| J-5 | Br | Br | 2-Cl-Ph | $CHF_2$ | J-6 | Br | Br | 2-Cl-Ph | $CHF_2$ |
| J-5 | Br | Br | 3-Cl-2-Py | $CH_2CF_3$ | J-6 | Br | Br | 3-Cl-2-Py | $CH_2CF_3$ |
| J-5 | Br | Br | 3-Cl-2-Py | $CHF_2$ | J-6 | Br | Br | 3-Cl-2-Py | $CHF_2$ |

Table 5

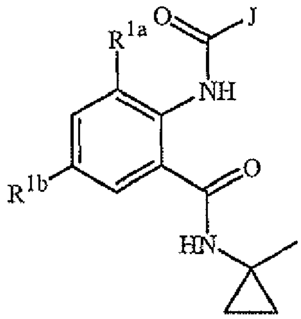

J is selected from the group consisting of

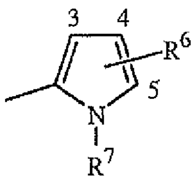

,

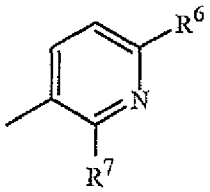

and

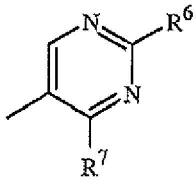

.

J-4 J-7 J-8

| J | R1a | R1b | R7 | R6 | J | R1a | R1b | R7 | R6 |
|---|---|---|---|---|---|---|---|---|---|
| J-4 | Me | Cl | 2-Cl-Ph | 4-Br | J-7 | Me | Cl | 2-Cl-Ph | $CF_3$ |
| J-4 | Me | Cl | 2-Cl-Ph | 5-Br | J-7 | Me | Cl | 3-Cl-2-Py | $CF_3$ |
| J-4 | Me | Cl | 3-Cl-2-Py | 4-Br | J-7 | Me | CN | 2-Cl-Ph | $CF_3$ |
| J-4 | Me | Cl | 3-Cl-2-Py | 5-Br | J-7 | Me | CN | 3-Cl-2-Py | $CF_3$ |
| J-4 | Me | CN | 2-Cl-Ph | 4-Br | J-7 | Cl | Cl | 2-Cl-Ph | $CF_3$ |
| J-4 | Me | CN | 2-Cl-Ph | 5-Br | J-7 | Cl | Cl | 3-Cl-2-Py | $CF_3$ |
| J-4 | Me | CN | 3-Cl-2-Py | 4-Br | J-7 | Br | Br | 2-Cl-Ph | $CF_3$ |
| J-4 | Me | CN | 3-Cl-2-Py | 5-Br | J-7 | Br | Br | 3-Cl-2-Py | $CF_3$ |
| J-4 | Cl | Cl | 2-Cl-Ph | 4-Br | J-8 | Me | Cl | 2-Cl-Ph | $CF_3$ |
| J-4 | Cl | Cl | 2-Cl-Ph | 5-Br | J-8 | Me | Cl | 3-Cl-2-Py | $CF_3$ |
| J-4 | Cl | Cl | 3-Cl-2-Py | 4-Br | J-8 | Me | CN | 2-Cl-Ph | $CF_3$ |
| J-4 | Cl | Cl | 3-Cl-2-Py | 5-Br | J-8 | Me | CN | 3-Cl-2-Py | $CF_3$ |
| J-4 | Br | Br | 2-Cl-Ph | 4-Br | J-8 | Cl | Cl | 2-Cl-Ph | $CF_3$ |
| J-4 | Br | Br | 2-Cl-Ph | 5-Br | J-8 | Cl | Cl | 3-Cl-2-Py | $CF_3$ |
| J-4 | Br | Br | 3-Cl-2-Py | 4-Br | J-8 | Br | Br | 2-Cl-Ph | $CF_3$ |
| J-4 | Br | Br | 3-Cl-2-Py | 5-Br | J-8 | Br | Br | 3-Cl-2-Py | $CF_3$ |

Table 6

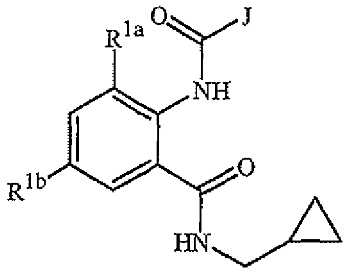

J is selected from the group consisting of

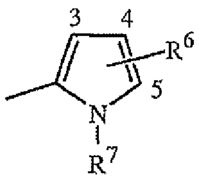

,

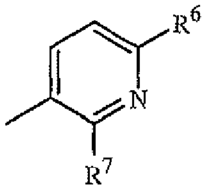

and

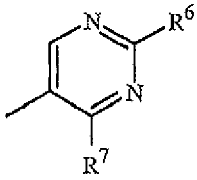

.

J-4 J-7 J-8

| J | $R^{1a}$ | $R^{1b}$ | $R^7$ | $R^6$ | J | $R^{1a}$ | $R^{1b}$ | $R^7$ | $R^6$ |
|---|---|---|---|---|---|---|---|---|---|
| J-4 | Me | Cl | 2-Cl-Ph | 4-Br | J-7 | Me | Cl | 2-Cl-Ph | $CF_3$ |
| J-4 | Me | Cl | 2-Cl-Ph | 5-Br | J-7 | Me | Cl | 3-Cl-2-Py | $CF_3$ |
| J-4 | Me | Cl | 3-Cl-2-Py | 4-Br | J-7 | Me | CN | 2-Cl-Ph | $CF_3$ |
| J-4 | Me | Cl | 3-Cl-2-Py | 5-Br | J-7 | Me | CN | 3-Cl-2-Py | $CF_3$ |
| J-4 | Me | CN | 2-Cl-Ph | 4-Br | J-7 | Cl | Cl | 2-Cl-Ph | $CF_3$ |
| J-4 | Me | CN | 2-Cl-Ph | 5-Br | J-7 | Cl | Cl | 3-Cl-2-Py | $CF_3$ |
| J-4 | Me | CN | 3-Cl-2-Py | 4-Br | J-7 | Br | Br | 2-Cl-Ph | $CF_3$ |
| J-4 | Me | CN | 3-Cl-2-Py | 5-Br | J-7 | Br | Br | 3-Cl-2-Py | $CF_3$ |
| J-4 | Cl | Cl | 2-Cl-Ph | 4-Br | J-8 | Me | Cl | 2-Cl-Ph | $CF_3$ |
| J-4 | Cl | Cl | 2-Cl-Ph | 5-Br | J-8 | Me | Cl | 3-Cl-2-Py | $CF_3$ |
| J-4 | Cl | Cl | 3-Cl-2-Py | 4-Br | J-8 | Me | CN | 2-Cl-Ph | $CF_3$ |
| J-4 | Cl | Cl | 3-Cl-2-Py | 5-Br | J-8 | Me | CN | 3-Cl-2-Py | $CF_3$ |
| J-4 | Br | Br | 2-Cl-Ph | 4-Br | J-8 | Cl | Cl | 2-Cl-Ph | $CF_3$ |
| J-4 | Br | Br | 2-Cl-Ph | 5-Br | J-8 | Cl | Cl | 3-Cl-2-Py | $CF_3$ |
| J-4 | Br | Br | 3-Cl-2-Py | 4-Br | J-8 | Br | Br | 2-Cl-Ph | $CF_3$ |
| J-4 | Br | Br | 3-Cl-2-Py | 5-Br | J-8 | Br | Br | 3-Cl-2-Py | $CF_3$ |

Table 7

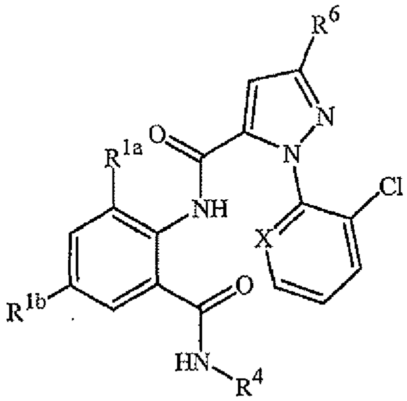

| $R^{1a}$ | $R^{1b}$ | $R^6$ | X | $R^4$ | $R^{1a}$ | $R^{1b}$ | $R^6$ | X | $R^4$ |
|---|---|---|---|---|---|---|---|---|---|
| Me | Cl | $CF_3$ | CH | oxiranylmethyl | Me | Cl | $CF_3$ | N | oxiranylmethyl |

(continued)

| $R^{1a}$ | $R^{1b}$ | $R^6$ | X | $R^4$ | $R^{1a}$ | $R^{1b}$ | $R^6$ | X | $R^4$ |
|---|---|---|---|---|---|---|---|---|---|
| Me | Cl | Br | CH | oxiranylmethyl | Me | Cl | Br | N | oxiranylmethyl |
| Me | Cl | Cl | CH | oxiranylmethyl | Me | Cl | Cl | N | oxiranylmethyl |
| Me | Br | $CF_3$ | CH | oxiranylmethyl | Me | Br | $CF_3$ | N | oxiranylmethyl |
| Me | Br | Br | CH | oxiranylmethyl | Me | Br | Br | N | oxiranylmethyl |
| Me | Br | Cl | CH | oxiranylmethyl | Me | Br | Cl | N | oxiranylmethyl |
| Me | CN | $CF_3$ | CH | oxiranylmethyl | Me | CN | $CF_3$ | N | oxiranylmethyl |
| Me | CN | Br | CH | oxiranylmethyl | Me | CN | Br | N | oxiranylmethyl |
| Me | CN | Cl | CH | oxiranylmethyl | Me | CN | Cl | N | oxiranylmethyl |
| Cl | Cl | $CF_3$ | CH | oxiranylmethyl | Cl | Cl | $CF_3$ | N | oxiranylmethyl |
| Cl | Cl | Br | CH | oxiranylmethyl | Cl | Cl | Br | N | oxiranylmethyl |
| Cl | Cl | Cl | CH | oxiranylmethyl | Cl | Cl | Cl | N | oxiranylmethyl |
| Br | Br | $CF_3$ | CH | oxiranylmethyl | Br | Br | $CF_3$ | N | oxiranylmethyl |
| Br | Br | Br | CH | oxiranylmethyl | Br | Br | Br | N | oxiranylmethyl |
| Br | Br | Cl | CH | oxiranylmethyl | Br | Br | Cl | N | oxiranylmethyl |
| Me | Cl | $CF_3$ | CH | 1-oxiranylethyl | Me | Cl | $CF_3$ | N | 1-oxiranylethyl |
| Me | Cl | Br | CH | 1-oxiranylethyl | Me | Cl | Br | N | 1-oxiranylethyl |
| Me | Cl | Cl | CH | 1-oxiranylethyl | Me | Cl | Cl | N | 1-oxiranylethyl |
| Me | Br | $CF_3$ | CH | 1-oxiranylethyl | Me | Br | $CF_3$ | N | 1-oxiranylethyl |
| Me | Br | Br | CH | 1-oxiranylethyl | Me | Br | Br | N | 1-oxiranylethyl |
| Me | Br | Cl | CH | 1-oxiranylethyl | Me | Br | Cl | N | 1-oxiranylethyl |
| Me | CN | $CF_3$ | CH | 1-oxiranylethyl | Me | CN | $CF_3$ | N | 1-oxiranylethyl |
| Me | CN | Br | CH | 1-oxiranylethyl | Me | CN | Br | N | 1-oxiranylethyl |
| Me | CN | Cl | CH | 1-oxiranylethyl | Me | CN | Cl | N | 1-oxiranylethyl |
| Cl | Cl | $CF_3$ | CH | 1-oxiranylethyl | Cl | Cl | $CF_3$ | N | 1-oxiranylethyl |
| Cl | Cl | Br | CH | 1-oxiranylethyl | Cl | Cl | Br | N | 1-oxiranylethyl |
| Cl | Cl | Cl | CH | 1-oxiranylethyl | Cl | Cl | Cl | N | 1-oxiranylethyl |
| Br | Br | $CF_3$ | CH | 1-oxiranylethyl | Br | Br | $CF_3$ | N | 1-oxiranylethyl |
| Br | Br | Br | CH | 1-oxiranylethyl | Br | Br | Br | N | 1-oxiranylethyl |
| Br | Br | Cl | CH | 1-oxiranylethyl | Br | Br | Cl | N | 1-oxiranylethyl |
| Me | Cl | $CF_3$ | CH | 1-methyl-1-oxiranylethyl | Me | Cl | $CF_3$ | N | 1-methyl-1-oxiranylethyl |
| Me | Cl | Br | CH | 1-methyl-1-oxiranylethyl | Me | Cl | Br | N | 1-methyl-1-oxiranylethyl |
| Me | Cl | Cl | CH | 1-methyl-1-oxiranylethyl | Me | Cl | Cl | N | 1-methyl-1-oxiranylethyl |
| Me | Br | $CF_3$ | CH | 1-methyl-1-oxiranylethyl | Me | Br | $CF_3$ | N | 1-methyl-1-oxiranylethyl |
| Me | Br | Br | CH | 1-methyl-1-oxiranylethyl | Me | Br | Br | N | 1-methyl-1-oxiranylethyl |
| Me | Br | Cl | CH | 1-methyl-1-oxiranylethyl | Me | Br | Cl | N | 1-methyl-1-oxiranylethyl |
| Me | CN | $CF_3$ | CH | 1-methyl-1-oxiranylethyl | Me | CN | $CF_3$ | N | 1-methyl-1-oxiranylethyl |
| Me | CN | Br | CH | 1-methyl-1-oxiranylethyl | Me | CN | Br | N | 1-methyl-1-oxiranylethyl |
| Me | CN | Cl | CH | 1-methyl-1-oxiranylethyl | Me | CN | Cl | N | 1-methyl-1-oxiranylethyl |
| Cl | Cl | $CF_3$ | CH | 1-methyl-1-oxiranylethyl | Cl | Cl | $CF_3$ | N | 1-methyl-1-oxiranylethyl |
| Cl | Cl | Br | CH | 1-methyl-1-oxiranylethyl | Cl | Cl | Br | N | 1-methyl-1-oxiranylethyl |
| Cl | Cl | Cl | CH | 1-methyl-1-oxiranylethyl | Cl | Cl | Cl | N | 1-methyl-1-oxiranylethyl |
| Br | Br | $CF_3$ | CH | 1-methyl-1-oxiranylethyl | Br | Br | $CF_3$ | N | 1-methyl-1-oxiranylethyl |
| Br | Br | Br | CH | 1-methyl-1-oxiranylethyl | Br | Br | Br | N | 1-methyl-1-oxiranylethyl |
| Br | Br | Cl | CH | 1-methyl-1-oxiranylethyl | Br | Br | Cl | N | 1-methyl-1-oxiranylethyl |
| Me | Cl | $CF_3$ | CH | (3-oxetanyl)methyl | Me | Cl | $CF_3$ | N | (3-oxetanyl)methyl |
| Me | Cl | Br | CH | (3-oxetanyl)methyl | Me | Cl | Br | N | (3-oxetanyl)methyl |
| Me | Cl | Cl | CH | (3-oxetanyl)methyl | Me | Cl | Cl | N | (3-oxetanyl)methyl |
| Me | Br | $CF_3$ | CH | (3-oxetanyl)methyl | Me | Br | $CF_3$ | N | (3-oxetanyl)methyl |
| Me | Br | Br | CH | (3-oxetanyl)methyl | Me | Br | Br | N | (3-oxetanyl)methyl |
| Me | Br | Cl | CH | (3-oxetanyl)methyl | Me | Br | Cl | N | (3-oxetanyl)methyl |

(continued)

| $R^{1a}$ | $R^{1b}$ | $R^6$ | X | $R^4$ | $R^{1a}$ | $R^{1b}$ | $R^6$ | X | $R^4$ |
|---|---|---|---|---|---|---|---|---|---|
| Me | CN | $CF_3$ | CH | (3-oxetanyl)methyl | Me | CN | $CF_3$ | N | (3-oxetanyl)methyl |
| Me | CN | Br | CH | (3-oxetanyl)methyl | Me | CN | Br | N | (3-oxetanyl)methyl |
| Me | CN | Cl | CH | (3-oxetanyl)methyl | Me | CN | Cl | N | (3-oxetanyl)methyl |
| Cl | Cl | $CF_3$ | CH | (3-oxetanyl)methyl | Cl | Cl | $CF_3$ | N | (3-oxetanyl)methyl |
| Cl | Cl | Br | CH | (3-oxetanyl)methyl | Cl | Cl | Br | N | (3-oxetanyl)methyl |
| Cl | Cl | Cl | CH | (3-oxetanyl)methyl | Cl | Cl | Cl | N | (3-oxetanyl)methyl |
| Br | Br | $CF_3$ | CH | (3-oxetanyl)methyl | Br | Br | $CF_3$ | N | (3-oxetanyl)methyl |
| Br | Br | Br | CH | (3-oxetanyl)methyl | Br | Br | Br | N | (3-oxetanyl)methyl |
| Br | Br | Cl | CH | (3-oxetanyl)methyl | Br | Br | Cl | N | (3-oxetanyl)methyl |
| Me | Cl | $CF_3$ | CH | 1-(3-oxetanyl)ethyl | Me | Cl | $CF_3$ | N | 1-(3-oxetanyl)ethyl |
| Me | Cl | Br | CH | 1-(3-oxetanyl)ethyl | Me | Cl | Br | N | 1-(3-oxetanyl)ethyl |
| Me | Cl | Cl | CH | 1-(3-oxetanyl)ethyl | Me | Cl | Cl | N | 1-(3-oxetanyl)ethyl |
| Me | Br | $CF_3$ | CH | 1-(3-oxetanyl)ethyl | Me | Br | $CF_3$ | N | 1-(3-oxetanyl)ethyl |
| Me | Br | Br | CH | 1-(3-oxetanyl)ethyl | Me | Br | Br | N | 1-(3-oxetanyl)ethyl |
| Me | Br | Cl | CH | 1-(3-oxetanyl)ethyl | Me | Br | Cl | N | 1-(3-oxetanyl)ethyl |
| Me | CN | $CF_3$ | CH | 1-(3-oxetanyl)ethyl | Me | CN | $CF_3$ | N | 1-(3-oxetanyl)ethyl |
| Me | CN | Br | CH | 1-(3-oxetanyl)ethyl | Me | CN | Br | N | 1-(3-oxetanyl)ethyl |
| Me | CN | Cl | CH | 1-(3-oxetanyl)ethyl | Me | CN | Cl | N | 1-(3-oxetanyl)ethyl |
| Cl | Cl | $CF_3$ | CH | 1-(3-oxetanyl)ethyl | Cl | Cl | $CF_3$ | N | 1-(3-oxetanyl)ethyl |
| Cl | Cl | Br | CH | 1-(3-oxetanyl)ethyl | Cl | Cl | Br | N | 1-(3-oxetanyl)ethyl |
| Cl | Cl | Cl | CH | 1-(3-oxetanyl)ethyl | Cl | Cl | Cl | N | 1-(3-oxetanyl)ethyl |
| Br | Br | $CF_3$ | CH | 1-(3-oxetanyl)ethyl | Br | Br | $CF_3$ | N | 1-(3-oxetanyl)ethyl |
| Br | Br | Br | CH | 1-(3-oxetanyl)ethyl | Br | Br | Br | N | 1-(3-oxetanyl)ethyl |
| Br | Br | Cl | CH | 1-(3-oxetanyl)ethyl | Br | Br | Cl | N | 1-(3-oxetanyl)ethyl |
| Me | Cl | $CF_3$ | CH | 1-methyl-1-(3-oxetanyl) ethyl | Me | Cl | $CF_3$ | N | 1-methyl-1-(3-oxetanyl) ethyl |
| Me | Cl | Br | CH | 1-methyl-1-(3-oxetanyl) ethyl | Me | Cl | Br | N | 1-methyl-1-(3-oxetanyl) ethyl |
| Me | Cl | Cl | CH | 1-methyl-1-(3-oxetanyl) ethyl | Me | Cl | Cl | N | 1-methyl-1-(3-oxetanyl) ethyl |
| Me | Br | $CF_3$ | CH | 1-methyl-1-(3-oxetanyl) ethyl | Me | Br | $CF_3$ | N | 1-methyl-1-(3-oxetanyl) ethyl |
| Me | Br | Br | CH | 1-methyl-1-(3-oxetanyl) ethyl | Me | Br | Br | N | 1-methyl-1-(3-oxetanyl) ethyl |
| Me | Br | Cl | CH | 1-methyl-1-(3-oxetanyl) ethyl | Me | Br | Cl | N | 1-methyl-1-(3-oxetanyl) ethyl |
| Me | CN | $CF_3$ | CH | 1-methyl-1-(3-oxetanyl) ethyl | Me | CN | $CF_3$ | N | 1-methyl-1-(3-oxetanyl) ethyl |
| Me | CN | Br | CH | 1-methyl-1-(3-oxetanyl) ethyl | Me | CN | Br | N | 1-methyl-1-(3-oxetanyl) ethyl |
| Me | CN | Cl | CH | 1-methyl-1-(3-oxetanyl) ethyl | Me | CN | Cl | N | 1-methyl-1-(3-oxetanyl) ethyl |
| Cl | Cl | $CF_3$ | CH | 1-methyl-1-(3-oxetanyl) ethyl | Cl | Cl | $CF_3$ | N | 1-methyl-1-(3-oxetanyl) ethyl |
| Cl | Cl | Br | CH | 1-methyl-1-(3-oxetanyl) ethyl | Cl | Cl | Br | N | 1-methyl-1-(3-oxetanyl) ethyl |
| Cl | Cl | Cl | CH | 1-methyl-1-(3-oxetanyl) ethyl | Cl | Cl | Cl | N | 1-methyl-1-(3-oxetanyl) ethyl |
| Br | Br | $CF_3$ | CH | 1-methyl-1-(3-oxetanyl) ethyl | Br | Br | $CF_3$ | N | 1-methyl-1-(3-oxetanyl) ethyl |

(continued)

| $R^{1a}$ | $R^{1b}$ | $R^6$ | X | $R^4$ | $R^{1a}$ | $R^{1b}$ | $R^6$ | X | $R^4$ |
|---|---|---|---|---|---|---|---|---|---|
| Br | Br | Br | CH | 1-methyl-1-(3-oxetanyl) ethyl | Br | Br | Br | N | 1-methyl-1-(3-oxetanyl) ethyl |
| Br | Br | Cl | CH | 1-methyl-1-(3-oxetanyl) ethyl | Br | Br | Cl | N | 1-methyl-1-(3-oxetanyl) ethyl |
| Me | Cl | $CF_3$ | CH | (2-oxetanyl)methyl | Me | Cl | $CF_3$ | N | (2-oxetanyl)methyl |
| Me | Cl | Br | CH | (2-oxetanyl)methyl | Me | Cl | Br | N | (2-oxetanyl)methyl |
| Me | Cl | Cl | CH | (2-oxetanyl)methyl | Me | Cl | Cl | N | (2-oxetanyl)methyl |
| Me | Br | $CF_3$ | CH | (2-oxetanyl)methyl | Me | Br | $CF_3$ | N | (2-oxetanyl)methyl |
| Me | Br | Br | CH | (2-oxetanyl)methyl | Me | Br | Br | N | (2-oxetanyl)methyl |
| Me | Br | Cl | CH | (2-oxetanyl)methyl | Me | Br | Cl | N | (2-oxetanyl)methyl |
| Me | CN | $CF_3$ | CH | (2-oxetanyl)methyl | Me | CN | $CF_3$ | N | (2-oxetanyl)methyl |
| Me | CN | Br | CH | (2-oxetanyl)methyl | Me | CN | Br | N | (2-oxetanyl)methyl |
| Me | CN | Cl | CH | (2-oxetanyl)methyl | Me | CN | Cl | N | (2-oxetanyl)methyl |
| Cl | Cl | $CF_3$ | CH | (2-oxetanyl)methyl | Cl | Cl | $CF_3$ | N | (2-oxetanyl)methyl |
| Cl | Cl | Br | CH | (2-oxetanyl)methyl | Cl | Cl | Br | N | (2-oxetanyl)methyl |
| Cl | Cl | Cl | CH | (2-oxetanyl)methyl | Cl | Cl | Cl | N | (2-oxetanyl)methyl |
| Br | Br | $CF_3$ | CH | (2-oxetanyl)methyl | Br | Br | $CF_3$ | N | (2-oxetanyl)methyl |
| Br | Br | Br | CH | (2-oxetanyl)methyl | Br | Br | Br | N | (2-oxetanyl)methyl |
| Br | Br | Cl | CH | (2-oxetanyl)methyl | Br | Br | Cl | N | (2-oxetanyl)methyl |
| Me | Cl | $CF_3$ | CH | 1-(2-oxetanyl)ethyl | Me | Cl | $CF_3$ | N | 1-(2-oxetanyl)ethyl |
| Me | Cl | Br | CH | 1-(2-oxetanyl)ethyl | Me | Cl | Br | N | 1-(2-oxetanyl)ethyl |
| Me | Cl | Cl | CH | 1-(2-oxetanyl)ethyl | Me | Cl | Cl | N | 1-(2-oxetanyl)ethyl |
| Me | Br | $CF_3$ | CH | 1-(2-oxetanyl)ethyl | Me | Br | $CF_3$ | N | 1-(2-oxetanyl)ethyl |
| Me | Br | Br | CH | 1-(2-oxetanyl)ethyl | Me | Br | Br | N | 1-(2-oxetanyl)ethyl |
| Me | Br | Cl | CH | 1-(2-oxetanyl)ethyl | Me | Br | Cl | N | 1-(2-oxetanyl)ethyl |
| Me | CN | $CF_3$ | CH | 1-(2-oxetanyl)ethyl | Me | CN | $CF_3$ | N | 1-(2-oxetanyl)ethyl |
| Me | CN | Br | CH | 1-(2-oxetanyl)ethyl | Me | CN | Br | N | 1-(2-oxetanyl)ethyl |
| Me | CN | Cl | CH | 1-(2-oxetanyl)ethyl | Me | CN | Cl | N | 1-(2-oxetanyl)ethyl |
| Cl | Cl | $CF_3$ | CH | 1-(2-oxetanyl)ethyl | Cl | Cl | $CF_3$ | N | 1-(2-oxetanyl)ethyl |
| Cl | Cl | Br | CH | 1-(2-oxetanyl)ethyl | Cl | Cl | Br | N | 1-(2-oxetanyl)ethyl |
| Cl | Cl | Cl | CH | 1-(2-oxetanyl)ethyl | Cl | Cl | Cl | N | 1-(2-oxetanyl)ethyl |
| Br | Br | $CF_3$ | CH | 1-(2-oxetanyl)ethyl | Br | Br | $CF_3$ | N | 1-(2-oxetanyl)ethyl |
| Br | Br | Br | CH | 1-(2-oxetanyl)ethyl | Br | Br | Br | N | 1-(2-oxetanyl)ethyl |
| Br | Br | Cl | CH | 1-(2-oxetanyl)ethyl | Br | Br | Cl | N | 1-(2-oxetanyl)ethyl |
| Me | Cl | $CF_3$ | CH | 1-methyl-1-(2-oxetanyl) ethyl | Me | Cl | $CF_3$ | N | 1-methyl-1-(2-oxetanyl) ethyl |
| Me | Cl | Br | CH | 1-methyl-1-(2-oxetanyl) ethyl | Me | Cl | Br | N | 1-methyl-1-(2-oxetanyl) ethyl |
| Me | Cl | Cl | CH | 1-methyl-1-(2-oxetanyl) ethyl | Me | Cl | Cl | N | 1-methyl-1-(2-oxetanyl) ethyl |
| Me | Br | $CF_3$ | CH | 1-methyl-1-(2-oxetanyl) ethyl | Me | Br | $CF_3$ | N | 1-methyl-1-(2-oxetanyl) ethyl |
| Me | Br | Br | CH | 1-methyl-1-(2-oxetanyl) ethyl | Me | Br | Br | N | 1-methyl-1-(2-oxetanyl) ethyl |
| Me | Br | Cl | CH | 1-methyl-1-(2-oxetanyl) ethyl | Me | Br | Cl | N | 1-methyl-1-(2-oxetanyl) ethyl |
| Me | CN | $CF_3$ | CH | 1-methyl-1-(2-oxetanyl) ethyl | Me | CN | $CF_3$ | N | 1-methyl-1-(2-oxetanyl) ethyl |
| Me | CN | Br | CH | 1-methyl-1-(2-oxetanyl) ethyl | Me | CN | Br | N | 1-methyl-1-(2-oxetanyl) ethyl |

(continued)

| $R^{1a}$ | $R^{1b}$ | $R^6$ | X | $R^4$ | $R^{1a}$ | $R^{1b}$ | $R^6$ | X | $R^4$ |
|---|---|---|---|---|---|---|---|---|---|
| Me | CN | Cl | CH | 1-methyl-1-(2-oxetanyl) ethyl | Me | CN | Cl | N | 1-methyl-1-(2-oxetanyl) ethyl |
| Cl | Cl | $CF_3$ | CH | 1-methyl-1-(2-oxetanyl) ethyl | Cl | Cl | $CF_3$ | N | 1-methyl-1-(2-oxetanyl) ethyl |
| Cl | Cl | Br | CH | 1-methyl-1-(2-oxetanyl) ethyl | Cl | Cl | Br | N | 1-methyl-1-(2-oxetanyl) ethyl |
| Cl | Cl | Cl | CH | 1-methyl-1-(2-oxetanyl) ethyl | Cl | Cl | Cl | N | 1-methyl-1-(2-oxetanyl) ethyl |
| Br | Br | $CF_3$ | CH | 1-methyl-1-(2-oxetanyl) ethyl | Br | Br | $CF_3$ | N | 1-methyl-1-(2-oxetanyl) ethyl |
| Br | Br | Br | CH | 1-methyl-1-(2-oxetanyl) ethyl | Br | Br | Br | N | 1-methyl-1-(2-oxetanyl) ethyl |
| Br | Br | Cl | CH | 1-methyl-1-(2-oxetanyl) ethyl | Br | Br | Cl | N | 1-methyl-1-(2-oxetanyl) ethyl |
| Me | Cl | $CF_3$ | CH | 3-oxetanyl | Me | Cl | $CF_3$ | N | 3-oxetanyl |
| Me | Cl | Br | CH | 3-oxetanyl | Me | Cl | Br | N | 3-oxetanyl |
| Me | Cl | Cl | CH | 3-oxetanyl | Me | Cl | Cl | N | 3-oxetanyl |
| Me | Br | $CF_3$ | CH | 3-oxetanyl | Me | Br | $CF_3$ | N | 3-oxetanyl |
| Me | Br | Br | CH | 3-oxetanyl | Me | Br | Br | N | 3-oxetanyl |
| Me | Br | Cl | CH | 3-oxetanyl | Me | Br | Cl | N | 3-oxetanyl |
| Me | CN | $CF_3$ | CH | 3-oxetanyl | Me | CN | $CF_3$ | N | 3-oxetanyl |
| Me | CN | Br | CH | 3-oxetanyl | Me | CN | Br | N | 3-oxetanyl |
| Me | CN | Cl | CH | 3-oxetanyl | Me | CN | Cl | N | 3-oxetanyl |
| Cl | Cl | $CF_3$ | CH | 3-oxetanyl | Cl | Cl | $CF_3$ | N | 3-oxetanyl |
| Cl | Cl | Br | CH | 3-oxetanyl | Cl | Cl | Br | N | 3-oxetanyl |
| Cl | Cl | Cl | CH | 3-oxetanyl | Cl | Cl | Cl | N | 3-oxetanyl |
| Br | Br | $CF_3$ | CH | 3-oxetanyl | Br | Br | $CF_3$ | N | 3-oxetanyl |
| Br | Br | Br | CH | 3-oxetanyl | Br | Br | Br | N | 3-oxe.tanyl |
| Br | Br | Cl | CH | 3-oxetanyl | Br | Br | Cl | N | 3-oxetanyl |
| Me | Cl | $CF_3$ | CH | 3-(3-methyloxetanyl) | Me | Cl | $CF_3$ | N | 3-(3-methyloxetanyl) |
| Me | Cl | Br | CH | 3-(3-methyloxetanyl) | Me | Cl | Br | N | 3-(3-methyloxetanyl) |
| Me | Cl | Cl | CH | 3-(3-methyloxetanyl) | Me | Cl | Cl | N | 3-(3-methyloxetanyl) |
| Me | Br | $CF_3$ | CH | 3-(3-methyloxetanyl). | Me | Br | $CF_3$ | N | 3-(3-methyloxetanyl) |
| Me | Br | Br | CH | 3-(3-methyloxetanyl) | Me | Br | Br | N | 3-(3-methyloxetanyl) |
| Me | Br | Cl | CH | 3-(3-methyloxetanyl) | Me | Br | Cl | N | 3-(3-methyloxetanyl) |
| Me | CN | $CF_3$ | CH | 3-(3-methyloxetanyl) | Me | CN | $CF_3$ | N | 3-(3-methyloxetanyl) |
| Me | CN | Br | CH | 3-(3-methyloxetanyl) | Me | CN | Br | N | 3-(3-methyloxetanyl) |
| Me | CN | Cl | CH | 3-(3-methyloxetanyl) | Me | CN | Cl | N | 3-(3-methyloxetanyl) |
| Cl | Cl | $CF_3$ | CH | 3-(3-methyloxetanyl) | Cl | Cl | $CF_3$ | N | 3-(3-methyloxetanyl) |
| Cl | Cl | Br | CH | 3-(3-methyloxetanyl) | Cl | Cl | Br | N | 3-(3-methyloxetanyl) |
| Cl | Cl | Cl | CH | 3-(3-methyloxetanyl) | Cl | Cl | Cl | N | 3-(3-methyloxetanyl) |
| Br | Br | $CF_3$ | CH | 3-(3-methyloxetanyl) | Br | Br | $CF_3$ | N | 3-(3-methyloxetanyl) |
| Br | Br | Br | CH | 3-(3-methyloxetanyl) | Br | Br | Br | N | 3-(3-methyloxetanyl) |
| Br | Br | Cl | CH | 3-(3-methyloxetanyl) | Br | Br | Cl | N | 3-(3-methyloxetanyl) |

Table 8

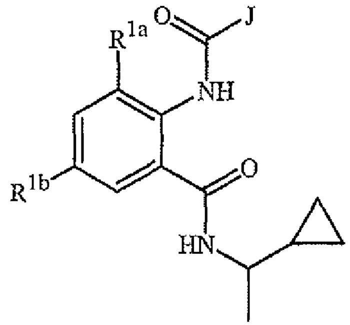

J is selected from the group consisting of

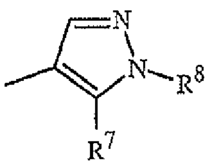

J-2 ,

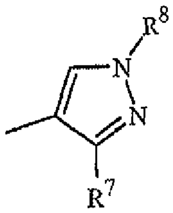

J-3 ,

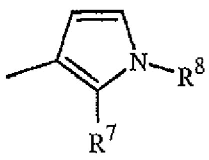

J-5 and

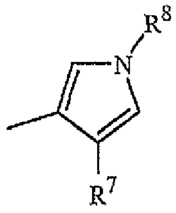

J-6 .

| J | $R^{1a}$ | $R^{1b}$ | $R^7$ | $R^8$ | J | $R^{1a}$ | $R^{1b}$ | $R^7$ | $R^8$ |
|---|---|---|---|---|---|---|---|---|---|
| J-2 | Me | Cl | 2-Cl-Ph | $CH_2CF_3$ | J-3 | Me | Cl | 2-Cl-Ph | $CH_2CF_3$ |
| J-2 | Me | Cl | 2-Cl-Ph | $CHF_2$ | J-3 | Me | Cl | 2-Cl-Ph | $CHF_2$ |
| J-2 | Me | Cl | 3-Cl-2-Py | $CH_2CF_3$ | J-3 | Me | Cl | 3-Cl-2-Py | $CH_2CF_3$ |
| J-2 | Me | Cl | 3-Cl-2-Py | $CHF_2$ | J-3 | Me | Cl | 3-Cl-2-Py | $CHF_2$ |
| J-2 | Me | CN | 2-Cl-Ph | $CH_2CF_3$ | J-3 | Me | CN | 2-Cl-Ph | $CH_2CF_3$ |
| J-2 | Me | CN | 2-Cl-Ph | $CHF_2$ | J-3 | Me | CN | 2-Cl-Ph | $CHF_2$ |
| J-2 | Me | CN | 3-Cl-2-Py | $CH_2CF_3$ | J-3 | Me | CN | 3-Cl-2-Py | $CH_2CF_3$ |
| J-2 | Me | CN | 3-Cl-2-Py | $CHF_2$ | J-3 | Me | CN | 3-Cl-2-Py | $CHF_2$ |
| J-2 | Cl | Cl | 2-Cl-Ph | $CH_2CF_3$ | J-3 | Cl | Cl | 2-Cl-Ph | $CH_2CF_3$ |
| J-2 | Cl | Cl | 2-Cl-Ph | $CHF_2$ | J-3 | Cl | Cl | 2-Cl-Ph | $CHF_2$ |
| J-2 | Cl | Cl | 3-Cl-2-Py | $CH_2CF_3$ | J-3 | Cl | Cl | 3-Cl-2-Py | $CH_2CF_3$ |
| J-2 | Cl | Cl | 3-Cl-2-Py | $CHF_2$ | J-3 | Cl | Cl | 3-Cl-2-Py | $CHF_2$ |
| J-2 | Br | Br | 2-Cl-Ph | $CH_2CF_3$ | J-3 | Br | Br | 2-Cl-Ph | $CH_2CF_3$ |

(continued)

| J | $R^{1a}$ | $R^{1b}$ | $R^7$ | $R^8$ | J | $R^{1a}$ | $R^{1b}$ | $R^7$ | $R^8$ |
|---|---|---|---|---|---|---|---|---|---|
| J-2 | Br | Br | 2-Cl-Ph | $CHF_2$ | J-3 | Br | Br | 2-Cl-Ph | $CHF_2$ |
| J-2 | Br | Br | 3-Cl-2-Py | $CH_2CF_3$ | J-3 | Br | Br | 3-Cl-2-Py | $CH_2CF_3$ |
| J-2 | Br | Br | 3-Cl-2-Py | $CHF_2$ | J-3 | Br | Br | 3-Cl-2-Py | $CHF_2$ |
| J-5 | Me | Cl | 2-Cl-Ph | $CH_2CF_3$ | J-6 | Me | Cl | 2-Cl-Ph | $CH_2CF_3$ |
| J-5 | Me | Cl | 2-Cl-Ph | $CHF_2$ | J-6 | Me | Cl | 2-Cl-Ph | $CHF_2$ |
| J-5 | Me | Cl | 3-Cl-2-Py | $CH_2CF_3$ | J-6 | Me | Cl | 3-Cl-2-Py | $CH_2CF_3$ |
| J-5 | Me | Cl | 3-Cl-2-Py | $CHF_2$ | J-6 | Me | Cl | 3-Cl-2-Py | $CHF_2$ |
| J-5 | Me | CN | 2-Cl-Ph | $CH_2CF_3$ | J-6 | Me | CN | 2-Cl-Ph | $CH_2CF_3$ |
| J-5 | Me | CN | 2-Cl-Ph | $CHF_2$ | J-6 | Me | CN | 2-Cl-Ph | $CHF_2$ |
| J-5 | Me | CN | 3-Cl-2-Py | $CH_2CF_3$ | J-6 | Me | CN | 3-Cl-2-Py | $CH_2CF_3$ |
| J-5 | Me | CN | 3-Cl-2-Py | $CHF_2$ | J-6 | Me | CN | 3-Cl-2-Py | $CHF_2$ |
| J-5 | Cl | Cl | 2-Cl-Ph | $CH_2CF_3$ | J-6 | Cl | Cl | 2-Cl-Ph | $CH_2CF_3$ |
| J-5 | Cl | Cl | 2-Cl-Ph | $CHF_2$ | J-6 | Cl | Cl | 2-Cl-Ph | $CHF_2$ |
| J-5 | Cl | Cl | 3-Cl-2-Py | $CH_2CF_3$ | J-6 | Cl | Cl | 3-Cl-2-Py | $CH_2CF_3$ |
| J-5 | Cl | Cl | 3-Cl-2-Py | $CHF_2$ | J-6 | Cl | Cl | 3-Cl-2-Py | $CHF_2$ |
| J-5 | Br | Br | 2-Cl-Ph | $CH_2CF_3$ | J-6 | Br | Br | 2-Cl-Ph | $CH_2CF_3$ |
| J-5 | Br | Br | 2-Cl-Ph | $CHF_2$ | J-6 | Br | Br | 2-Cl-Ph | $CHF_2$ |
| J-5 | Br | Br | 3-Cl-2-Py | $CH_2CF_3$ | J-6 | Br | Br | 3-Cl-2-Py | $CH_2CF_3$ |
| J-5 | Br | Br | 3-Cl-2-Py | $CHF_2$ | J-6 | Br | Br | 3-Cl-2-Py | $CHF_2$ |

Table 9

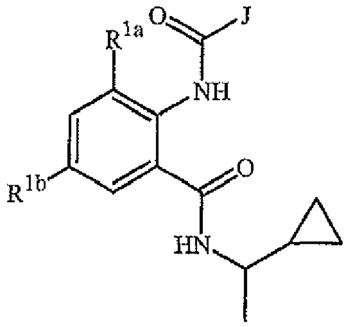

J is selected from the group consisting of

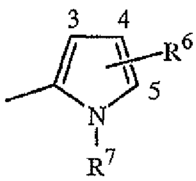 ,

J-4

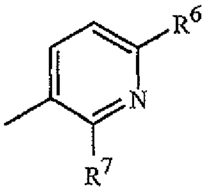

J-7

and

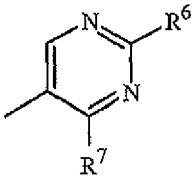 .

J-8

| J | $R^{1a}$ | $R^{1b}$ | $R^7$ | $R^6$ | J | $R^{1a}$ | $R^{1b}$ | $R^7$ | $R^6$ |
|---|---|---|---|---|---|---|---|---|---|
| J-4 | Me | Cl | 2-Cl-Ph | 4-Br | J-7 | Me | Cl | 2-Cl-Ph | $CF_3$ |
| J-4 | Me | Cl | 2-Cl-Ph | 5-Br | J-7 | Me | Cl | 3-Cl-2-Py | $CF_3$ |
| J-4 | Me | Cl | 3-Cl-2-Py | 4-Br | J-7 | Me | CN | 2-Cl-Ph | $CF_3$ |
| J-4 | Me | Cl | 3-Cl-2-Py | 5-Br | J-7 | Me | CN | 3-Cl-2-Py | $CF_3$ |
| J-4 | Me | CN | 2-Cl-Ph | 4-Br | J-7 | Cl | Cl | 2-Cl-Ph | $CF_3$ |
| J-4 | Me | CN | 2-Cl-Ph | 5-Br | J-7 | Cl | Cl | 3-Cl-2-Py | $CF_3$ |
| J-4 | Me | CN | 3-Cl-2-Py | 4-Br | J-7 | Br | Br | 2-Cl-Ph | $CF_3$ |
| J-4 | Me | CN | 3-Cl-2-Py | 5-Br | J-7 | Br | Br | 3-Cl-2-Py | $CF_3$ |
| J-4 | Cl | Cl | 2-Cl-Ph | 4-Br | J-8 | Me | Cl | 2-Cl-Ph | $CF_3$ |
| J-4 | Cl | Cl | 2-Cl-Ph | 5-Br | J-8 | Me | Cl | 3-Cl-2-Py | $CF_3$ |
| J-4 | Cl | Cl | 3-Cl-2-Py | 4-Br | J-8 | Me | CN | 2-Cl-Ph | $CF_3$ |
| J-4 | Cl | Cl | 3-Cl-2-Py | 5-Br | J-8 | Me | CN | 3-Cl-2-Py | $CF_3$ |
| J-4 | Br | Br | 2-Cl-Ph | 4-Br | J-8 | Cl | Cl | 2-Cl-Ph | $CF_3$ |
| J-4 | Br | Br | 2-Cl-Ph | 5-Br | J-8 | Cl | Cl | 3-Cl-2-Py | $CF_3$ |
| J-4 | Br | Br | 3-Cl-2-Py | 4-Br | J-8 | Br | Br | 2-Cl-Ph | $CF_3$ |
| J-4 | Br | Br | 3-Cl-2-Py | 5-Br | J-8 | Br | Br | 3-Cl-2-Py | $CF_3$ |

Table 10 lists particular amides of Formula **10**, which according to the methods of Schemes 6 and 7 are useful as intermediates for preparing compounds of Formulae **1** and **1a**.

Table 10

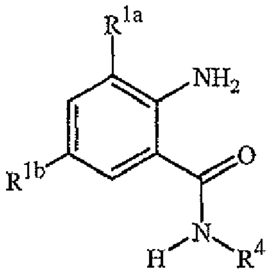

| $R^{1a}$ | $R^{1b}$ | $R^4$ | (m.p. °C) | $R^{1a}$ | $R^{1b}$ | $R^4$ | (m.p. °C) |
|---|---|---|---|---|---|---|---|
| Me | Cl | 1-methylcyclopropyl | | Me | Cl | 1-methylcyclobutyl | |

(continued)

| $R^{1a}$ | $R^{1b}$ | $R^4$ | (m.p. °C) | $R^{1a}$ | $R^{1b}$ | $R^4$ | (m.p. °C) |
|---|---|---|---|---|---|---|---|
| Me | Br | 1-methylcyclopropyl | | Me | Br | 1-methylcyclobutyl | |
| Me | CN | 1-methylcyclopropyl | | Me | CN | 1-methylcyclobutyl | |
| Cl | Cl | 1-methylcyclopropyl | | Cl | Cl | 1-methylcyclobutyl | |
| Br | Br | l-methylcyclopropyl | | Br | Br | 1-methylcyclobutyl | |
| Me | Cl | 2-methylcyclopropyl | | Me | Cl | 2,2-dimethylcyclopropyl | |
| Me | Br | 2-methylcyclopropyl | | Me | Br | 2,2-dimethylcyclopropyl | |
| Me | CN | 2-methylcyclopropyl | | Me | CN | 2,2-dimethylcyclopropyl | |
| Cl | Cl | 2-methylcyclopropyl | | Cl | Cl | 2,2-dimethylcyclopropyl | |
| Br | Br | 2-methylcyclopropyl | | Br | Br | 2,2-dimethylcyclopropyl | |
| Me | Cl | cyclopropylmethyl | (127-128) | Me | Cl | 1-cyclopropylethyl | (143-144) |
| Me | Br | cyclopropylmethyl | | Me | Br | 1-cyclopropylethyl | |
| Me | CN | cyclopropylmethyl | (113-114) | Me | CN | 1-cyclopropylethyl | (135-136) |
| Cl | Cl | cyclopropylmethyl | | Cl | Cl | 1-cyclopropylethyl | |
| Br | Br | cyclopropylmethyl | | Br | Br | 1-cyclopropylethyl | |
| Me | Cl | 1-cyclopropyl-(1-methyl)ethyl | | Me | Cl | (2,2-dimethylcyclopropyl)methyl | |
| Me | Br | 1-cyclopropyl-(1-methyl)ethyl | | Me | Br | (2,2-dimethylcyclopropyl)methyl | |
| Me | CN | 1-cyclopropyl-(1-methyl)ethyl | | Me | CN | (2,2-dimethycyclopropyl)methyl | |
| Cl | Cl | 1-cyclopropyl-(1-methyl)ethyl | | Cl | Cl | (2,2-dimethylcyclopropyl)methyl | |
| Br | Br | 1-cyclopropyl-(1-methyl)ethyl | | Br | Br | (2,2-dimethylcyclopropyl)methyl | |
| Me | Cl | (2,2-dichlorocyclopropyl)methyl | | Me | Cl | oxiranylmethyl | |
| Me | Br | (2,2-dichlorocyclopropyl)methyl | | Me | Br | oxiranylmethyl | |
| Me | CN | (2,2-dichlorocyclopropyl)methyl | | Me | CN | oxiranylmethyl | |
| Cl | Cl | (2,2-dichlorocyclopropyl)methyl | | Cl | Cl | oxiranylmethyl | |
| Br | Br | (2,2-dichlorocyclopropyl)methyl | | Br | Br | oxiranylmethyl | |
| Me | Cl | 1-oxiranylethyl | | Me | Cl | 1-methyl-1-oxiranylethyl | |
| Me | Br | 1-oxiranylethyl | | Me | Br | 1-methyl-l-oxiranylethyl | |
| Me | CN | 1-oxiranylethyl | | Me | CN | 1-methyl-1-oxiranylethyl | |
| Cl | Cl | 1-oxiranylethyl | | Cl | Cl | 1-methyl-1-oxiranylethyl | |
| Br | Br | 1-oxiranylethyl | | Br | Br | 1-methyl-1-oxiranylethyl | |
| Me | Cl | (3-oxetanyl)methyl | | Me | Cl | 1-(3-oxetanyl)ethyl | |
| Me | Br | (3-oxetanyl)methyl | | Me | Br | 1-(3-oxetanyl)ethyl | |
| Me | CN | (3-oxetanyl)methyl | | Me | CN | 1-(3-oxetanyl)ethyl | |
| Cl | Cl | (3-oxetanyl)methyl | | Cl | Cl | 1-(3-oxetanyl)ethyl | |
| Br | Br | (3-oxetanyl)methyl | | Br | Br | 1-(3-oxetanyl)ethyl | |
| Me | Cl | 1-methyl-1-(3-oxetanyl)ethyl | | Me | Cl | (2-oxetanyl)methyl | |
| Me | Br | 1-methyl-1-(3-oxetanyl)ethyl | | Me | Br | (2-oxetanyl)methyl | |
| Me | CN | 1-methyl-1-(3-oxetanyl)ethyl | | Me | CN | (2-oxetanyl)methyl | |
| Cl | Cl | 1-methyl-1-(3-oxetanyl)ethyl | | Cl | Cl | (2-oxetanyl)methyl | |
| Br | Br | 1-methyl-1-(3-oxetanyl)ethyl | | Br | Br | (2-oxetanyl)methyl | |
| Me | Cl | 1-(2-axetanyl)ethyl | | Me | Cl | 1-methyl-1-(2-oxetanyl)ethyl | |
| Me | Br | 1-(2-oxetanyl)ethyl | | Me | Br | 1-methyl-1-(2-oxetanyl)ethyl | |
| Me | CN | 1-(2-oxetanyl)ethyl | | Me | CN | 1-methyl-1-(2-oxetanyl)ethyl | |
| Cl | Cl | 1-(2-oxetanyl)ethyl | | Cl | Cl | 1-methyl-1-(2-oxetanyl)ethyl | |
| Br | Br | 1-(2-oxetanyl)ethyl | | Br | Br | 1-methyl-1-(2-oxetanyl)ethyl | |
| Me | Cl | 3-oxetanyl | | Me | Cl | 3-(3-methyloxetanyl) | |

(continued)

| R1a | R1b | R4 | (m.p. °C) | R1a | R1b | R4 | (m.p. °C) |
|---|---|---|---|---|---|---|---|
| Me | Br | 3-oxetanyl | | Me | Br | 3-(3-methyloxetanyl) | |
| Me | CN | 3-oxetanyl | | Me | CN | 3-(3-methyloxetanyl) | |
| Cl | Cl | 3-oxetanyl | | Cl | Cl | 3-(3-methyloxetanyl) | |
| Br | Br | 3-oxetanyl | | Br | Br | 3-(3-methyloxetanyl) | |

Formulation/Utility

Compounds of this invention can generally be used as a formulation or a composition with a carrier suitable for agronomic or nonagronomic uses comprising at least one of a liquid diluent, a solid diluent or a surfactant. The formulation or composition ingredients arc selected to be consistent with the physical properties of the active ingredient, mode of application and environmental factors such as soil type, moisture and temperature. Useful formulations include liquids such as solutions (including emulsifiable concentrates), suspensions, emulsions (including microemulsions and/or suspoemulsions) and the like which optionally can be thickened into gels. Useful formulations further include solids such as dusts, powders, granules, pellets, tablets, films (including seed treatment), and the like which can be water-dispersible ("wettable") or water-soluble. Active ingredient can be (micro)encapsulated and further formed into a suspension or solid formulation; alternatively the entire formulation of active ingredient can be encapsulated (or "overcoated"). Encapsulation can control or delay release of the active ingredient. Compositions of this invention can also optionally comprise plant nutrients, e.g., a fertilizer composition comprising at least one plant nutrient selected from nitrogen, phosphorus, potassium, sulfur, calcium, magnesium, iron, copper, boron, manganese, zinc, and molybdenum. Of note are compositions comprising at least one fertilizer composition comprising at least one plant nutrient selected from nitrogen, phosphorus, potassium, sulfur, calcium and magnesium. Compositions of the present invention which further comprise at least one plant nutrient can be in the form of liquids or solids. Of note are solid formulations in the form of granules, small sticks or tablets. Solid formulations comprising a fertilizer composition can be prepared by mixing the compound or composition of the present invention with the fertilizer composition together with formulating ingredients and then preparing the formulation by methods such as granulation or extrusion. Alternatively solid formulations can be prepared by spraying a solution or suspension of a compound or composition of the present invention in a volatile solvent onto a previously prepared fertilizer composition in the form of dimensionally stable mixtures, e.g., granules, small sticks or tablets, and then evaporating the solvent. Sprayable formulations can be extended in suitable media and used at spray volumes from about one to several hundred liters per hectare. High-strength compositions can be primarily used as intermediates for further formulation.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders. | 0.001-90 | 0-99.999 | 0-15 |
| Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-99 | 5-99.999 | 0-15 |
| High Strength Compositions | 90-99 | 0-10 | 0-2 |

Typical solid diluents are described in Watkins, et al., Handbook of Insecticide Dust Diluents and Carriers, 2nd Ed., Dorland Books, Caldwell, New Jersey. Typical liquid diluents are described in Marsden, Solvents Guide, 2nd Ed., Interscience, New York, 1950. McCutcheon's Detergents and Emulsifiers Annual, Allured Publ. Corp., Ridgewood, New Jersey, as well as Sisely and Wood, Encyclopedia of Surface Active Agents, Chemical Publ. Co., Inc., New York, 1964, list surfactants and recommended uses. All formulations can contain minor amounts of additives to reduce foam, caking, corrosion, microbiological growth and the like, or thickeners to increase viscosity.

Surfactants include, for example, polyethoxylated alcohols, polyethoxylated alkylphenols, polyethoxylated sorbitan fatty acid esters, dialkyl sulfosuccinates, alkyl sulfates, alkylbenzene sulfonates, organosilicones, *N,N*-dialkyltaurates, lignin sulfonates, naphthalene sulfonate formaldehyde condensates, polycarboxylates, glycerol esters, polyoxyethylene/polyoxypropylene block copolymers, and alkylpolyglycosides where the number of glucose units, referred to as degree of polymerization (D.P.), can range from 1 to 3 and the alkyl units can range from $C_6$-$C_{14}$ (see Pure and

Applied Chemistry 72, 1255-1264). Solid diluents include, for example, clays such as bentonite, montmorillonite, attapulgite and kaolin, starch, sugar, silica, talc, diatomaceous earth, urea, calcium carbonate, sodium carbonate and bicarbonate, and sodium sulfate. Liquid diluents include, for example, water, *N,N*-dimethylformamide, dimethyl sulfoxide, *N*-alkylpyrrolidone, ethylene glycol, polypropylene glycol, paraffins, alkylbenzenes, alkylnaphthalenes, glycerine, triacetine, oils of olive, castor, linseed, tung, sesame, corn, peanut, cotton-seed, soybean, rape-seed and coconut, fatty acid esters, ketones such as cyclohexanone, 2-heptanone, isophorone and 4-hydroxy-4-methyl-2-pentanone, acetates and alcohols such as methanol, cyclohexanol, decanol and tetrahydrofurfuryl alcohol.

Useful formulations of this invention can also contain materials known as formulation aids including antifoams, film formers and dyes and are well known to those skilled in the art.

Antifoams can include water dispersible liquids comprising polyorganosiloxanes such as Rhodorsil® 416. The film formers can include polyvinyl acetates, polyvinyl acetate copolymers, polyvinylpyrrolidone-vinyl acetate copolymer, polyvinyl alcohols, polyvinyl alcohol copolymers and waxes. Dyes can include water dispersible liquid colorant compositions such as Pro-Ized® Colorant Red. One skilled in the art will appreciate that this is a non-exhaustive list of formulation aids. Suitable examples of formulation aids include those listed herein and those listed in McCutcheon's 2001, Volume 2: Functional Materials, published by MC Publishing Company and PCT Publication WO 03/024222.

Solutions, including emulsifiable concentrates, can be prepared by simply mixing the ingredients. Dusts and powders can be prepared by blending and, usually, grinding as in a hammer mill or fluid-energy mill. Suspensions are usually prepared by wet-znilling; see, for example, U.S. 3,060,084. Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Compound numbers refer to compounds in Index Table A. Without further elaboration, it is believed that one skilled in the art using the preceding description can utilize the present invention to its fullest extent. The following Examples are, therefore, to be constructed as merely illustrative, and not limiting of the disclosure in any way whatsoever. Percentages are by weight except where otherwise indicated.

Example A

Wettable Powder

| | |
|---|---|
| Compound 1 | 65.0% |
| dodecylphenol polyethylene glycol ether | 2.0% |
| sodium ligninsulfonate | 4.0% |
| sodium silicoaluminate | 6.0% |
| montmorillonite (calcined) | 23,0% |

Example B

Granule

| | |
|---|---|
| Compound 2 | 10.0% |

(continued)

| | |
|---|---|
| Granule | |
| attapulgite granules (low volatile matter, 0.71/0.30 mm; U.S.S. No. 25-50 sieves) | 90.0% |

Example C

| Extruded Pellet | |
|---|---|
| Compound 5 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

Example D

| Emulsifiable Concentrate | |
|---|---|
| Compound 9 | 20.0% |
| blend of oil soluble sulfonates and polyoxyethylene ethers | 10.0% |
| isophorone | 70.0% |

Example E

| Microemulsion | |
|---|---|
| Compound 30 | 5.0% |
| polyvinylpyrrolidone-vinyl acetate copolymer | 30.0% |
| alkylpolyglycoside | 30.0% |
| glyceryl monooleate | 15.0% |
| water | 20.0% |

Example F

| Seed Treatment | |
|---|---|
| Compound 31 | 20.00% |
| polyvinylpyrrolidone-vinyl acetate copolymer | 5.00% |
| montan acid wax | 5.00% |
| calcium ligninsulfonate | 1.00% |
| polyoxyethylene/polyoxypropylene block copolymers | 1.00% |
| stearyl alcohol (POE 20) | 2.00% |
| polyorganosilane | 0.20% |
| colorant red dye | 0.05% |
| water | 65.75% |

Example G

Fertilizer Stick

| | |
|---|---|
| Compound 35 | 2.50% |
| pyrrolidone-styrene copolymer | 4.80% |
| tristyrylphenyl 16-ethoxylate | 2.30% |
| talc | 0.80% |
| corn starch | 5.00% |
| Nitrophoska® Permanent 15-9-15 slow-release fertilizer (BASF) | 36.00% |
| kaolin | 38,00% |
| water | 10.60% |

Compounds of this invention are characterized by favorable metabolic and/or soil residual patterns and exhibit activity controlling a spectrum of agronomic and nonagronomic invertebrate pests. Compounds of this invention are also characterized by favorable foliar and or soil-applied systernicity in plants exhibiting translocation to protect foliage and other plant parts not directly contacted with invertebrate pest control compositions comprising the present compounds. In the context of this disclosure "invertebrate pest control" means inhibition of invertebrate pest development (including mortality, feeding reduction, and/or mating disruption) and as a result significant reduction in feeding or injury to an agronomic crop or damage to a building structure caused by the invertebrate pest; related expressions are defined analogously.

The term "agronomic" refers to the production of field crops such as for food and fiber and includes the growth of corn, soybeans and other legumes, rice, cereal (e.g., wheat, oats, barley, rye, rice, maize), leafy vegetables (e.g., lettuce, cabbage, and other cole crops), fruiting vegetables (e.g., tomatoes, pepper, eggplant, crucifers and cucurbits), potatoes, sweet potatoes, grapes, cotton, tree fruits (e.g., pome, stone and citrus), small fruit (berries, cherries) and other specialty crops (e.g., canola, sunflower, olives). The term "agronomic" also refers to the production of such crops that contain genetic material introduced by genetic engineering (i.e. transgenic) or modified by mutagenesis to provide advantageous traits. Examples of such traits include tolerance to herbicides, resistance to phytophagous pests (e.g., insects, mites, aphids, spiders, nematodes, snails, plant-pathogenic fungi, bacteria and viruses), improved plant growth, increased tolerance of adverse growing conditions such as high and low temperatures, low or high soil moisture, and high salinity, increased flowering or fruiting, greater harvest yields, more rapid maturation, higher quality and/or nutritional value of the harvested product, and improved storage or process properties of the harvested products. Transgenic plants can be modified to express multiple traits, Examples of plants containing traits provided by genetic engineering or mutagenesis include varieties of corn, cotton, soybean and potato expressing an insecticidal *Bacillus thuringiensis* toxin such as YIELD GARD®, KNOCKOUT®, STARLINK®, BOLLARD®, NuCOTN® and NEWLEAF®, and herbicide-tolerant varieties of corn, cotton, soybean and rapeseed such as ROUNDUP READY®, LIBERTY LINK®, IMI® STS® and CLEARFIELD®, as well as crops expressing N-acetyltransferase (GAT) to provide resistance to glyphosate herbicide, or crops containing the HRA gene providing resistance to herbicides inhibiting acetylactate synthase (ALS).

The term "nonagronomic" refers to other horticultural crops (e.g., greenhouse, nursery or ornamental plants not grown in a field), residential and commercial structures in urban and industrial settings, turf (commercial, golf, residential, recreational, etc.), wood products, stored product, agro-forestry and vegetation management, public health (human) and animal health (domestical animals, pets, livestock, poultry, undomestical animals such as wildlife) applications. For reasons of invertebrate pest control spectrum and economic importance, protection of agronomic crops from damage or injury caused by invertebrate pests by controlling invertebrate pests are embodiments of the invention.

As referred to in this disclosure, the term "invertebrate pest" includes arthropods, gastropods and nematodes of economic importance as pests. The term "arthropod" includes insects, mites, spiders, scorpions, centipedes, millipedes, pill bugs and symphylans. The term "gastropod" includes snails, slugs and other Stylommatophora. The term "nematode" includes all of the helminths, such as: roundworms, heartworms, and phytophagous nematodes (Nematoda), flukes (Tematoda), Acanthocephala, and tapeworms (Cestoda). Compounds of this invention exhibit activity against a wide spectrum of foliar-feeding, fruit-feeding, stem or root feeding, seed-feeding, aquatic and soil-inhabiting invertebrate pests which are pests of growing and stored agronomic crops, forestry, greenhouse crops, ornamentals, nursery crops, stored food and fiber products, livestock, household, public health and animal health. Those skilled in the art will appreciate that not all compounds are equally effective against all growth stages of all pests.

Agronomic or nonagronomic pests include eggs, larvae and adults of the order Lepidoptera, such as armyworms, cutworms, loopers, and heliothines in the family Noctuidae (e,g., fall armyworm (*Spodoptera fugiperda* J. E. Smith), beet

armyworm (*Spodoptera exigua* Hiibner), black cutworm (*Agrotis ipsilon* Hufnagel), cabbage looper (*Trichoplusia ni* Hübner), tobacco budworm (*Heliothis virescens* Fabricius)); borers, casebearers, webworms, coneworms, cabbageworms and skeletonizers from the family Pyralidae (e.g., European corn borer (*Ostrinia nubilalis* Hübner), navel orangeworm (*Amyelois transitella* Walker), corn root webworm (*Crambus caliginosellus* Clemens), sod webworms (Pyralidae: *Crambinae*) such as sod worm (*Herpetogramma licarsisalis* Walker)); leafrollers, budworms, seed worms, and fruit worms in the family Tortricidae (e.g., codling moth (*Cydia pomonella* Linnaeus), grape berry moth (*Endopiza viteana* Clemens), oriental fruit moth (*Grapholita molesta* Busck)); and many other economically important lepidoptera (e.g., diamondback moth (*Plutella xylostella* Linnaeus), pink bollworm (*Pectinophora gossypiella* Saunders), gypsy moth (*Lymantria dispar* Linnaeus)); eggs, nymphs and adults of the order Blattodea including cockroaches from the families Blattellidae and Blattidae (e.g., oriental cockroach (*Blatta orientalis* Linnaeus), Asian cockroach (*Blatella asahinai* Mizukubo), German cockroach (*Blattella germanica* Linnaeus), brownbanded cockroach (*Supella longipalpa* Fabricius), American cockroach (*Periplaneta americana* Linnaeus), brown cockroach (*Periplaneta brunnea* Burmeister), Madeira cockroach (*Leucophaea maderae* Fabricius)), smoky brown cockroach (*Periplaneta fuliginosa* Service), Australian cockroach (*Periplaneta australasiae* Fabr.), lobster cockroach (*Nauphoeta cinerea* Olivier) and smooth cockroach (*Symploce pallens* Stephens)); eggs, foliar feeding, fruit feeding, root feeding, seed feeding and vesicular tissue feeding larvae and adults of the order Coleoptera including weevils from the families Anthribidae, Bruchidae, and Curculionidae (e.g., boll weevil (*Anthonomus grandis* Boheman), rice water weevil (*Lissorhoptrus oryzophilus* Kuschel), granary weevil (*Sitophilus granarius* Linnaeus)), rice weevil (*Sitophilus oryzae* Linnaeus)), annual bluegrass weevil (*Listronotus maculicollis* Dietz), bluegrass billbug (*Sphenophorus parvulus* Gyllenhal), hunting billbug (*Sphenophorus venatus vestitus*), Denver billbug (*Sphenophorus cicatristriatus* Fahraeus)); flea beetles, cucumber beetles, rootworms, leaf beetles, potato beetles, and leafminers in the family Chrysomelidae (e.g., Colorado potato beetle (*Leptinotarsa decemlineata* Say), western corn rootworm (*Diabrotica virgifera virgifera* LeConte)); chafers and other beetles from the family Scaribaeidae (e.g., Japanese beetle (*Popillia japonica* Newman), oriental beetle (*Anomala orientalis* Waterhouse), northern masked chafer (*Cyclocephala borealis* Arrow), southern masked chafer (*Cyclocephala immaculata* Olivier), black turfgrass ataenius (*Ataenius spretulus* Haldeman), green June beetle (*Cotinis nitida* Linnaeus), Asiatic garden beetle (*Maladera castanea* Arrow), May/June beetles (*Phyllophaga* spp.) and European chafer (*Rhizotrogus majalis* Razoumowsky)); carpet beetles from the family Dermestidae; wireworms from the family Elateridae; bark beetles from the family Scolytidae and flour beetles from the family Tenebrionidae. In addition, agronomic and nonagronomic pests include: eggs, adults and larvae of the order Dermaptera including earwigs from the family Forficulidae (e.g., European earwig (*Forficula auricularia* Linnaeus), black earwig (*Chelisoches morio* Fabricius)); eggs, immatures, adults and nymphs of the orders Hemiptera and Homoptera such as, plant bugs from the family Miridae, cicadas from the family Cicadidae, leafhoppers (e.g. *Empoasca* spp.) from the family Cicadellidae, planthoppers from the families Fulgoroidae and Delphacidae, treehoppers from the family Membracidae, psyllids from the family Psyllidae, whiteflies from the family Aleyrodidae, aphids from the family Aphididae, phylloxera from the family Phylloxeridae, mealybugs from the family Pseudococcidae, scales from the families Coccidae, Diaspididae and Margarodidae, lace bugs from the family Tingidae, stink bugs from the family Pentatomidae, chinch bugs (e.g., hairy chinch bug (*Blissus leucopterus hirtus* Montandon) and southern chinch bug (*Blissus insularis* Barber)) and other seed bugs from the family Lygaeidae, spittlebugs from the family Cercopidae squash bugs from the family Coreidae, and red bugs and cotton stainers from the family Pyrrhocoridae. Also included are eggs, larvae, nymphs and adults of the order Acari (mites) such as spider mites and red mites in the family Tetranychidae (e.g., European red mite (*Panonychus ulmi* Koch), two spotted spider mite (*Tetranychus urticae* Koch), McDaniel mite (*Tetranychus mcdanieli* McGregor)); flat mites in the family Tenuipalpidae (e.g., citrus flat mite (*Brevipalpus lewisi* McGregor)); rust and bud mites in the family Eriophyidae and other foliar feeding mites and mites important in human and animal health, i.e. dust mites in the family Epidermoptidae, follicle mites in the family Demodicidae, grain mites in the family Glycyphagidae, ticks in the order Ixodidae (e.g., deer tick *(Ixodes scapularis* Say), Australian paralysis tick (*Ixodes holocyclus* Neumann), American dog tick (*Dermacentor variabilis* Say), lone star tick (*Amblyomma americanum* Linnaeus)) and scab and itch mites in the families Psoroptidae, Pyemotidae, and Sarcoptidae; eggs, adults and immatures of the order Orthoptera including grasshoppers, locusts and crickets (e.g., migratory grasshoppers (e.g., *Melanoplus sanguinipes* Fabricius, *M. differentialis* Thomas), American grasshoppers (e.g., *Schistocerca americana* Drury), desert locust (*Schistocerca gregaria* Forskal), migratory locust (*Locusta migratoria* Linnaeus), bush locust (*Zonocerus* spp.), house cricket (*Acheta domesticus* Linnaeus), mole crickets (e.g., tawny mole cricket (*Scapteriscus vicinus* Scudder) and southern mole cricket (*Scapteriscus borellii* Giglio-Tos)); eggs, adults and immatures of the order Diptera including leafminers, midges, fruit flies (Tephritidae), frit flies (e.g., *Oscinella frit* Linnaeus), soil maggots, house flies (e.g., *Musca domestica* Linnaeus), lesser house flies (e.g., *Fannia canicularis* Linnaeus, *F. femoralis* Stein), stable flies (e.g., *Stomoxys calcitrans* Linnaeus), face flies, horn flies, blow flies (e.g., *Chrysomya* spp., *Phormia* spp.), and other muscoid fly pests, horse flies (e.g., *Tabanus* spp.), bot flies (e.g., *Gastrophilus* spp., *Oestrus* spp.), cattle grubs (e.g., *Hypoderma* spp.), deer flies (e.g., *Chrysops* spp.), keds (e.g., *Melophagus ovinus* Linnaeus) and other Brachycera, mosquitoes (e.g., *Aedes* spp., *Anopheles* spp., *Culex* spp.), black flies (e.g., *Prosimulium* spp., *Simulium* spp.), biting midges, sand flies, sciarids, and other Nematocera; eggs, immatures and adults of the order Thysanoptera including onion thrips (*Thrips tabaci* Lindeman), flower thrips (*Fran-*

*kliniella* spp.), and other foliar feeding thrips; insect pests of the order Hymenoptera including ants (e.g., red carpenter ant *(Camponotus ferrugineus* Fabricius), black carpenter ant (*Camponotus pennsylvanicus* De Geer), Pharaoh ant (*Monomorium pharaonis* Linnaeus), little fire ant (*Wasmannia auropunctata* Roger), fire ant (*Solenopsis geminata* Fabricius), red imported fire ant (*Solenopsis invicta* Buren), Argentine ant (*Iridomyrmex humilis* Mayr), crazy ant (*Paratrechina longicornis* Latreille), pavement ant (*Tetramorium caespitum.* Linnaeus), cornfield ant (*Lasius alienus* Förster), odorous house ant (*Tapinoma sessile* Say), bees (including carpenter bees), hornets, yellow jackets, wasps, and sawflies (*Neodiprion* spp.; *Cephus* spp.); insect pests of the Family Formicidae including the Florida carpenter ant (*Camponotus floridanus* Buckley), white-footed ant (*Technomyrmex albipes* fr. Smith), big headed ants (*Pheidole* sp.) and ghost ant (*Tapinoma melanocephalum* Fabricius); insect pests of the order Isoptera including termites in the Termitidae (ex. *Macrotermes* sp.), Kalotermitidae (ex. *Cryptotermes* sp.), and Rhinotermitidae (ex. *Reticulitermes* sp., *Coptotermes* sp.) families, the eastern subterranean termite (*Reticulitermes flavipes* Kollar), western subterranean termite (*Reticulitermes hesperus* Banks), Formosan subterranean termite (*Coptotermes formosanus* Shiraki), West Indian drywood termite (*Incisitermes immigrans* Snyder), powder post termite (*Cryptotermes brevis* Walker), drywood termite (*Incisitermes snyderi* Light), southeastern subterranean termite (*Reticulitermes virginicus* Banks), western drywood termite (*Incisitermes minor* Hagen), arboreal termites such as *Nasutitermes* sp. and other termites of economic importance; insect pests of the order Thysanura such as silverfish (*Lepisma saccharina* Linnaeus) and firebrat (*Thermobia domestica* Packard); insect pests of the order Mallophaga and including the head louse (*Pediculus humanus capitis* De Geer), body louse (*Pediculus humanus* Linnaeus), chicken body louse (*Menacanthus stramineus* Nitszcb), dog biting louse (*Trichodectes canis* De Geer), fluff louse (*Goniocotes gallinae* De Geer), sheep body louse (*Bovicola ovis* Schrank), short-nosed cattle louse (*Haematopinus eurysternus* Nitzsch), long-nosed cattle louse (*Linognathus vituli* Linnaeus) and other sucking and chewing parasitic lice that attack man and animals; insect pests of the order Siphonoptera including the oriental rat flea (*Xenopsylla cheopis* Rothschild), cat flea (*Ctenocephalides felis* Bouche), dog flea (*Ctenocephalides canis* Curtis), hen flea (*Ceratophyllus gallinae* Schrank), sticktight flea (*Echidnophaga gallinacea* Westwood), human flea (*Pulex irritans* Linnaeus) and other fleas afflicting mammals and birds. Additional arthropod pests covered include: spiders in the order Araneae such as the brown recluse spider (*Loxosceles reclusa* Gertsch & Mulaik) and the black widow spider (*Latrodectus mactans* Fabricius), and centipedes in the order Scutigeromorpha such as the house centipede (*Scutigera coleoptrata* Linnaeus). Compounds of the present invention also have activity on members of the Classes Nematoda, Cestoda, Trematoda, and Acanthocephala including economically important members of the orders Strongylida, Ascaridida, Oxyurida, Rhabditida, Spirurida, and Enoplida such as but not limited to economically important agricultural pests (i.e. root knot nematodes in the genus *Meloidogyne,* lesion nematodes in the genus *Pratylenchus,* stubby root nematodes in the genus *Trichodorus,* etc.) and animal and human health pests (i.e. all economically important flukes, tapeworms, and roundworms, such as *Strongylus vulgaris* in horses, *Toxocara canis* in dogs, *Haemonchus contortus* in sheep, *Dirofilaria immitis* Leidy in dogs, *Anoplocephala perfoliata* in horses, *Fasciola hepatica* Linnaeus in ruminants, etc.).

Compounds of the invention show particularly high activity against pests in the order Lepidoptera (e.g., *Alabama argillacea* Hübner (cotton leaf worm), *Archips argyrospila* Walker (fruit tree leaf roller), *A. rosana* Linnaeus (European leaf roller) and other *Archips* species, *Chilo suppressalis* Walker (rice stem borer), *Cnaphalocrosis medinalis* Guenee (rice leaf roller), *Crambus caliginosellus* Clemens (corn root webworm), *Crambus teterrellus* Zincken (bluegrass webworm), *Cydia pomonella* Linnaeus (codling moth), *Earias insulana* Boisduval (spiny bollworm), *Earias vittella* Fabricius (spotted bollworm), *Helicoverpa armigera* Hübner (American bollworm), *Helicoverpa zea* Boddie (corn earworm), *Heliothis virescens* Fabricius (tobacco budworm), *Herpetogramma licarsisalis* Walker (sod webworm), *Lobesia botrana* Denis & Schiffermüller (grape berry moth), *Pectinophora gossypiella* Saunders (pink bollworm), *Phyllocnistis citrella* Stainton (citrus leafminer), *Pieris brassicae* Linnaeus (large white butterfly), *Pieris rapae* Linnaeus (small white butterfly), *Plutella xylostella* Linnaeus (diamondback moth), *Spodoptera exigua* Hübner (beet armyworm), *Spodoptera litura* Fabricius (tobacco cutworm, cluster caterpillar), *Spodoptera frugiperda* J. E. Smith (fall armyworm), *Trichoplusia ni* Hübner (cabbage looper) and *Tuta absoluta* Meyrick (tomato leafminer)).

Compounds of the invention also have significant activity on members from the order Homoptera including: *Acyrthisiphon pisum* Harris (pea aphid), *Aphis craccivora* Koch (cowpea aphid), *Aphis fabae* Scopoli (black bean aphid), *Aphis gossypii* Glover (cotton aphid, melon aphid), *Aphis pomi* De Geer (apple aphid), *Aphis spiraecola* Patch (spirea aphid), *Aulacorthum solani* Kaltenbach (foxglove aphid), *Chaetosiphon fragaefolii* Cockerell (strawberry aphid), *Diuraphis noxia* Kurdjumov/Mordvilko (Russian wheat aphid), *Dysaphis plantaginea* Paaserini (rosy apple aphid), *Eriosoma lanigerum* Hausmann (woolly apple aphid), *Hyalopterus pruni* Geoffroy (mealy plum aphid), *Lipaphis erysimi* Kaltenbach (turnip aphid), *Metopolophium dirrhodum* Walker (cereal aphid), *Macrosipum euphorbiae* Thomas (potato aphid), *Myzus persicae* Sulzer (peach-potato aphid, green peach aphid), *Nasonovia ribisnigri* Mosley (lettuce aphid), *Pemphigus* spp. (root aphids and gall aphids), *Rhopalosiphum maidis* Fitch (corn leaf aphid), *Rhopalosiphum padi* Linnaeus (bird cherry-oat aphid), *Schizaphis graminum* Rondani (greenbug), *Sitobion avenae* Fabricius (English grain aphid), *Therioaphis maculata* Buckton (spotted alfalfa aphid), *Toxoptera aurantii* Boyer de Fonscolombe (black citrus aphid), and *Toxoptera citricida* Kirkaldy (brown citrus aphid); *Adelges* spp. (adelgids); *Phylloxera devastatrix* Pergande (pecan phylloxera);

*Bemisia tabaci* Gennadius (tobacco whitefly, sweetpotato whitefly), *Bemisia argentifolii* Bellows & Perring (silverleaf whitefly), *Dialeurodes citri* Ashmead (citrus whitefly) and *Trialeurodes vaporariorum* Westwood (greenhouse whitefly); *Empoasca fabae* Harris (potato leafhopper), *Laodelphax striatellus* Fallen (smaller brown planthopper), *Macrolestes quadrilineatus* Forbes (aster leafhopper), *Nephotettix cinticeps* Uhler (green leafhopper), *Nephotettix nigropictus* Stål (rice leafhopper), *Nilaparvata lugens* Stål (brown planthopper), *Peregrinus maidis* Ashmead (corn planthopper), *Sogatella furcifera* Horvath (white-backed planthopper), *Sogatodes orizicola* Muir (rice delphacid), *Typhlocyba pomaria* McAtee white apple leafhopper, *Erythroneoura* spp. (grape leafhoppers); *Magicidada septendecim* Linnaeus (periodical cicada); *Icerya purchasi* Maskell (cottony cushion scale), *Quadraspidiotus perniciosus* Comstock (San Jose scale); *Planococcus citri* Risso (citrus mealybug); *Pseudococcus* spp. (other mealybug complex); *Cacopsylla pyricola* Foerster (pear psylla), *Trioza diospyri* Ashmead (persimmon psylla).

Compounds of this invention also have activity on members from the order Hemiptera including: *Acrosternum hilare* Say (green stink bug), *Anasa tristis* De Geer (squash bug), *Blissus leucopterus leucopterus* Say (chinch bug), *Corythuca gossypii* Fabricius (cotton lace bug), *Cyrtopeltis modesta* Distant (tomato bug), *Dysdercus suturellus* Herrich-Schäffer (cotton stainer), *Euchistus servus* Say (brown stink bug), *Euchistus variolarius* Palisot de Beauvois (one-spotted stink bug), *Graptosthetus* spp. (complex of seed bugs), *Leptoglossus corculus* Say (leaf-footed pine seed bug), *Lygus lineolaris* Palisot de Beauvois (tarnished plant bug), *Nezara viridula* Linnaeus (southern green stink bug), *Oebalus pugnax* Fabricius (rice stink bug), *Oncopeltus fasciatus* Dallas (large milkweed bug), *Pseudatomoscelis seriatus* Reuter (cotton fleahopper). Other insect orders controlled by compounds of the invention include Thysanoptera (e.g., *Frankliniella occidentalis* Pergande (western flower thrip), *Scirthothrips citri* Moulton (citrus thrip), *Sericothrips variabilis* Beach (soybean thrip), and *Thrips tabaci* Lindeman (onion thrip); and the order Coleoptera (e.g., *Leptinotarsa decemlineata* Say (Colorado potato beetle), *Epilachna varivestis* Mulsant (Mexican bean beetle) and wireworms of the genera *Agriotes, Athous* or *Limonius*).

Of note is use of compounds of this invention for controlling silverleaf whitefly (*Bemisia argentifolii*). Of note is use of compounds of this invention for controlling potato leafhopper (*Empoasca fabae*). Of note is use of compounds of this invention for controlling corn planthopper (*Peregrinus maidis*). Of note is use of compounds of this invention for controlling cotton melon aphid (*Aphis gossypii*). Of note is use of compounds of this invention for controlling green peach aphid (*Myzus persicae*). Of note is use of compounds of this invention for controlling diamondback moth (*Plutella xylostella*). Of note is use of compounds of this invention for controlling fall armyworm (*Spodoptera frugiperda*).

Compounds of this invention can also be mixed with one or more other biologically active compounds or agents including insecticides, fungicides, nematocides, bactericides, acaricides, herbicides, growth regulators such as rooting stimulants, chemosterilants, semiochemicals, repellents, attractants, pheromones, feeding stimulants, other biologically active compounds or entomopathogenic bacteria, virus or fungi to form a multi-component pesticide giving an even broader spectrum of agronomic and non agronomic utility. Thus the present invention also pertains to a composition comprising a biologically effective amount of a compound of Formula 1 and an effective amount of at least one additional biologically active compound or agent and can further comprise at least one of a surfactant, a solid diluent or a liquid diluent. The other biologically active compounds or agents can be formulated in compositions comprising at least one of a surfactant, solid or liquid diluent. For mixtures of the present invention, the other biologically active compounds or agents can be formulated together with the present compounds, including the compounds of Formula 1, to form a premix, or the other biologically active compounds or agents can be formulated separately from the present compounds, including the compounds of Formula 1, and the two formulations combined together before application (e.g., in a spray tank) or, alternatively, applied in succession.

Examples of such biologically active compounds or agents with which compounds of this invention can be formulated are: insecticides such as abamectin, acephate, acetamiprid, amidoflumet (S-1955), avermectin, azadirachtin, azinphos-methyl, bifenthrin, bifenazate, buprofezin, carbofuran, cartap, chlorfenapyr, chlorfluazuron, chlorpyrifos, chlorpyrifos-methyl, chromafenozide, clothianidin, cyflumetofen, cyfluthrin, beta-cyfluthrin, cyhalothrin, lambda-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim (UR-50701), flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, monocrotophos, methoxyfenozide, nitenpyram, nithiazine, novaluron, noviflumuron (XDE-007), oxamyl, parathion, parathion-methyl, permethrin, phorate, phosalone, phosmet, phosphamidon, pirimicarb, profenofos, profluthrin, pymetrozine, pyrafluprole, Pyrethrin, pyridalyl, pyriprole, pyriproxyfen, rotenone, ryanodine, spinosad, spirodiclofen, spiromesifen (BSN 2060), spirotetramat, sulprofos, tebufenozide, teflubenzuron, tefluthrin, terbufos, tetrachlorvinphos, thiacloprid, thiamethoxam, thiodicarb, thiosultap-sodium, tralomethrin, triazamate, trichlorfon and triflumuron; fungicides such as acibenzolar, amisulbrom, azoxystrobin, benomyl, blasticidin-S, Bordeaux mixture (tribasic copper sulfate), boscalid, bromuconazole, carpropamid, captafol, captan, carbendazim, chloroneb, chlorothalonil, copper oxychloride, copper salts, cyflufenamid, cymoxanil, cyproconazole, cyprodinil, (S)-3,5-dichloro-N-(3-chloro-1-ethyl-1-methyl-2-oxopropyl)-4-methylbenzamide (RH 7281), diclocymet (S-2900), diclomezine, dicloran, difenoconazole, (*S*)-3,5-dihydro-5-methyl-2-(methylthio)-5-phenyl-3-(phenylamino)-4*H*-imidazol-4-one (RP 407213), dimethomorph, dimoxystrobin, diniconazole, diniconazole-M, dodine, edifenphos, epoxiconazole, famoxadone, fenamidone, fenarimol, fenbuconazole, fencaramid (SZX0722), fenpiclonil, fenpropidin, fenpropimorph, fentin acetate, fentin hydroxide, fluazinam, fluclioxonil, flumetover (RPA 403397), flumorf/flumorlin (SYP-L190), fluoxastrobin (HEC 5725), fluopicolide, fluquinconazole, flusilazole, flutolanil, flutriafol, folpet, fosetyl-aluminium, furalaxyl, furametapyr (S-82658), hexaconazole, ipconazole, iprobenfos, iprodione, isoprothiolane, kasugamycin, kresoxim-methyl, mancozeb, mandipropamid, maneb, mefenoxam, mepronil, metalaxyl, metconazole, metominostrobin/fenominostrobin (SSF-126), metrafenone (AC375839), myclobutanil, neo-asozin (ferric methanearsonate), nicobifen (BAS 510), orysastrobin, oxadixyl, penconazole, pencycuron, probenazole, penthiopyrad, prochloraz, propamocarb, propiconazole, proquinazid (DPX-KQ926), prothioconazole (JAU 6476), pyrifenox, pyraclostrobin, pyrimethanil, pyroquilon, quinoxyfen, spiroxamine, sulfur, tebuconazole, tetraconazole, thiabendazole, thifluzamide, thiophanate-methyl, thiram, tiadinil, triadimefon, triadimenol, tricyclazole, trifloxystrobin, triticonazole, validamycin and vinclozolin; nematocides such as aldicarb, oxamyl and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents including entomopathogenic bacteria, such as *Bacillus thuringiensis* subsp. *Aizawai, Bacillus thuringiensis* subsp. *Kurstaki,* and the encapsulated delta-endotoxins of *Bacillus thuringiensis* (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV.

Compounds of this invention and compositions thereof can be applied to plants genetically transformed to express proteins toxic to invertebrate pests (such as *Bacillus thuringiensis* delta-endotoxins). The effect of the exogenously applied invertebrate pest control compounds of this invention may be synergistic with the expressed toxin proteins.

General references for these agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

In certain instances, combinations with other arthropodicides having a similar spectrum of control but a different mode of action will be particularly advantageous for resistance management. Thus, compositions of the present invention can further comprise a biologically effective amount of at least one additional invertebrate pest control compound or agent having a similar spectrum of control but a different mode of action. Contacting a plant genetically modified to express a plant protection compound (e.g., protein) or the locus of the plant with a biologically effective amount of a compound of this invention can also provide a broader spectrum of plant protection and be advantageous for resistance management.

In certain instances, combinations of a compound of this invention with other invertebrate pest control compounds or agents can result in a greater-than-additive (i.e. synergistic) effect and/or a less-than-additive effect (i.e. antagonistic). It is always desirable to reduce the quantity of chemical agents released in the environment while ensuring effective pest control. When synergism of invertebrate pest control agents is found at application rates giving agronomically satisfactory levels of pest control, such combinations can be advantageous for lowering crop production cost and reducing environmental load.

Table A lists specific combinations of a compound of Formula 1 with other invertebrate pest control agents illustrative of the mixtures, compositions and methods of the present invention. The first column of Table A lists the specific invertebrate pest control agents (e.g., "Abamectin" in the first line). The second column of Table A lists the mode of action (if known) of the invertebrate pest control agents. The third column of Table A lists embodiment(s) of ranges of weight ratios for rates at which the invertebrate pest control agent can be applied relative to a compound of Formula 1, an *N*-oxide, or a salt thereof, (e.g., "50:1 to 1:50" of abamectin relative to a compound of Formula 1 by weight). Thus, for example, the first line of Table A specifically discloses the combination of a compound of Formula 1 with abamectin can be applied in a weight ratio between 50:1 to 1:50. The remaining lines of Table A are to be construed similarly. Of further note Table A lists specific combinations of a compound of Formula 1 with other invertebrate pest control agents illustrative of the mixtures, compositions and methods of the present invention and includes additional embodiments of weight ratio ranges for application rates, some of the specific mixtures showing notable synergistic effect.

Table A

| Invertebrate Pest Control Agent | Mode of Action or chemical classes | Typical Weight Ratio |
|---|---|---|
| Abamectin | macrocyclic lactones | 50:1 to 1:50 |
| Acetamiprid | neonicotinoids | 150:1 to 1:200 |
| Amitraz | octopamine receptor ligands | 200:1 to 1:100 |

(continued)

| Invertebrate Pest Control Agent | Mode of Action or chemical classes | Typical Weight Ratio |
|---|---|---|
| Anthranilamides | ryanodine receptor ligands | 100:1 to 1:120 |
| Avermectin | macrocyclic lactones | 50:1 to 1:50 |
| Azadirachtin | ecdysone agonists | 100:1 to 1:120 |
| Beta-cyfluthrin | sodium channel modulators | 150:1 to 1:200 |
| Bifenthrin | sodium channel modulators | 100:1 to 1:10 |
| Buprofezin | chitin synthesis inhibitors | 500:1 to 1:50 |
| Cartap | nereistoxin analogs | 100:1 1 to 1:200 |
| Chlorfenapyr | mitochondrial electron transport inhibitors | 300:1 to 1:200 |
| Chlorpyrifos | cholinesterase inhibitors | 500:1 to 1:200 |
| Clothianidin | neonicotinoids | 100:1 to 1:400 |
| Cyfluthrin | sodium channel modulators | 150:1 to 1:200 |
| Cyhalothrin | sodium channel modulators | 150:1 to 1:200 |
| Cypermethrin | sodium channel modulators | 150:1 to 1:200 |
| Cyromazine | chitin synthesis inhibitors | 400:1 to 1:50 |
| Deltamethrin | sodium channel modulators | 50:1 to 1:400 |
| Dieldrin | cyclodiene insecticides | 200:1 to 1:100 |
| Dinotefuran | neonicotinoids | 150:1 to 1:200 |
| Diofenolan | molting inhibitor | 150:1 to 1:200 |
| Emamectin | macrocyclic lactones | 50:1 to 1:10 |
| Emamectin Benzoate | macrocyclic lactones | 50:1 to 1:10 |
| Endosulfan | cyclodiene insecticides | 200:1 to 1:100 |
| Esfenvalerate | sodium channel modulators | 100:1 to 1:400 |
| Ethiprole | GABA-regulated chloride channel blockers | 200:1 to 1:100 |
| Fenothiocarb |  | 150:1 to 1:200 |
| Fenoxycarb | juvenile hormone mimics | 500:1 to 1:100 |
| Fenvalerate | sodium channel modulators | 150:1 to 1:200 |
| Fipronil | GABA-regulated chloride channel blockers | 150:1 to 1:100 |
| Flonicamid |  | 200:1 to 1:100 |
| Flubendiamide | ryanodine receptor ligands | 100:1 to 1:120 |
| Flufenoxuron | chitin synthesis inhibitors | 200:1 to 1:100 |
| Hexaflumuron | chitin synthesis inhibitors | 300:1 to 1:50 |
| Hydramethylnon | mitochondrial electron transport inhibitors | 150:1 to 1:250 |
| Imidacloprid | neonicotinoids | 1000:1 to 1:1000 |
| Indoxacarb | sodium channel modulators | 200:1 to 1:50 |
| Lambda-cyhalothrin | sodium channel modulators | 50:1 to 1:250 |
| Lufenuron | chitin synthesis inhibitors | 500:1 to 1:250 |
| Metaflumizone |  | 200:1 to 1:200 |
| Methomyl | cholinesterase inhibitors | 500:1 to 1:100 |

(continued)

| Invertebrate Pest Control Agent | Mode of Action or chemical classes | Typical Weight Ratio |
|---|---|---|
| Methoprene | juvenile hormone mimics | 500:1 to 1:100 |
| Methoxyfenozide | ecdysone agonists | 50:1 to 1:50 |
| Nitenpyram | neonicotinoids | 150:1 to 1:200 |
| Nithiazine | neonicotinoids | 150:1 to 1:200 |
| Novaluron | chitin synthesis inhibitors | 500:1 to 1:150 |
| NPV (e.g., Gemstar) | biological agents | 50:1 to 1:10 |
| Oxamyl | cholinesterase inhibitors | 200:1 to 1:200 |
| Pymetrozine | | 200:1 to 1: 100 |
| Pyrethrin | sodium channel modulators | 100:1 to 1:10 |
| Pyridaben | mitochondrial electron transport inhibitors | 200:1 to 1:100 |
| Pyridalyl | | 200:1 to 1:100 |
| Pyriproxyfen | juvenile hormone mimics | 500:1 to 1:100 |
| Ryanodine | ryanodine receptor ligands | 100:1 to 1:120 |
| Spinosad | macrocyclic lactones | 500:1 to 1:10 |
| Spirodiclofen | lipid biosynthesis inhibitors | 200:1 to 1:200 |
| Spiromesifen | lipid biosynthesis inhibitors | 200:1 to 1:200 |
| Tebufenozide | ecdysone agonists | 500:1 to 1:250 |
| Thiacloprid | neonicotinoids | 100:1 to 1:200 |
| Thiamethoxam | neonicotinoids | 1250:1 to 1:1000 |
| Thiodicarb | cholinesterase inhibitors | 500:1 to 1:400 |
| Tralomethrin | sodium channel modulators | 150:1 to 1:200 |
| Triazamate | cholinesterase inhibitors | 250:1 to 1:100 |
| Triflumuron | chitin synthesis inhibitors | 200:1 to 1:100 |
| *Bacillus thuringiensis* | biological agents | 50:1 to 1:10 |
| *Bacillus thuringiensis* delta toxin | biological agents | 50:1 to 1:10 |
| *Beauvaria bassiana* | biological agents | 50:1 to 1:10 |

One embodiment of insecticides and acaricides for mixing with compounds of this invention include sodium channel modulators such as cypermethrin, cyhalothrin, cyfluthrin, beta-cyfluthrin, esfenvalerate, fenvalerate, indoxacarb and tralomethrin; cholinesterase inhibitors such as methomyl, oxamyl and thiodicarb; neonicotinoids such as acetamiprid, clothianidin, imidacloprid, thiacloprid and thiamethoxam; neuronal sodium channel blockers such as indoxacarb; insecticidal macrocyclic lactones such as spinosad, abamectin, avermectin and emamectin; GABA ($\gamma$-aminobutyric acid)-regulated chloride channel blockers such as endosulfan, ethiprole and fipronil; chitin synthesis inhibitors such as flufenoxuron and triflumuron; juvenile hormone mimics such as diofenolan and pyriproxyfen; octopamine receptor ligands such as amitraz; ecdysone agonists such as methoxyfenozide and tebufenozide; ryanodine receptor ligands such as ryanodine, anthranilamides and flubendiamide; fenothiocarb; flonicamid; metaflumizone; pyridalyl; and pymetrozine. One embodiment of biological agents for mixing with compounds of this invention include *Bacillus thuringiensis* and *Bacillus thuringiensis* delta-endotoxin as well as naturally occurring and genetically modified viral insecticides including members of the family Baculoviridae as well as entomophagous fungi.

The weight ratios of a compound, including a compound of Formula 1, an N-oxide or a salt thereof, to the additional invertebrate pest control agent typically are between 1000:1 and 1:1000, with one embodiment being between 500:1 and 1:500, another embodiment being between 250:1 and 1:200 and another embodiment being between 100:1 and 1:50.

Listed below in Table B are embodiments of specific compositions comprising a mixture of the present invention and/or a compound of Formula 1 (compound numbers refer to compounds in Index Table A) and an additional invertebrate pest control agent.

Table B

| Mixture No. | Comp. No. | and | Invertebrate Pest Control Agent | Mixture No. | Comp. No. | and | Invertebrate Pest Control Agent |
|---|---|---|---|---|---|---|---|
| A-1 | 1 | and | Abamectin | B-1 | 2 | and | Abamectin |
| A-2 | 1 | and | Acetamiprid | B-2 | 2 | and | Acetamiprid |
| A-3 | 1 | and | Amitraz | B-3 | 2 | and | Amitraz |
| A-4 | 1 | and | Anthranilamides | B-4 | 2 | and | Anthranilamides |
| A-5 | 1 | and | Avermectin | B-5 | 2 | and | Avermectin |
| A-6 | 1 | and | Azadirachtin | B-6 | 2 | and | Azadirachtin |
| A-7 | 1 | and | Beta-cyfluthrin | B-7 | 2 | and | Beta-cyfluthrin |
| A-8 | 1 | and | Bifenthrin | B-8 | 2 | and | Bifenthrin |
| A-9 | 1 | and | Buprofezin | B-9 | 2 | and | Buprofezin |
| A-10 | 1 | and | Cartap | B-10 | 2 | and | Cartap |
| A-11 | 1 | and | Chlorfenapyr | B-11 | 2 | and | Chlorfenapyr |
| A-12 | 1 | and | Chlorpyrifos | B-12 | 2 | and | Chlorpyrifos |
| A-13 | 1 | and | Clothianidin | B-13 | 2 | and | Clothianidin |
| A-14 | 1 | and | Cyfluthrin | B-14 | 2 | and | Cyfluthrin |
| A-15 | 1 | and | Cyhalothrin | B-15 | 2 | and | Cyhalothrin |
| A-16 | 1 | and | Cypermethrin | B-16 | 2 | and | Cypermethrin |
| A-17 | 1 | and | Cyromazine | B-17 | 2 | and | Cyromazine |
| A-18 | 1 | and | Deltamethrin | B-18 | 2 | and | Deltamethrin |
| A-19 | 1 | and | Dieldrin | B-19 | 2 | and | Dieldrin |
| A-20 | 1 | and | Dinotefuran | B-20 | 2 | and | Dinotefuran |
| A-21 | 1 | and | Diofenolan | B-21 | 2 | and | Diofenolan |
| A-22 | 1 | and | Emamectin | B-22 | 2 | and | Emamectin |
| A-23 | 1 | and | Emamectin Benzoate | B-23 | 2 | and | Emamectin Benzoate |
| A-24 | 1 | and | Endosulfan | B-24 | 2 | and | Endosulfan |
| A-25 | 1 | and | Esfenvalerate | B-25 | 2 | and | Esfenvalerate |
| A-26 | 1 | and | Ethiprole | B-26 | 2 | and | Ethiprole |
| A-27 | 1 | and | Fenothiocarb | B-27 | 2 | and | Fenothiocarb |
| A-28 | 1 | and | Fenoxycarb | B-28 | 2 | and | Fenoxycarb |
| A-29 | 1 | and | Fenvalerate | B-29 | 2 | and | Fenvalerate |
| A-30 | 1 | and | Fipronil | B-30 | 2 | and | Fipronil |
| A-31 | 1 | and | Flonicamid | B-31 | 2 | and | Flonicamid |
| A-32 | 1 | and | Flubendiamide | B-32 | 2 | and | Flubendiamide |
| A-33 | 1 | and | Flufenoxuron | B-33 | 2 | and | Flufenoxuron |
| A-34 | 1 | and | Hexaflumuron | B-34 | 2 | and | Hexaflumuron |
| A-35 | 1 | and | Hydramethylnon | B-35 | 2 | and | Hydramethylnon |
| A-36 | 1 | and | Imidacloprid | B-36 | 2 | and | Imidacloprid |
| A-37 | 1 | and | Indoxacarb | B-37 | 2 | and | Indoxacarb |
| A-38 | 1 | and | Lambda-cyhalothrin | B-38 | 2 | and | Lambda-cyhalothrin |
| A-39 | 1 | and | Lufenuron | B-39 | 2 | and | Lufenuron |
| A-40 | 1 | and | Metaflumizone | B-40 | 2 | and | Metaflumizone |
| A-41 | 1 | and | Methomyl | B-41 | 2 | and | Methomyl |
| A-42 | 1 | and | Methoprene | B-42 | 2 | and | Methoprene |
| A-43 | 1 | and | Methoxyfenozide | B-43 | 2 | and | Methoxyfenozide |
| A-44 | 1 | and | Nitenpyram | B-44 | 2 | and | Nitenpyram |
| A-45 | 1 | and | Nithiazine | B-45 | 2 | and | Nithiazine |

(continued)

| Mixture No. | Comp. No. | and | Invertebrate Pest Control Agent | Mixture No. | Comp. No. | and | Invertebrate Pest Control Agent |
|---|---|---|---|---|---|---|---|
| A-46 | 1 | and | Novaluron | B-46 | 2 | and | Novaluron |
| A-47 | 1 | and | NPV (e.g., Gemstar) | B-47 | 2 | and | NPV (e.g., Gemstar) |
| A-48 | 1 | and | Oxamyl | B-48 | 2 | and | Oxamyl |
| A-49 | 1 | and | Pymetrozine | B-49 | 2 | and | Pymetrozine |
| A-50 | 1 | and | Pyrethrin | B-50 | 2 | and | Pyrethrin |
| A-51 | I | and | Pyridaben | B-51 | 2 | and | Pyridaben |
| A-52 | I | and | Pyridalyl | B-52 | 2 | and | Pyridalyl |
| A-53 | 1 | and | Pyriproxyfen | B-53 | 2 | and | Pyriproxyfen |
| A-54 | 1 | and | Ryanodine | B-54 | 2 | and | Ryanodine |
| A-55 | 1 | and | Spinosad | B-55 | 2 | and | Spinosad |
| A-56 | 1 | and | Spirodiclofen | B-56 | 2 | and | Spirodiclofen |
| A-57 | 1 | and | Spiromesifen | B-57 | 2 | and | Spiromesifen |
| A-58 | 1 | and | Tebufenozide | B-58 | 2 | and | Tebufenozide |
| A-59 | 1 | and | Thiacloprid | B-59 | 2 | and | Thiacloprid |
| A-60 | 1 | and | Thiamethoxam | B-60 | 2 | and | Thiamethoxam |
| A-61 | 1 | and | Thiodicarb | B-61 | 2 | and | Thiodicarb |
| A-62 | 1 | and | Tralomethrin | B-62 | 2 | and | Tralomethrin |
| A-63 | 1 | and | Triazamate | B-63 | 2 | and | Triazamate |
| A-64 | 1 | and | Triflumuron | B-64 | 2 | and | Triflumuron |
| A-65 | 1 | and | *Bacillus thuringiensis* | B-65 | 2 | and | *Bacillus thuringiensis* |
| A-66 | 1 | and | *Bacillus thuringiensis* delta toxin | B-66 | 2 | and | *Bacillus thuringiensis* delta toxin |
| A-67 | 1 | and | *Beauvaria bassiana* | B-67 | 2 | and | *Beauvaria bassiana* |
| C-1 | 3 | and | Abamectin | D-1 | 5 | and | Abamectin |
| C-2 | 3 | and | Acetamiprid | D-2 | 5 | and | Acetamiprid |
| C-3 | 3 | and | Amitraz | D-3 | 5 | and | Amitraz |
| C-4 | 3 | and | Anthianilamides | D-4 | 5 | and | Anthranilamides |
| C-5 | 3 | and | Avermectin | D-5 | 5 | and | Avermectin |
| C-6 | 3 | and | Azadirachtin | D-6 | 5 | and | Azadirachtin |
| C-7 | 3 | and | Beta-cyfluthrin | D-7 | 5 | and | Beta-cyfluthrin |
| C-8 | 3 | and | Bifenthrin | D-8 | 5 | and | Bifenthrin |
| C-9 | 3 | and | Buprofezin | D-9 | 5 | and | Buprofezin |
| C-10 | 3 | and | Cartap | D-10 | 5 | and | Cartap |
| C-11 | 3 | and | Chlorfenapyr | D-11 | 5 | and | Chlorfenapyr |
| C-12 | 3 | and | Chlorpyrifos | D-12 | 5 | and | Chlorpyrifos |
| C-13 | 3 | and | Clothianidin | D-13 | 5 | and | Clothianidin |
| C-14 | 3 | and | Cyfluthrin | D-14 | 5 | and | Cyfluthrin |
| C-15 | 3 | and | Cyhalothrin | D-15 | 5 | and | Cyhalothrin |
| C-16 | 3 | and | Cypermethrin | D-16 | 5 | and | Cypermethrin |
| C-17 | 3 | and | Cyromazine | D-17 | 5 | and | Cyromazine |
| C-18 | 3 | and | Deltamethrin | D-18 | 5 | and | Deltamethrin |
| C-19 | 3 | and | Dieldrin | D-19 | 5 | and | Dieldrin |
| C-20 | 3 | and | Dinotefuran | D-20 | 5 | and | Dinotefuran |
| C-21 | 3 | and | Diofenolan | D-21 | 5 | and | Diofenolan |
| C-22 | 3 | and | Emamectin | D-22 | 5 | and | Emamectin |
| C-23 | 3 | and | Emamectin Benzoate | D-23 | 5 | and | Emamectin Benzoate |
| C-24 | 3 | and | Endosulfan | D-24 | 5 | and | Endosulfan |
| C-25 | 3 | and | Esfenvalerate | D-25 | 5 | and | Esfenvalerate |

(continued)

| Mixture No. | Comp. No. | and | Invertebrate Pest Control Agent | Mixture No. | Comp. No. | and | Invertebrate Pest Control Agent |
|---|---|---|---|---|---|---|---|
| C-26 | 3 | and | Ethiprole | D-26 | 5 | and | Ethiprole |
| C-27 | 3 | and | Fenothiocarb | D-27 | 5 | and | Fenothiocarb |
| C-28 | 3 | and | Fenoxycarb | D-28 | 5 | and | Fenoxycarb |
| C-29 | 3 | and | Fenvalerate | D-29 | 5 | and | Fenvalerate |
| C-30 | 3 | and | Fipronil | D-30 | 5 | and | Fipronil |
| C-31 | 3 | and | Flonicamid | D-31 | 5 | and | Flonicamid |
| C-32 | 3 | and | Flubendiamide | D-32 | 5 | and | Flubendiamide |
| C-33 | 3 | and | Flufenoxuron | D-33 | 5 | and | Flufenoxuron |
| C-34 | 3 | and | Hexaflumuron | D-34 | 5 | and | Hexaflumuron |
| C-35 | 3 | and | Hydramethylnon | D-35 | 5 | and | Hydramethylnon |
| C-36 | 3 | and | Imidacloprid | D-36 | 5 | and | Imidacloprid |
| C-37 | 3 | and | Indoxacarb | D-37 | 5 | and | Indoxacarb |
| C-38 | 3 | and | Lambda-cyhalothrin | D-38 | 5 | and | Lambda-cyhalothrin |
| C-39 | 3 | and | Lufenuron | D-39 | 5 | and | Lufenuron |
| C-40 | 3 | and | Metaflumizone | D-40 | 5 | and | Metaflumizone |
| C-41 | 3 | and | Methomyl | D-41 | 5 | and | Methomyl |
| C-42 | 3 | and | Methoprene | D-42 | 5 | and | Methoprene |
| C-43 | 3 | and | Methoxyfenozide | D-43 | 5 | and | Methoxyfenozide |
| C-44 | 3 | and | Nitenpyram | D-44 | 5 | and | Nitenpyram |
| C-45 | 3 | and | Nithiazine | D-45 | 5 | and | Nithiazine |
| C-46 | 3 | and | Novaluron | D-46 | 5 | and | Novaluron |
| C-47 | 3 | and | NPV (e.g., Gemstar) | D-47 | 5 | and | NPV (e.g., Gemstar) |
| C-48 | 3 | and | Oxamyl | D-48 | 5 | and | Oxamyl |
| C-49 | 3 | and | Pymetrozine | D-49 | 5 | and | Pymetrozine |
| C-50 | 3 | and | Pyrethrin | D-50 | 5 | and | Pyrethrin |
| C-51 | 3 | and | Pyridaben | D-51 | 5 | and | Pyridaben |
| C-52 | 3 | and | Pyridalyl | D-52 | 5 | and | Pyridalyl |
| C-53 | 3 | and | Pyriproxyfen | D-53 | 5 | and | Pyriproxyfen |
| C-54 | 3 | and | Ryanodine | D-54 | 5 | and | Ryanodine |
| C-55 | 3 | and | Spinosad | D-55 | 5 | and | Spinosad |
| C-56 | 3 | and | Spirodiclofen | D-56 | 5 | and | Spirodiclofen |
| C-57 | 3 | and | Spiromesifen | D-57 | 5 | and | Spiromesifen |
| C-58 | 3 | and | Tebufenozide | D-58 | 5 | and | Tebufenozide |
| C-59 | 3 | and | Thiacloprid | D-59 | 5 | and | Thiacloprid |
| C-60 | 3 | and | Thiamethoxam | D-60 | 5 | and | Thiamethoxam |
| C-61 | 3 | and | Thiodicarb | D-61 | 5 | and | Thiodicarb |
| C-62 | 3 | and | Tralomethrin | D-62 | 5 | and | Tralomethrin |
| C-63 | 3 | and | Triazamate | D-63 | 5 | and | Triazamate |
| C-64 | 3 | and | Triflumuron | D-64 | 5 | and | Triflumuron |
| C-65 | 3 | and | *Bacillus thuringiensis* | D-65 | 5 | and | *Bacillus thuringiensis* |
| C-66 | 3 | and | *Bacillus thuringiensis* delta toxin | D-66 | 5 | and | *Bacillus thuringiensis* delta toxin |
| C-67 | 3 | and | *Beauvaria bassiana* | D-67 | 5 | and | *Beauvaria bassiana* |
| E-1 | 23 | and | Abamectin | F-1 | 24 | and | Abamectin |
| E-2 | 23 | and | Acetamiprid | F-2 | 24 | and | Acetamiprid |
| E-3 | 23 | and | Amitraz | F-3 | 24 | and | Amitraz |
| E-4 | 23 | and | Anthranilamides | F-4 | 24 | and | Anthranilamides |
| E-5 | 23 | and | Avermectin | F-5 | 24 | and | Avermectin |

(continued)

| Mixture No. | Comp. No. | and | Invertebrate Pest Control Agent | Mixture No. | Comp. No. | and | Invertebrate Pest Control Agent |
|---|---|---|---|---|---|---|---|
| E-6 | 23 | and | Azadirachtin | F-6 | 24 | and | Azadirachtin |
| E-7 | 23 | and | Beta-cyfluthrin | F-7 | 24 | and | Beta-cyfluthrin |
| E-8 | 23 | and | Bifenthrin | F-8 | 24 | and | Bifenthrin |
| E-9 | 23 | and | Buprofezin | F-9 | 24 | and | Buprofezin |
| E-10 | 23 | and | Cartap | F-10 | 24 | and | Cartap |
| E-11 | 23 | and | Chlorfenapyr | F-11 | 24 | and | Chlorfenapyr |
| E-12 | 23 | and | Chlorpyrifos | F-12 | 24 | and | Chlorpyrifos |
| E-13 | 23 | and | Clothianidin | F-13 | 24 | and | Clothianidin |
| E-14 | 23 | and | Cyfluthrin | F-14 | 24 | and | Cyfluthrin |
| E-15 | 23 | and | Cyhalothrin | F-15 | 24 | and | Cyhalothrin |
| E-16 | 23 | and | Cypermethrin | F-16 | 24 | and | Cypermethrin |
| E-17 | 23 | and | Cyromazine | F-17 | 24 | and | Cyromazine |
| E-18 | 23 | and | Deltamethrin | F-18 | 24 | and | Deltamethrin |
| E-19 | 23 | and | Dieldrin | F-19 | 24 | and | Dieldrin |
| E-20 | 23 | and | Dinotefuran | F-20 | 24 | and | Dinotefuran |
| E-21 | 23 | and | Diofenolan | F-21 | 24 | and | Diofenolan |
| E-22 | 23 | and | Emamectin | F-22 | 24 | and | Emamectin |
| E-23 | 23 | and | Emamectin Benzoate | F-23 | 24 | and | Emamectin Benzoate |
| E-24 | 23 | and | Endosulfan | F-24 | 24 | and | Endosulfan |
| E-25 | 23 | and | Esfenvalerate | F-25 | 24 | and | Esfenvalerate |
| E-26 | 23 | and | Ethiprole | F-26 | 24 | and | Ethiprole |
| E-27 | 23 | and | Fenothiocarb | F-27 | 24 | and | Fenothiocarb |
| E-28 | 23 | and | Fenoxycarb | F-28 | 24 | and | Fenoxycarb |
| E-29 | 23 | and | Fenvalerate | F-29 | 24 | and | Fenvalerate |
| E-30 | 23 | and | Fipronil | F-30 | 24 | and | Fipronil |
| E-31 | 23 | and | Flonicamid | F-31 | 24 | and | Flonicamid |
| E-32 | 23 | and | Flubendiamide | F-32 | 24 | and | Flubendiamide |
| E-33 | 23 | and | Flufenoxuron | F-33 | 24 | and | Flufenoxuron |
| E-34 | 23 | and | Hexaflumuron | F-34 | 24 | and | Hexaflumuron |
| E-35 | 23 | and | Hydramethylnon | F-35 | 24 | and | Hydramethylnon |
| E-36 | 23 | and | Imidacloprid | F-36 | 24 | and | Imidacloprid |
| E-37 | 23 | and | Indoxacarb | F-37 | 24 | and | Indoxacarb |
| E-38 | 23 | and | Lambda-cyhalothrin | F-38 | 24 | and | Lambda-cyhalothrin |
| E-39 | 23 | and | Lufenuron | F-39 | 24 | and | Lufenuron |
| E-40 | 23 | and | Metaflumizone | F-40 | 24 | and | Metaflumizone |
| E-41 | 23 | and | Methomyl | F-41 | 24 | and | Methomyl |
| E-42 | 23 | and | Methoprene | F-42 | 24 | and | Methoprene |
| E-43 | 23 | and | Methoxyfenozide | F-43 | 24 | and | Methoxyfenozide |
| E-44 | 23 | and | Nitenpyram | F-44 | 24 | and | Nitenpyram |
| E-45 | 23 | and | Nithiazine | F-45 | 24 | and | Nithiazine |
| E-46 | 23 | and | Novaluron | F-46 | 24 | and | Novaluron |
| E-47 | 23 | and | NPV (e.g., Gemstar) | F-47 | 24 | and | NPV (e.g., Gemstar) |
| E-48 | 23 | and | Oxamyl | F-48 | 24 | and | Oxamyl |
| E-49 | 23 | and | Pymetrozine | F-49 | 24 | and | Pymetrozine |
| E-50 | 23 | and | Pyrethrin | F-50 | 24 | and | Pyrethrin |
| E-51 | 23 | and | Pyridaben | F-51 | 24 | and | Pyridaben |
| E-52 | 23 | and | Pyridalyl | F-52 | 24 | and | Pyridalyl |
| E-53 | 23 | and | Pyriproxyfen | F-53 | 24 | and | Pyriproxyfen |

(continued)

| Mixture No. | Comp. No. | and | Invertebrate Pest Control Agent | Mixture No. | Comp. No. | and | Invertebrate Pest Control Agent |
|---|---|---|---|---|---|---|---|
| E-54 | 23 | and | Ryanodine | F-54 | 24 | and | Ryanodine |
| E-55 | 23 | and | Spinosad | F-55 | 24 | and | Spinosad |
| E-56 | 23 | and | Spiradiclofen | F-56 | 24 | and | Spirodiclofen |
| E-57 | 23 | and | Spiromesifen | F-57 | 24 | and | Spiromesifen |
| E-58 | 23 | and | Tebufenozide | F-58 | 24 | and | Tebufenozide |
| E-59 | 23 | and | Thiacloprid | F-59 | 24 | and | Thiacloprid |
| E-60 | 23 | and | Thiamethoxam | F-60 | 24 | and | Thiamethoxam |
| E-61 | 23 | and | Thiodicarb | F-61 | 24 | and | Thiodicarb |
| E-62 | 23 | and | Tralomethrin | F-62 | 24 | and | Tralomethrin |
| E-63 | 23 | and | Triazamate | F-63 | 24 | and | Triazamate |
| E-64 | 23 | and | Triflumuron | F-64 | 24 | and | Triflumuron |
| E-65 | 23 | and | *Bacillus thuringiensis* | F-65 | 24 | and | *Bacillus thuringiensis* |
| E-66 | 23 | and | *Bacillus thuringiensis* delta toxin | F-66 | 24 | and | *Bacillus thuringiensis* delta toxin |
| E-67 | 23 | and | *Beauvaria bassiana* | F-67 | 24 | and | *Beauvaria bassiana* |
| G-1 | 29 | and | Abamectin | H-1 | 33 | and | Abamectin |
| G-2 | 29 | and | Acetamiprid | H-2 | 33 | and | Acetamiprid |
| G-3 | 29 | and | Amitraz | H-3 | 33 | and | Amitraz |
| G-4 | 29 | and | Anthranilamides | H-4 | 33 | and | Anthranilamides |
| G-5 | 29 | and | Avermectin | H-5 | 33 | and | Avermectin |
| G-6 | 29 | and | Azadirachtin | H-6 | 33 | and | Azadirachtin |
| G-7 | 29 | and | Beta-cyfluthrin | H-7 | 33 | and | Beta-cyfluthrin |
| G-8 | 29 | and | Bifenthrin | H-8 | 33 | and | Bifenthrin |
| G-9 | 29 | and | Buprofezin | H-9 | 33 | and | Buprofezin |
| G-10 | 29 | and | Cartap | H-10 | 33 | and | Cartap |
| G-11 | 29 | and | Chlorfenapyr | H-11 | 33 | and | Chlorfenapyr |
| G-12 | 29 | and | Chlorpyrifos | H-12 | 33 | and | Chlorpyrifos |
| G-13 | 29 | and | Clothianidin | H-13 | 33 | and | Clothianidin |
| G-14 | 29 | and | Cyfluthrin | H-14 | 33 | and | Cyfluthrin |
| G-15 | 29 | and | Cyhalothrin | H-15 | 33 | and | Cyhalothrin |
| G-16 | 29 | and | Cypermethrin | H-16 | 33 | and | Cypermethrin |
| G-17 | 29 | and | Cyromazine | H-17 | 33 | and | Cyromazine |
| G-18 | 29 | and | Deltamethrin | H-18 | 33 | and | Deltamethrin |
| G-19 | 29 | and | Dieldrin | H-19 | 33 | and | Dieldrin |
| G-20 | 29 | and | Dinotefuran | H-20 | 33 | and | Dinotefuran |
| G-21 | 29 | and | Diofenolan | H-21 | 33 | and | Diofenolan |
| G-22 | 29 | and | Emamectin | H-22 | 33 | and | Emamectin |
| G-23 | 29 | and | Emamectin Benzoate | H-23 | 33 | and | Emamectin Benzoate |
| G-24 | 29 | and | Endosulfan | H-24 | 33 | and | Endosulfan |
| G-25 | 29 | and | Esfenvalerate | H-25 | 33 | and | Esfenvalerate |
| G-26 | 29 | and | Ethiprole | H-26 | 33 | and | Ethiprole |
| G-27 | 29 | and | Fenothiocarb | H-27 | 33 | and | Fenothiocarb |
| G-28 | 29 | and | Fenoxycarb | H-28 | 33 | and | Fenoxycarb |
| G-29 | 29 | and | Fenvalerate | H-29 | 33 | and | Fenvalerate |
| G-30 | 29 | and | Fipronil | H-30 | 33 | and | Fipronil |
| G-31 | 29 | and | Flonicamid | H-31 | 33 | and | Flonicamid |
| G-32 | 29 | and | Flubendiamide | H-32 | 33 | and | Flubendiamide |
| G-33 | 29 | and | Flufenoxuron | H-33 | 33 | and | Flufenoxuron |

(continued)

| Mixture No. | Comp. No. | and | Invertebrate Pest Control Agent | | Mixture No. | Comp. No. | and | Invertebrate Pest Control Agent |
|---|---|---|---|---|---|---|---|---|
| G-34 | 29 | and | Hexaflumuron | | H-34 | 33 | and | Hexaflumuron |
| G-35 | 29 | and | Hydramethylnon | | H-35 | 33 | and | Hydramethylnon |
| G-36 | 29 | and | Imidacloprid | | H-36 | 33 | and | Imidacloprid |
| G-37 | 29 | and | Indoxacarb | | H-37 | 33 | and | Indoxacarb |
| G-38 | 29 | and | Lambda-cyhalothrin | | H-38 | 33 | and | Lambda-cyhalothrin |
| G-39 | 29 | and | Lufenuron | | H-39 | 33 | and | Lufenuron |
| G-40 | 29 | and | Metaflumizone | | H-40 | 33 | and | Metaflumizone |
| G-41 | 29 | and | Methomyl | | H-41 | 33 | and | Methomyl |
| G-42 | 29 | and | Methoprene | | H-42 | 33 | and | Methoprene |
| G-43 | 29 | and | Methoxyfenozide | | H-43 | 33 | and | Methoxyfenozide |
| G-44 | 29 | and | Nitenpyram | | H-44 | 33 | and | Nitenpyram |
| G-45 | 29 | and | Nithiazine | | H-45 | 33 | and | Nithiazine |
| G-46 | 29 | and | Novaluron | | H-46 | 33 | and | Novaluron |
| G-47 | 29 | and | NPV (e.g., Gemstar) | | H-47 | 33 | and | NPV (e.g., Gemstar) |
| G-48 | 29 | and | Oxamyl | | H-48 | 33 | and | Oxamyl |
| G-49 | 29 | and | Pymetrozine | | H-49 | 33 | and | Pymetrozine |
| G-50 | 29 | and | Pyrethrin | | H-50 | 33 | and | Pyrethrin |
| G-51 | 29 | and | Pyridaben | | H-51 | 33 | and | Pyridaben |
| G-52 | 29 | and | Pyridalyl | | H-52 | 33 | and | Pyridalyl |
| G-53 | 29 | and | Pyriproxyfen | | H-53 | 33 | and | Pyriproxyfen |
| G-54 | 29 | and | Ryanodine | | H-54 | 33 | and | Ryanodine |
| G-55 | 29 | and | Spinosad | | H-55 | 33 | and | Spinosad |
| G-56 | 29 | and | Spirodiclofen | | H-56 | 33 | and | Spirodiclofen |
| G-57 | 29 | and | Spiromesifen | | H-57 | 33 | and | Spiromesifen |
| G-58 | 29 | and | Tebufenozide | | H-58 | 33 | and | Tebufenozide |
| G-59 | 29 | and | Thiacloprid | | H-59 | 33 | and | Thiacloprid |
| G-60 | 29 | and | Thiamethoxam | | H-60 | 33 | and | Thiamethoxam |
| G-61 | 29 | and | Thiodicarb | | H-61 | 33 | and | Thiodicarb |
| G-62 | 29 | and | Tralomethrin | | H-62 | 33 | and | Tralomethrin |
| G-63 | 29 | and | Triazamate | | H-63 | 33 | and | Triazamate |
| G-64 | 29 | and | Triflumuron | | H-64 | 33 | and | Triflumuron |
| G-65 | 29 | and | *Bacillus thuringiensis* | | H-65 | 33 | and | *Bacillus thuringiensis* |
| G-66 | 29 | and | *Bacillus thuringiensis* delta toxin | | H-66 | 33 | and | *Bacillus thuringiensis* delta toxin |
| G-67 | 29 | and | *Beauvaria bassiana* | | H-67 | 33 | and | *Beauvaria bassiana* |
| I-1 | 34 | and | Abamectin | | J-1 | 35 | and | Abamectin |
| I-2 | 34 | and | Acetamiprid | | J-2 | 35 | and | Acetamiprid |
| I-3 | 34 | and | Amitraz | | J-3 | 35 | and | Amitraz |
| I-4 | 34 | and | Anthranilamides | | J-4 | 35 | and | Anthranilamides |
| I-5 | 34 | and | Avermectin | | J-5 | 35 | and | Avermectin |
| I-6 | 34 | and | Azadirachtin | | J-6 | 35 | and | Azadirachtin |
| I-7 | 34 | and | Beta-cyfluthrin | | J-7 | 35 | and | Beta-cyfluthrin |
| I-8 | 34 | and | Bifenthrin | | J-8 | 35 | and | Bifenthrin |
| I-9 | 34 | and | Buprofezin | | J-9 | 35 | and | Buprofezin |
| I-10 | 34 | and | Cartap | | J-10 | 35 | and | Cartap |
| I-11 | 34 | and | Chlorfenapyr | | J-11 | 35 | and | Chlorfenapyr |
| I-12 | 34 | and | Chlorpyrifos | | J-12 | 35 | and | Chlorpyrifos |
| I-13 | 34 | and | Clothianidin | | J-13 | 35 | and | Clothianidin |

(continued)

| Mixture No. | Comp. No. | and | Invertebrate Pest Control Agent | | Mixture No. | Comp. No. | and | Invertebrate Pest Control Agent |
|---|---|---|---|---|---|---|---|---|
| I-14 | 34 | and | Cyfluthrin | | J-14 | 35 | and | Cyfluthrin |
| I-15 | 34 | and | Cyhalothrin | | J-15 | 35 | and | Cyhalothrin |
| I-16 | 34 | and | Cypermethrin | | J-16 | 35 | and | Cypermethrin |
| I-17 | 34 | and | Cyromazine | | J-17 | 35 | and | Cyromazine |
| I-18 | 34 | and | Deltamethrin | | J-18 | 35 | and | Deltamethrin |
| I-19 | 34 | and | Dieldrin | | J-19 | 35 | and | Dieldrin |
| I-20 | 34 | and | Dinotefuran | | J-20 | 35 | and | Dinotefuran |
| I-21 | 34 | and | Diofenolan | | J-21 | 35 | and | Diofenolan |
| I-22 | 34 | and | Emamectin | | J-22 | 35 | and | Emamectin |
| I-23 | 34 | and | Emamectin Benzoate | | J-23 | 35 | and | Emamectin Benzoate |
| I-24 | 34 | and | Endosulfan | | J-24 | 35 | and | Endosulfan |
| I-25 | 34 | and | Esfenvalerate | | J-25 | 35 | and | Esfenvalerate |
| I-26 | 34 | and | Ethiprole | | J-26 | 35 | and | Ethiprole |
| I-27 | 34 | and | Fenothiocarb | | J-27 | 35 | and | Fenothiocarb |
| I-28 | 34 | and | Fenoxycarb | | J-28 | 35 | and | Fenoxycarb |
| I-29 | 34 | and | Fenvalerate | | J-29 | 35 | and | Fenvalerate |
| I-30 | 34 | and | Fipronil | | J-30 | 35 | and | Fipronil |
| I-31 | 34 | and | Flonicamid | | J-31 | 35 | and | Flonicamid |
| I-32 | 34 | and | Flubendiamide | | J-32 | 35 | and | Flubendiamide |
| I-33 | 34 | and | Flufenoxuron | | J-33 | 35 | and | Flufenoxuron |
| I-34 | 34 | and | Hexaflumuron | | J-34 | 35 | and | Hexaflumuron |
| I-35 | 34 | and | Hydramethylnon | | J-35 | 35 | and | Hydramethylnon |
| I-36 | 34 | and | Imidacloprid | | J-36 | 35 | and | Imidacloprid |
| I-37 | 34 | and | Indoxacarb | | J-37 | 35 | and | Indoxacarb |
| I-38 | 34 | and | Lambda-cyhalothrin | | J-38 | 35 | and | Lambda-cyhalothrin |
| I-39 | 34 | and | Lufenuron | | J-39 | 35 | and | Lufenuron |
| I-40 | 34 | and | Metaflumizone | | J-40 | 35 | and | Metaflumizone |
| I-41 | 34 | and | Methomyl | | J-41 | 35 | and | Methomyl |
| I-42 | 34 | and | Methoprene | | J-42 | 35 | and | Methoprene |
| I-43 | 34 | and | Methoxyfenozide | | J-43 | 35 | and | Methoxyfenozide |
| I-44 | 34 | and | Nitenpyram | | J-44 | 35 | and | Nitenpyram |
| I-45 | 34 | and | Nithiazine | | J-45 | 35 | and | Nithiazine |
| I-46 | 34 | and | Novaluron | | J-46 | 35 | and | Novaluron |
| I-47 | 34 | and | NPV (e.g., Gemstar) | | J-47 | 35 | and | NPV (e.g., Gemstar) |
| I-48 | 34 | and | Oxamyl | | J-48 | 35 | and | Oxamyl |
| I-49 | 34 | and | Pymetrozine | | J-49 | 35 | and | Pymetrozine |
| I-50 | 34 | and | Pyrethrin | | J-50 | 35 | and | Pyrethrin |
| I-51 | 34 | and | Pyridaben | | J-51 | 35 | and | Pyridaben |
| I-52 | 34 | and | Pyridalyl | | J-52 | 35 | and | Pyridalyl |
| I-53 | 34 | and | Pyriproxyfen | | J-53 | 35 | and | Pyriproxyfen |
| I-54 | 34 | and | Ryanodine | | J-54 | 35 | and | Ryanodine |
| I-55 | 34 | and | Spinosad | | J-55 | 35 | and | Spinosad |
| I-56 | 34 | and | Spirodiclofen | | J-56 | 35 | and | Spirodiclofen |
| I-57 | 34 | and | Spiromesifen | | J-57 | 35 | and | Spiromesifen |
| I-58 | 34 | and | Tebufenozide | | J-58 | 35 | and | Tebufenozide |
| I-59 | 34 | and | Thiacloprid | | J-59 | 35 | and | Thiacloprid |
| I-60 | 34 | and | Thiamethoxam | | J-60 | 35 | and | Thiamethoxam |
| I-61 | 34 | and | Thiodicarb | | J-61 | 35 | and | Thiodicarb |

(continued)

| Mixture No. | Comp. No. | and | Invertebrate Pest Control Agent | Mixture No. | Comp. No. | and | Invertebrate Pest Control Agent |
|---|---|---|---|---|---|---|---|
| I-62 | 34 | and | Tralomethrin | J-62 | 35 | and | Tralomethrin |
| I-63 | 34 | and | Triazamate | J-63 | 35 | and | Triazamate |
| I-64 | 34 | and | Triflumuron | J-64 | 35 | and | Triflumuron |
| I-65 | 34 | and | *Bacillus thuringiensis* | J-65 | 35 | and | *Bacillus thuringiensis* |
| I-66 | 34 | and | *Bacillus thuringiensis* delta toxin | J-66 | 35 | and | *Bacillus thuringiensis* delta toxin |
| I-67 | 34 | and | *Beauvaria bassiana* | J-67 | 35 | and | *Beauvaria bassiana* |

The specific mixtures listed in Table B typically combine a compound of Formula 1 and/or a compound listed in Index Table A with the other invertebrate pest agent in the ratios specified in Table A.

Invertebrate pests are controlled in agronomic and nonagronomic applications by applying a composition comprising a compound of this invention, in a biologically effective amount, to the environment of the pests, including the agronomic and/or nonagronomic locus of infestation, to the area to be protected, or directly on the pests to be controlled. Agronomic applications include protecting a field crop from invertebrate pests typically by applying a composition or a mixture of the invention to the seed of the crop before the planting, to the foliage, stems, flowers and/or fruit of crop plants, or to the soil or other growth medium before or after the crop is planted. Nonagronomic applications refer to invertebrate pest control in the areas other than fields of crop plants. Nonagronomic applications include control of invertebrate pests in stored grains, beans and other foodstuffs, and in textiles such as clothing and carpets. Nonagronomic applications also include invertebrate pest control in ornamental plants, forests, in yards, along roadsides and railroad rights of way, and on turf such as lawns, golf courses and pastures. Nonagronomic applications also include invertebrate pest control in houses and other buildings which may be occupied by humans and/or companion, farm, ranch, zoo or other animals. Nonagronomic applications also include the control of pests such as termites that can damage wood or other structural materials used in buildings.

Nonagronomic applications also include protecting human and animal health by controlling invertebrate pests that are parasitic or transmit infectious diseases. The controlling of animal parasites includes controlling external parasites that are parasitic to the surface of the body of the host animal (e.g., shoulders, armpits, abdomen, inner part of the thighs) and internal parasites that are parasitic to the inside of the body of the host animal (e.g., stomach, intestine, lung, veins, under the skin, lymphatic tissue). External parasitic or disease transmitting pests include, for example, chiggers, ticks, lice, mosquitoes, flies, mange, mites and fleas. Internal parasites include heartworms, hookworms and helminths. Compounds and compositions of the present invention are particular suitable for combating external parasitic or disease transmitting pests.

Compounds and compositions of the present invention are suitable for combating parasites that infest agricultural working animals, such as cattle, sheep, goats, horses, pigs, donkeys, camels, buffalos, rabbits, hens, turkeys, ducks, geese and bees; pet animals and domestic animals such as dogs, cats, pet birds and aquarium fish; as well as so-called experimental animals, such as hamsters, guinea pigs, rats and mice. By combating these parasites, fatalities and performance reduction (in term of meat, milk, wool, skins, eggs, honey, etc.) are to be reduced, so that applying a composition comprising a compound of the present invention is intended to allow more economic and simple husbandry of animals.

Nonagronomic applications in the veterinary sector takes place in the known manner, by enteral administration in the form of, for example, tablets, capsules, drinks, drenching preparations, granulates, pastes, boli, feed-through procedures, suppositories; by parenteral administration, such as by injection (including intramuscular, subcutaneous, intravenous, intraperitoneal), implants; by nasal administration; by dermal administration, for example, in the form of immersion or dipping, spraying, pouring, washing, coating with powder, and through bodied devices such as neck collars, ear marks, tail marks, limb measuring tapes or halters which comprise compounds or compositions of the present invention.

Therefore, the present invention further comprises a method for controlling an invertebrate pest in agronomic and/or nonagronomic applications, comprising contacting the invertebrate pest or its environment with a biologically effective amount of one or more of the compounds of the invention, or with a composition comprising at least one such compound or a composition comprising at least one such compound and a biologically effective amount of at least one additional biologically active compound or agent. Examples of suitable compositions comprising a compound of the invention and a biologically effective amount of at least one additional biologically active compound or agent include granular compositions wherein the additional active compound is present on the same granule as the compound of the invention or on granules separate from those of the compound of the invention.

One embodiment of a method of contact is by spraying. Alternatively, a granular composition comprising a compound of the invention can be applied to the plant foliage or the soil. Compounds of this invention can also be effectively delivered through plant uptake by contacting the plant with a composition comprising a compound of this invention applied as a soil drench of a liquid formulation, a granular formulation to the soil, a nursery box treatment or a dip of transplants. Of note is a composition of the present invention in the form of a soil drench liquid formulation. Also of note is a method for controlling an invertebrate pest comprising contacting the soil environment of the invertebrate pest with a biologically effective amount of a compound of the present invention. Of further note are compounds of this invention also effective by topical application to the locus of infestation. Other methods of contact include application of a compound or a composition of the invention by direct and residual sprays, aerial sprays, gels, seed coatings, microencapsulations, systemic uptake, baits, ear tags, boluses, foggers, fumigants, aerosols, dusts and many others. One embodiment of a method of contact is a dimensionally stable fertilizer granule, stick or tablet comprising a mixture or composition of the invention. The compounds of this invention can also be impregnated into materials for fabricating invertebrate control devices (e.g., insect netting). Seed coatings can be applied to all types of seeds, including those from which plants genetically transformed to express specialized traits will germinate. Representative examples include those expressing proteins toxic to invertebrate pests, such as *Bacillus thuringiensis* toxin or those expressing herbicide resistance, such as "Roundup Ready" seed.

The compounds of this invention can be incorporated into a bait composition that is consumed by an invertebrate pest or used within a device such as a trap, bait station, and the like. Such a bait composition can be in the form of granules which comprise (a) active ingredients, namely a biologically effective amount of a compound of Formula 1, an *N*-oxide, or salt thereof; (b) one or more food materials,; optionally (c) an attractant, and optionally (d) one or more humectants. Of note are granules or bait compositions which comprise between about 0.001-5% active ingredients, about 40-99% food material and/or attractant; and optionally about 0.05-10% humectants, which are effective in controlling soil invertebrate pests at very low application rates, particularly at doses of active ingredient that are lethal by ingestion rather than by direct contact. Some food materials can function both as a food source and an attractant. Food materials include carbohydrates, proteins and lipids. Examples of food materials are vegetable flour, sugar, starches, animal fat, vegetable oil, yeast extracts and milk solids. Examples of attractants are odorants and flavorants, such as fruit or plant extracts, perfume, or other animal or plant component, pheromones or other agents known to attract a target invertebrate pest. Examples of humectants, i.e. moisture retaining agents, are glycols and other polyols, glycerine and sorbitol. Of note is a bait composition (and a method utilizing such a bait composition) used to control at least one invertebrate pest selected from the group consisting of ants, termites and cockroaches. A device for controlling an invertebrate pest can comprise the present bait composition and a housing adapted to receive the bait composition, wherein the housing has at least one opening sized to permit the invertebrate pest to pass through the opening so the invertebrate pest can gain access to the bait composition from a location outside the housing, and wherein the housing is further adapted to be placed in or near a locus of potential or known activity for the invertebrate pest.

The compounds of this invention can be applied without other adjuvants, but most often application will be of a formulation comprising one or more active ingredients with suitable carriers, diluents, and surfactants and possibly in combination with a food depending on the contemplated end use. One method of application involves spraying a water dispersion or refined oil solution of a compound of the present invention. Combinations with spray oils, spray oil concentrations, spreader stickers, adjuvants, other solvents, and synergists such as piperonyl butoxide often enhance compound efficacy. For nonagronomic uses such sprays can be applied from spray containers such as a can, a bottle or other container, either by means of a pump or by releasing it from a pressurized container, e.g., a pressurized aerosol spray can. Such spray compositions can take various forms, for example, sprays, mists, foams, fumes or fog. Such spray compositions thus can further comprise propellants, foaming agents, etc. as the case may be. Of note is a spray composition comprising a biologically effective amount of a compound or a composition of the present invention and a carrier. One embodiment of such a spray composition comprises a biologically effective amount of a compound or a composition of the present invention and a propellant. Representative propellants include, but are not limited to, methane, ethane, propane, butane, isobutane, butene, pentane, isopentane, neopentane, pentene, hydrofluorocarbons, chlorofluorocarbons, dimethyl ether, and mixtures of the foregoing. Of note is a spray composition (and a method utilizing such a spray composition dispensed from a spray container) used to control at least one invertebrate pest selected from the group consisting of mosquitoes, black flies, stable flies, deer flies, horse flies, wasps, yellow jackets, hornets, ticks, spiders, ants, gnats, and the like, including individually or in combinations.

The rate of application required for effective control (i.e. "biologically effective amount") will depend on such factors as the species of invertebrate to be controlled, the pest's life cycle, life stage, its size, location, time of year, host crop or animal, feeding behavior, mating behavior, ambient moisture, temperature, and the like. Under normal circumstances, application rates of about 0.01 to 2 kg of active ingredients per hectare are sufficient to control pests in agronomic ecosystems, but as little as 0.0001 kg/hectare may be sufficient or as much as 8 kg/hectare may be required. For nonagronomic applications, effective use rates will range from about 1.0 to 50mg/square meter but as little as 0.1 mg/square meter may be sufficient or as much as 150 mg/square meter may be required. One skilled in the art can easily

determine the biologically effective amount necessary for the desired level of invertebrate pest control.

Synergism has been described as "the cooperative action of two components in a mixture, such that the total effect is greater or more prolonged than the sum of the effects of the two (or more) taken independently" (see P. M. L. Tames, Neth. J. Plant Pathology 1964, 70, 73-80). The presence of a synergistic effect between two active ingredients is established with the aid of the Colby equation (see S. R. Colby, "Calculating Synergistic and Antagonistic Responses of Herbicide Combinations", Weeds, 1967, 15, 20-22):

$$p = A + B - \left[\frac{A \times B}{100}\right]$$

Using the method of Colby, the presence of a synergistic interaction between two active ingredients is established by first calculating the predicted activity, p, of the mixture based on activities of the two components applied alone. If p is lower than the experimentally established effect, synergism has occurred. If p is equal or higher than the experimentally established effect, the interaction between the two components is characterized to be only additive or antagonism. In the equation above, A is the observed result of one component applied alone at rate x. The B term is the observed result of the second component applied at rate y. The equation estimates p, the observed result of the mixture of A at rate x with B at rate y if their effects are strictly additive and no interaction has occurred. To use the Colby equation the active ingredients of the mixture are applied in the test separately as well as in combination.

The following TESTS demonstrate the control efficacy of compounds, mixtures or compositions of this invention on specific pests. The pest control protection afforded by the compounds, mixtures or compositions is not limited, however, to these species. In certain instances, combinations of a compound of this invention with other invertebrate pest control compounds or agents are found to exhibit synergistic effects against certain important invertebrate pests.

The analysis of synergism or antagonism between the mixtures or compositions was determined using Colby's equation. The average % mortality data for the test compounds alone were inserted into the Colby's equation. If the observed (obs) average % mortality was higher than "p", the expected % mortality, the mixture or composition had synergistic effects. If the observed average % mortality was equal to or lower than the expected mortality, the mixture or composition either had no synergistic effect or an antagonistic effect. See Index Table A for compound descriptions. The following abbreviations are used in the Index Tables which follow: Me is methyl, and CN is cyano. The abbreviation "Ex." stands for "Example" and is followed by a number indicating in which example the compound is prepared.

INDEX TABLE A

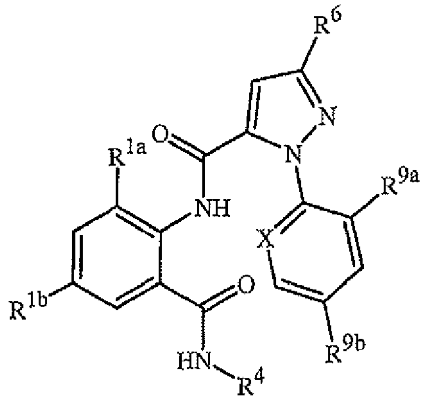

| Compound | $R^{1a}$ | $R^{1b}$ | $R^6$ | $R^{9a}$ | $R^{9b}$ | X | $R^4$ | m.p. (°C) |
|---|---|---|---|---|---|---|---|---|
| 1 (Ex. 1) | Me | CN | $CF_3$ | Cl | H | N | cyclopropylmethyl | 235-236 |
| 2 (Ex. 2,4) | Me | Cl | Br | Cl | H | N | cyclopropylmethyl | 172-173 |
| 3 | Me | CN | Br | Cl | H | N | cyclopropylmethyl | 223-224 |
| 4 | Cl | Cl | Br | Cl | H | N | (2-oxetanyl)methyl | 120-122* |
| 5 (Ex.3) | Me | Cl | Br | Cl | H | N | (2-oxetanyl)methyl | 95-97* |
| 6 | Cl | Cl | Cl | Cl | H | N | (2-oxetanyl)methyl | * |
| 7 | Me | CN | $CF_3$ | Cl | H | CH | (2-oxetanyl)methyl | * |
| 8 | Me | CN | Br | Cl | H | N | (2-oxetanyl)methyl | * |

(continued)

| Compound | $R^{1a}$ | $R^{1b}$ | $R^6$ | $R^{9a}$ | $R^{9b}$ | X | $R^4$ | m.p. (°C) |
|---|---|---|---|---|---|---|---|---|
| 9 (Ex. 8) | Me | Cl | Br | Cl | H | N | 1-methylcyclopropyl | >250 |
| 10 | Cl | Cl | Br | Cl | H | N | 1-methylcyclopropyl | 200-201 |
| 11 | Me | CN | Br | Cl | H | N | 1-methylcyclopropyl | >250 |
| 12 | Me | CN | Br | Cl | F | CH | cyclopropylmethyl | 221-222 |
| 13 | Cl | Cl | Br | Cl | Cl | CH | cyclopropylmethyl | 232-233 |
| 14 | Cl | Cl | Br | Cl | F | CH | cyclopropylmethyl | 218-219 |
| 15 | Me | Cl | Br | Cl | F | CH | cyclopropylmethyl | 240-241 |
| 16 | Cl | Cl | Cl | Cl | F | CH | cyclopropylmethyl | 212-213 |
| 17 | Cl | Cl | Cl | Cl | Cl | CCl | cyclopropylmethyl | 233-234 |
| 18 | Me | CN | Cl | Cl | Cl | CCl | cyclopropylmethyl | 197-198 |
| 19 | Cl | Cl | Br | Cl | Cl | CCl | cyclopropylmethyl | 237-238 |
| 20 | Me | CN | $CF_3$ | Cl | Cl | CCl | cyclopropylmethyl | 227-228 |
| 21 | Cl | Cl | $CF_3$ | Cl | Cl | CCl | cyclopropylmethyl | 196-197 |
| 22 | Me | CN | $CF_3$ | F | H | CH | cyclopropylmethyl | 249-250 |
| 23 | Me | CN | Br | Cl | H | CH | cyclopropylmethyl | 231-231 |
| 24 | Me | Cl | Br | Cl | H | CH | cyclopropylmethyl | 207-208 |
| 25 | Cl | Cl | Br | Cl | H | CH | cyclopropylmethyl | 199-200 |
| 26 | Me | CN | $CF_3$ | Cl | H | CH | cyclopropylmethyl | 134-135 |
| 27 | Me | Cl | $CF_3$ | Cl | H | CH | cyclopropylmethyl | 217-218 |
| 28 | Cl | Cl | $CF_3$ | Cl | H | CH | cyclopropylmethyl | 240-241 |
| 29 (Ex. 5) | Me | Cl | Br | Cl | H | N | 1-cyclopropylethyl | 182-183 |
| 30 | Me | CN | $CF_3$ | Cl | H | CH | 1-cyclopropylethyl | 189-190 |
| 31 (Ex.7) | Me | Cl | Br | Cl | H | CH | 1-cyclopropylethyl | 180-181 |
| 32 | Me | Cl | $CF_3$ | Cl | H | CH | 1-cyclopropylethyl | 179-180 |
| 33 | Me | CN | Cl | Cl | H | N | 1-cyclopropylethyl | 155-156 |
| 34 | Me | CN | $CF_3$ | Cl | H | N | 1-cyclopropylethyl | 185-186 |
| 35 (Ex. 6) | Me | CN | Br | Cl | H | N | 1-cyclopropylethyl | 244-245 |

*See Index Table B for $^1H$ NMR data

INDEX TABLE B

| Compd. No. | $^1H$ NMR Data ($CDCl_3$ solution unless indicated otherwise)[a] |
|---|---|
| 4 | δ 9.76 (s, 1H), 8.42 (m, 1H), 7.81 (m, 1H), 7.32 (m, 1H), 7.24 (m, 2H), 7.17 (s, 1H), 6.97 (m, 1H), 4.89 (m, 1H), 4.61 (m, 1H), 4.41 (m, 1H), 3.60 (m, 1H), 3.49 (m, 1H), 2.62 (m, 1H), 2.41 (m, 1H). |
| 5 | δ 10.1 (br s, 1H), 8.43 (m, 1H), 7.82 (m, 1H), 7.35 (m, 1H), 7.23 (m, 2H), 7.09 (br s, 1H), 6.84 (m, 1H), 4.92 (m, 1H), 4.64 (m, 1H), 4.44 (m, 1H), 3.67 (m, 1H), 3.53 (m, 1H), 2.65 (m, 1H), 2.41 (m, 1H), 2.14 (s, 3H). |
| 6 | δ 9.75 (s, 1H), 8.41 (m, 1H), 7.80 (m, 1H), 7.31 (m, 1H), 7.21 (s, 2H), 7.05 (m, 2H), 4.88 (m, 1H), 4.61 (m, 1H), 4.41 (m, 1H), 3.59 (m, 1H), 3.48 (m, 1H) 2.62 (m, 1H), 2.41 (m, 1H). |
| 7 | δ 10.6 (s, 1H), 7.65 (br s, 1H), 7.57 (br s, 1H), 7.51 (m, 1H), 7.43 (m, 3H), 7.29 (br s, 1H), 7.04 (m, 1H), 4.95 (m, 1H), 4.67 (m, 1H), 4.48 (m, 1H), 3.68 (m, 1H), 3.58 (m, 1H), 2.68 (m, 1H), 2.43 (m, 1H), 2.49 (s, 3H). |
| 8 | δ 10.5 (s, 1H), 8.44 (m, 1H), 7.84 (m, 1H), 7.64 (m, 1H), 7.56 (m, 1H), 7.37 (m, 1H), 7.05 (m, 2H), 4.95 (m, 1H), 4.66 (m, 1H), 4.48 (m, 1H), 3.70 (m, 1H), 3.58 (m, 1H), 2.69 (m, 1H), 2.43 (m, 1H), 2.23 (s, 3H). |

[a] $^1H$ NMR data are in ppm downfield from tetramethylsilane. Couplings are designated by (s)-singlet, (d)-doublet, (t)-triplet, (q)-quartet, (m)-multiplet, (dd)-doublet of doublets, (dt)-doublet of triplets, (br s)-broad singlet.

BIOLOGICAL EXAMPLES OF THE INVENTION

TEST A

For evaluating control of diamondback moth (*Plutella xylostella*) the test unit consisted of a small open container with a 12-14-day-old radish plant inside. This was pre-infested with 10-15 neonate larvae on a piece of insect diet by use of a core sampler to remove a plug from a sheet of hardened insect diet having many larvae growing on it and transfer the plug containing larvae and diet to the test unit. The larvae moved onto the test plant as the diet plug dried out.

Test compounds or mixtures were formulated using a solution containing 10% acetone, 90% water and 300 ppm X-77™ Spreader Lo-Foam Formula non-ionic surfactant containing alkylarylpolyoxyethylene, free fatty acids, glycols and 2-propanol (Loveland Industries, Inc. Greeley, Colorado, USA). The formulated compounds or mixtures were applied in 1 mL of liquid through a SUJ2 atomizer nozzle with 1/8 JJ custom body (Spraying Systems Co. Wheaton, Illinois, USA) positioned 1.27 cm (0.5 inches) above the top of each test unit. All experimental compounds in these tests were sprayed at 10 ppm replicated three times. For experimental mixtures in these tests, to obtain the desired mixture concentrations of each compound, twice the desired concentration of each of the two mixture partner compounds were mixed together in equal volumes.

After spraying of the formulated test compound or mixture, each test unit was allowed to dry for 1 hour and then a black, screened cap was placed on top. The test units were held for 6 days in a growth chamber at 25 °C and 70% relative humidity. Plant feeding damage was then visually assessed based on foliage consumed.

Of the compounds of Formula **1** tested the following provided very good to excellent levels of plant protection (20% or less feeding damage): 1, 2 and 3.

For the mixtures tested the results are listed in Table A1. The "*" indicates the observed % protection is higher than the calculated % protection by the Colby equation.

Table A1

| Diamondback moth | ppm | % protection (obs) | ppm | % protection (obs) | ppm | % protection (obs) |
|---|---|---|---|---|---|---|
| Compound 2 | 0.03 | 37 | 0.04 | 77 | 0.06 | 93 |
| Compound 3 | 0.04 | 87 | 0.06 | 93 | 0.09 | 93 |
| Imidacloprid | 0.8 | 73 | 17 | 80 | 50 | 90 |
| Thiamethoxam | 30 | 43 | 40 | 73 | 50 | 90 |
| Compound 2 + Imidacloprid | 0.03 + 0.8 | 87* | 0.04 + 0.8 | 97* | 0.06 + 0.8 | 90 |
| | 0.03 + 17 | 100* | 0.04 + 17 | 97* | 0.06 + 17 | 93 |
| | 0.03 + 50 | 90 | 0.04 + 50 | 97 | 0.06 + 50 | 93 |
| Compound 2 + Thiamethoxam | 0.03 + 30 | 67* | 0.04 + 30 | 73 | 0.06 + 30 | 73 |
| | 0.03 + 40 | 80 | 0.04 + 40 | 80 | 0.06 + 40 | 83 |
| | 0.03 + 50 | 87 | 0.04 + 50 | 93 | 0.06 + 50 | 97 |
| Compound 3 + Imidacloprid | 0.04 + 0.8 | 57 | 0.06 + 0.8 | 33 | 0.09 + 0.8 | 33 |
| | 0.04 + 17 | 67 | 0.06 + 17 | 73 | 0.09 + 17 | 77 |
| | 0.04 + 50 | 90 | 0.06 + 50 | 77 | 0.09 + 50 | 90 |
| Compound 3 + Thiamethoxam | 0.04 + 30 | 63 | 0.06 + 30 | 47 | 0.09 + 30 | 63 |
| | 0.04 + 40 | 73 | 0.06 + 40 | 63 | 0.09 + 40 | 73 |
| | 0.04 + 50 | 90 | 0.06 + 50 | 90 | 0.09 + 50 | 93 |

TEST B

For evaluating control of fall armyworm (*Spodoptera frugiperda*) the test unit consisted of a small open container with a 4-5-day-old corn (maize) plant inside. This was pre-infested (using a core sampler) with 10-15 1-day-old larvae on a piece of insect diet.

Test compounds 1, 2 and 3 were formulated and sprayed at 10 ppm as described for Test A. The applications were replicated three times. After spraying, the test units were maintained in a growth chamber and then visually rated as described for Test A.

Of the compounds of Formula **1** tested, the following provided excellent levels of plant protection (20% or less

feeding damage): 1, 2, 3, 4, 5, 9, 10, 11, 12, 13, 14, 15, 16, 17, 18, 19, 20, 21, 22, 23, 24, 25, 26, 27, 28, 29, 30 and 31.

TEST C

For evaluating control of green peach aphid (*Myzus persicae*) through contact and/or systemic means, the test unit consisted of a small open container with a 12- to 15-day-old radish plant inside. This was pre-infested by placing on a leaf of the test plant 30-40 aphids on a piece of leaf excised from a culture plant (cut-leaf method). The larvae moved onto the test plant as the leaf piece desiccated. After pre-infestation, the soil of the test unit was covered with a layer of sand.

All test compounds were formulated and sprayed at 50 ppm as described for Test A and replicated three times. Test mixtures were formulated as described for Test A and replicated three times. After spraying of the formulated test compound or mixture, each test unit was allowed to dry for 1 hour and then a black, screened cap was placed on top. The test units were held for 6 days in a growth chamber at 19-21 °C and 50-70% relative humidity. Dead and total number of aphids were counted in each test unit to determine percent mortality.

Of the compounds of Formula **1** tested, the following resulted in at least 80% mortality: 1, 3, 4, 6, 8, 9, 10, 11 and 12.

For the mixtures tested the results are listed in Table C1. The "*" indicates the observed % mortality is higher than the calculated % mortality by the Colby equation.

Table C1

| Green Peach Aphid: | ppm | Mortality(obs) | ppm | Mortality(obs) | ppm | Mortality(obs) |
|---|---|---|---|---|---|---|
| Compound 2 | 8 | 4 | 50 | 17 | 250 | 76 |
| Compound 3 | 2 | 1 | 5 | 9 | 9 | 6 |
| Imidacloprid | 0.08 | 5 | 0.15 | 44 | 0.3 | 50 |
| Thiamethoxam | 0.2 | 44 | 0.4 | 73 | 0.6 | 87 |
| Compound 2 + Imidacloprid | 8 + 0.08 | 9 | 50 + 0.08 | 42* | 250 + 0.08 | 59 |
| | 8 + 0.15 | 33 | 50 + 0.15 | 67* | 250 + 0.15 | 78 |
| | 8 + 0.3 | 62* | 50 + 0.3 | 74* | 250 + 0.3 | 94* |
| Compound 2 + Thiamethoxam | 8 + 0.2 | 37 | 50+0.2 | 49 | 250 + 0.2 | 68 |
| | 8 + 0.4 | 39 | 50 + 0.4 | 49 | 250 + 0.4 | 85 |
| | 8 + 0.6 | 64 | 50 + 0.6 | 82 | 250 + 0.6 | 92 |
| Compound 3 + Imidacloprid | 2 + 0.08 | 7* | 5+0.08 | 5 | 9 + 0.08 | 11 |
| | 2 + 0.15 | 18 | 5 + 0.15 | 28 | 9 + 0.15 | 26 |
| | 2+0.3 | 58* | 5 + 0.3 | 61* | 9+ 0.3 | 68* |
| Compound 3 + Thiamethoxam | 2+0.2 | 12 | 5+0.2 | 9 | 9 + 0.2 | 10 |
| | 2 + 0.4 | 23 | 5 + 0.4 | 45 | 9 + 0.4 | 46 |
| | 2 + 0.6 | 92* | 5 + 0.6 | 62 | 9 + 0.6 | 90* |

TEST D

For evaluating control of potato leafhopper (*Empoasca fabae* Harris) through contact and/or systemic means, the test unit consisted of a small open container with a 5-6 day old Longio bean plant (primary leaves emerged) inside. White sand was added to the top of the soil and one of the primary leaves was excised prior to application. Test compounds were formulated and sprayed at 50 ppm and replicated three times as described for Test A. After spraying, the test units were allowed to dry for 1 hour before they were post-infested with 5 potato leafhoppers (18 to 21 day old adults). A black, screened cap was placed on the top of the cylinder. The test units were held for 6 days in a growth chamber at 19-21 °C and 50-70% relative humidity. Each test unit was then visually assessed for insect mortality.

Of the compounds of Formula **1** tested, the following resulted in at least 80% mortality: 2, 3, 9, 10 and 11.

TEST E

For evaluating control of cotton melon aphid (*Aphis gossypii*) through contact and/or systemic means, the test unit consisted of a small open container with a 6-7-day-old cotton plant inside. This was pre-infested with 30-40 insects

on a piece of leaf according to the cut-leaf method described for Test C, and the soil of the test unit was covered with a layer of sand. Test compounds were formulated and sprayed at 50 ppm as described for Test A. The applications were replicated three times. After spraying, the test units were maintained in a growth chamber and then visually rated as described for Test A.

Of the compounds of Formula **1** tested, the following resulted in at least 80% mortality: 1, 2, 3, 4, 5, 6, 8, 9, 10 and 11.

TEST F

For evaluating control of corn planthopper (*Peregrinus maidis*) through contact and/or systemic means, each test unit consisted of a small open cylindrical container with a 3- to 4- day-old corn (maize) plant (spike) inside. White sand was added to the top of the soil prior to application. Test mixtures were formulated and sprayed with 3 replications as described for Test A. After spraying, the test units were allowed to dry for 1 hour before they were post-infested with 10 to 20 corn planthoppers (18- to 20-day-old nymphs) by sprinkling them onto the sand with a salt shaker. A black, screened cap was placed on the top of each container. The test units were held for 6 days in a growth chamber at 19-21 °C and 50-70% relative humidity. Dead and total number of corn planthoppers were counted in each test unit to determine percent mortality.

For the mixtures tested the results are listed in Table F1. The "*" indicates the observed % mortality is higher than the calculated % mortality by the Colby equation.

Table F1

| Corn Planthopper | ppm | Mortality(obs) | ppm | Mortality(obs) | ppm | Mortality(obs) |
|---|---|---|---|---|---|---|
| Compound 2 | 30 | 2 | 100 | 10 | 250 | 31 |
| Compound 3 | 40 | 3 | 110 | 10 | 250 | 81 |
| Imidacloprid | 0.08 | 2 | 0.2 | 10 | 0.8 | 71 |
| Thiamethoxam | 0.2 | 19 | 0.4 | 73 | 0.6 | 100 |
| Compound 2 + Imidacloprid | 30 + 0.08 | 2 | 100 + 0.08 | 3 | 250 + 0.08 | 3 |
| | 30 + 0.2 | 18* | 100 + 0.2 | 14 | 250 + 0.2 | 12 |
| | 30 + 0.8 | 100* | 100 + 0.8 | 82* | 250 + 0.8 | 100* |
| Compound 2 + Thiamethoxam | 30 + 0.2 | 9 | 100 + 0.2 | 9 | 250 + 0.2 | 37 |
| | 30 + 0.4 | 40 | 100 + 0.4 | 100* | 250 + 0.4 | 100* |
| | 30 + 0.6 | 100 | 100 + 0.6 | 100 | 250 + 0.6 | 100 |
| Compound 3 + Imidacloprid | 40 + 0.08 | 3 | 110 + 0.08 | 2 | 250 + 0.08 | 56 |
| | 40 + 0.2 | 9 | 110 + 0.2 | 53* | 250 + 0.2 | 100* |
| | 40 + 0.8 | 100* | 110 + 0.8 | 100* | 250 + 0.8 | 100* |
| Compound 3 + Thiamethoxam | 40 + 0.2 | 87* | 110 + 0.2 | 49* | 250 + 0.2 | 97* |
| | 40 + 0.4 | 100* | 110 + 0.4 | 87* | 250 + 0.4 | 79 |
| | 40 + 0.6 | 100 | 110 + 0.6 | 100 | 250 + 0.6 | 100 |

TEST G

For evaluating control of the western flower thrips (*Frankliniella occidentalis* Pergande) through contact and/or systemic means, each test unit consisted of a small open container with a 5- to 7-day-old bean (var. Soleil) plant inside.

Test mixtures were formulated and sprayed with 3 replications as described for Test A. After spraying, the test units were allowed to dry for 1 hour, 22 to 27 adult thrips were added to each unit and then a black, screened cap was placed on top. The test units were held for 7 days at 25 °C and 45-55% relative humidity. The amount of plant damage for each test unit was rated to determine the percent plant protection.

For the mixtures tested the results are listed in Table G1. The "*" indicates the observed % protection is higher than the calculated % protection by Colby equation.

Table G1

| Western Flower Thrips | ppm | % protection (obs) | ppm | % protection (obs) | ppm | % protection (obs) |
|---|---|---|---|---|---|---|
| Compound 2 | 5 | 20 | 150 | 27 | 250 | 53 |
| Compound 3 | 9 | 20 | 40 | 13 | 250 | 0 |
| Imidacloprid | 11 | 27 | 77 | 40 | 250 | 90 |
| Thiamethoxam | 5 | 0 | 70 | 77 | 250 | 100 |
| Compound 2 + Imidacloprid | 5 + 11 | 7 | 150 + 11 | 63* | 250 + 11 | 67* |
| | 5 + 77 | 23 | 150 + 77 | 57* | 250 + 77 | 83* |
| | 5 + 250 | 83 | 150 + 250 | 90 | 250 + 250 | 83 |
| Compound 2 + Thiamethoxam | 5 + 5 | 20 | 150 + 5 | 27 | 250 + 5 | 60* |
| | 5 + 70 | 57 | 150 + 70 | 83 | 250 + 70 | 83 |
| | 5 + 250 | 93 | 150 + 250 | 97 | 250 + 250 | 97 |
| Compound 3 + Imidactoprid | 9 + 11 | 53* | 40 + 11 | 43* | 250 + 11 | 60* |
| | 9 + 77 | 23 | 40 + 77 | 30 | 250 + 77 | 37 |
| | 9 + 250 | 87 | 40 + 250 | 83 | 250 + 250 | 90 |
| Compound 3 + Thiamethoxam | 9 + 5 | 20 | 40 + 5 | 7 | 250 + 5 | 16 |
| | 9 + 70 | 43 | 40 + 70 | 40 | 250 + 70 | 67 |
| | 9 + 250 | 97 | 40 + 250 | 100 | 250 + 250 | 100 |

## Claims

**1.** A compound that is 2-amino-5-chloro-*N*-(cyclopropylmethyl)-3-methylbenzamide.

**2.** A compound that is 2-amino-5-bromo-*N*-(cyclopropylmethyl)-3-methylbenzamide.

**3.** A compound that is 2-amino-3,5-dichloro-*N*-(cyclopropylmethyl)-benzamide.

**4.** A compound that is 2-amino-3,5-dibromo-*N*-(cyclopropylmethyl)-benzamide.

**5.** A compound that is 2-amino-5-chloro-*N*-(1-cyclopropylethyl)-3-methylbenzamide.

**6.** A compound that is 2-amino-5-bromo-*N*-(1-cyclopropylethyl)-3-methylbenzamide.

**7.** A compound that is 2-amino-3,5-dichloro-*N*-(1-cyclopropylethyl)-benzamide.

**8.** A compound that is 2-amino-3,5-dibromo-*N*-(1-cyclopropylethyl)-benzamide.

Europäisches Patentamt
European Patent Office
Office européen des brevets

# EUROPEAN SEARCH REPORT

Application Number
EP 12 19 4234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/024222 A1 (DU PONT [US]; BERGER RICHARD ALAN [US]; FLEXNER JOHN LINDSEY [US]) 27 March 2003 (2003-03-27)<br>* scheme 1;<br>page 12 *<br>----- | 1-8 | INV.<br>A01N43/56<br>C07D231/16<br>C07D401/04<br>C07D405/12<br>C07D405/14 |

TECHNICAL FIELDS SEARCHED (IPC)
C07D

The present search report has been drawn up for all claims

| Place of search | Date of completion of the search | Examiner |
|---|---|---|
| Munich | 20 December 2012 | Koch, Kristian |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 19 4234

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2012

| Patent document cited in search report | Publication date | Patent family member(s) | Publication date |
|---|---|---|---|
| WO 03024222 A1 | 27-03-2003 | AR 036606 A1 | 22-09-2004 |
| | | AT 370656 T | 15-09-2007 |
| | | BR 0212993 A | 17-08-2004 |
| | | CA 2458163 A1 | 27-03-2003 |
| | | CN 1713819 A | 28-12-2005 |
| | | DE 60221994 T2 | 15-05-2008 |
| | | DK 1427285 T3 | 05-11-2007 |
| | | EP 1427285 A1 | 16-06-2004 |
| | | ES 2291500 T3 | 01-03-2008 |
| | | HU 0401893 A2 | 28-01-2005 |
| | | IL 159947 A | 24-12-2009 |
| | | JP 3770495 B2 | 26-04-2006 |
| | | JP 2005502716 A | 27-01-2005 |
| | | KR 20040035857 A | 29-04-2004 |
| | | MX 248274 B | 23-08-2007 |
| | | MY 136698 A | 28-11-2008 |
| | | NZ 532269 A | 28-10-2005 |
| | | PL 206331 B1 | 30-07-2010 |
| | | PT 1427285 E | 27-11-2007 |
| | | RU 2292138 C2 | 27-01-2007 |
| | | TW I283164 B | 01-07-2007 |
| | | US 2004209923 A1 | 21-10-2004 |
| | | US 2010152194 A1 | 17-06-2010 |
| | | WO 03024222 A1 | 27-03-2003 |
| | | ZA 200400413 A | 20-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03015518 A
- WO 2003015519 A
- WO 2004011447 A
- WO 2004067528 A
- WO 03024222 A
- US 3060084 A
- WO 9113546 A
- US 4172714 A
- US 4144050 A
- US 3920442 A
- DE 3246493
- US 5180587 A
- US 5232701 A
- US 5208030 A
- GB 2095558 A
- US 3299566 A
- US 3235361 A
- US 3309192 A
- US 2891855 A


### Non-patent literature cited in the description


- **T. L. GILCHRIST.** Comprehensive Organic Synthesis. Pergamon Press, vol. 7, 748-750
- **M. TISLER ; B. STANOVNIK.** Comprehensive Heterocyclic Chemistry. Pergamon Press, vol. 3, 18-20
- **M. R. GRIMMETT ; B. R. T. KEENE.** Advances in Heterocyclic Chemistry. Academic Press, vol. 43, 149-161
- **M. TISLER ; B. STANOVNIK.** Advances in Heterocyclic Chemistry. Academic Press, vol. 9, 285-291
- **G. W. H. CHEESEMAN ; E. S. G. WERSTIUK.** Advances in Heterocyclic Chemistry. Academic Press, vol. 22, 390-392
- **JAKOBSEN et al.** *Bioorganic and Medicinal Chemistry,* 2000, vol. 8, 2095-2103
- **G. M. COPPOLA.** *J. Heterocyclic Chemistry,* 1999, vol. 36, 563-588
- **MITSUNOBU, O.** Comprehensive Organic Synthesis. Pergamon, 1991, vol. 6, 65-101
- **SALVATORE, R. N. et al.** *Tetrahedron,* 2001, vol. 57, 7785-7811
- **GREENE, T. W. ; WUTS,P. G. M.** Protective Groups in Organic Synthesis. Wiley, 1991
- **WATKINS et al.** Handbook of Insecticide Dust Diluents and Carriers. Dorland Books
- **MARSDEN.** Solvents Guide. Interscience, 1950
- McCutcheon's Detergents and Emulsifiers Annual. Allured Publ. Corp,
- **SISELY ; WOOD.** Encyclopedia of Surface Active Agents. Chemical Publ. Co., Inc, 1964
- *Pure and Applied Chemistry,* vol. 72, 1255-1264
- **MCCUTCHEON'S.** Functional Materials. MC Publishing Company, 2001, vol. 2
- **BROWNING.** Agglomeration. *Chemical Engineering,* 04 December 1967, 147-48
- Perry's Chemical Engineer's Handbook. McGraw-Hill, 1963, 8-57
- **T. S. WOODS.** The Formulator's Toolbox - Product Forms for Modern Agriculture. *Pesticide Chemistry and Bioscience, The Food-Environment Challenge*
- Proceedings of the 9th International Congress on Pesticide Chemistry. The Royal Society of Chemistry, 1999, 120-133
- **KLINGMAN.** Weed Control as a Science. John Wiley and Sons, Inc, 1961, 81-96
- **HANCE et al.** Weed Control Handbook. Blackwell Scientific Publications, 1989
- Developments in formulation technology. PJB Publications, 2000
- The Pesticide Manual. British Crop Protection Council, 2003
- The BioPesticide Manual. British Crop Protection Council, 2001
- **P. M. L. TAMES.** *Neth. J. Plant Pathology,* 1964, vol. 70, 73-80
- **S. R. COLBY.** Calculating Synergistic and Antagonistic Responses of Herbicide Combinations. *Weeds,* 1967, vol. 15, 20-22